# EUROPEAN PATENT APPLICATION

(11) **EP 4 393 496 A1**
(43) Date of publication of application: **03.07.2024**
(21) Application number: 21962467.3
(22) Date of filing: 29.10.2021
(51) Int. Cl.: A61K 31/737, A61K 47/36, A61P 27/02

(54) **OPHTHALMIC COMPOSITION**

(71) Applicant: Rohto Pharmaceutical Co., Ltd., Osaka-shi, Osaka 544-8666 (JP)
(72) Inventor: ANDO Tatsuya, Osaka-shi, Osaka 544-8666 (JP); MITSUGUCHI Yoko, Osaka-shi, Osaka 544-8666 (JP); MATSUMOTO Sachiko, Osaka-shi, Osaka 544-8666 (JP); NAKATA Atsuko, Osaka-shi, Osaka 544-8666 (JP); KIYOBAYASHI Yuka, Osaka-shi, Osaka 544-8666 (JP)
(74) Representative: Mewburn Ellis LLP
(86) International application number: PCT/JP2021/040030
(87) International publication number: WO 2023/073925

(57) **Abstract**

The first present invention relates to an ophthalmic composition for reducing eye dryness containing at least one selected from the group consisting of chondroitin sulfate and salts thereof which have a weight-average-molecular weight of 40,000 to 70,000. The second present invention relates to an ophthalmic composition containing at least one selected from the group consisting of chondroitin sulfate and salts thereof which have a weight-average-molecular weight of 40,000 to 70,000, and at least one selected from the group consisting of an antihistamine, a zinc salt, tetrahydrozoline and salts thereof, hydroxyethyl cellulose and salts thereof, and a polyvinyl-based polymer compound. The third present invention relates to an ophthalmic composition containing at least one selected from the group consisting of chondroitin sulfate and salts thereof which have a weight-average-molecular weight of 40,000 to 70,000, and menthol.

## Description

### Technical Field

Hereinafter, a first present invention, a second present invention and a third present invention will be described in order.

The first present invention relates to an ophthalmic composition.

### Background Art

Eye dryness (dry eye) can be caused by dry air due to use of an air conditioner, a reduced number of blinks due to long hours of computer work, wearing of contact lenses, and the like, and can cause various unpleasant symptoms such as eye pain. Eye dryness symptoms can be alleviated, for example, by frequently instilling artificial tears into the eyes, but little is known about ophthalmic formulations that can prevent eye dryness in advance.

Chondroitin sulfate or salts thereof are a type of acidic mucopolysaccharide, and are added to an ophthalmic formulation for promoting energy metabolism, promoting metabolism and cell respiration to relieve eye fatigue, replenishing lachrymal fluid components, and the like (for example, Patent Literature 1).

### Citation List

### Patent Literature

[Patent Literature 1] Japanese Unexamined Patent Publication No. 2011-148791

### Summary of Invention

### Technical Problem

An object of the first present invention is to provide an ophthalmic composition that can reduce eye dryness.

### Solution to Problem

Surprisingly, the inventors found that sodium chondroitin sulfate having a specific weight-average-molecular weight significantly reduces corneal damage caused by eye dryness, has high affinity for contact lenses, and also reduces dryness of contact lenses.

The first present invention provides, for example, the following inventions.
[1] An ophthalmic composition for reducing eye dryness containing at least one selected from the group consisting of chondroitin sulfate and salts thereof which have a weight-average-molecular weight of 40,000 to 70,000.
[2] An ophthalmic composition for reducing dryness of contact lenses containing at least one selected from the group consisting of chondroitin sulfate and salts thereof which have a weight-average-molecular weight of 40,000 to 70,000.
[3] The ophthalmic composition according to [1] or [2], further containing at least one selected from the group consisting of an anti-inflammatory agent, A vitamins, B vitamins, E vitamins, aminoethylsulfonic acid and salts thereof, aspartic acid and salts thereof, neostigmine and salts thereof, and a cellulose-based polymer compound.

The first present invention also provides, for example, the following inventions.
[2-1] An ophthalmic composition containing at least one selected from the group consisting of chondroitin sulfate and salts thereof which have a weight-average-molecular weight of 40,000 to 70,000, and at least one selected from the group consisting of A vitamins, aminoethylsulfonic acids and salts thereof.
[2-2] An ophthalmic composition containing at least one selected from the group consisting of chondroitin sulfate and salts thereof which have a weight-average-molecular weight of 40,000 to 70,000, and at least one selected from the group consisting of an anti-inflammatory agent, A vitamins, B vitamins, E vitamins, aminoethylsulfonic acid and salts thereof, aspartic acid and salts thereof, neostigmine and salts thereof, and a cellulose-based polymer compound.

### Advantageous Effects of Invention

According to the first present invention, it is possible to provide an ophthalmic composition that can reduce eye dryness. In addition, according to the first present invention, it is possible to provide an ophthalmic composition that can reduce dryness of contact lenses.

### Brief Description of Drawings

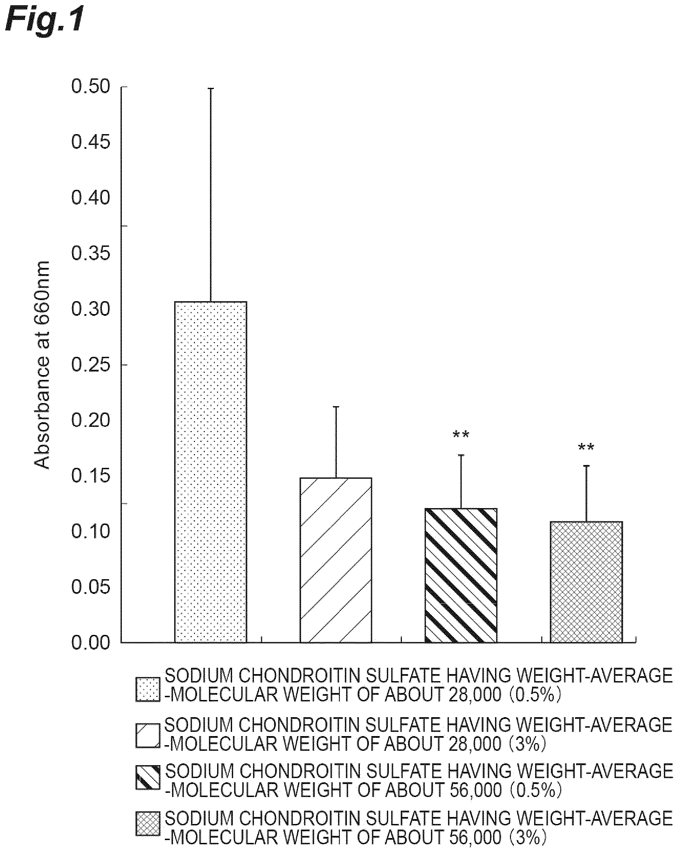

FIG. 1 is a graph showing the results of Test Example 1.

### Description of Embodiments

Hereinafter, embodiments for implementing a first present invention will be described in detail. However, the first present invention is not limited to the following embodiments.

In this specification, unless otherwise specified, the content unit "%" indicates "w/v%" and is synonymous with "g/100 mL."

The ophthalmic composition according to the present embodiment contains at least one selected from the group consisting of chondroitin sulfate and salts thereof which have a weight-average-molecular weight of 40,000 to 70,000 (simply referred to as a "component (A)").

Chondroitin sulfate and salts thereof are not particularly limited as long as they are pharmaceutically, pharmacologically (medicinally) or physiologically acceptable.

Examples of salts of chondroitin sulfate include alkali metal salts and alkaline earth metal salts. Examples of alkali metal salts include sodium salts and potassium salts. Examples of alkaline earth metal salts include magnesium salts and calcium salts.

The chondroitin sulfate and salts thereof are preferably chondroitin sulfate and alkali metal salts of chondroitin sulfate, more preferably chondroitin sulfate and sodium chondroitin sulfate, and still more preferably sodium chondroitin sulfate.

The chondroitin sulfate and salts thereof may be natural products or synthetic products, and generally, chondroitin sulfate and salts thereof derived from animals (preferably mammals, fish, mollusks, etc.; and more preferably cows, sharks, squids, rays, etc.), which are natural products, are preferably used, chondroitin sulfate and salts thereof derived from sharks and/or rays are more preferably used, and chondroitin sulfate and salts thereof derived from sharks are still more preferably used.

Commercially available chondroitin sulfates and salts thereof can be used. The chondroitin sulfates and salts thereof may be used alone or two or more thereof may be used in combination. In addition, as the chondroitin sulfate and salts thereof, chondroitin sulfate and salts thereof which have a weight-average-molecular weight of 40,000 to 70,000 defined in this specification and chondroitin sulfate and salts thereof which have a weight-average-molecular weight outside the range defined in this specification can be used in combination. For example, sodium chondroitin sulfate having a weight-average-molecular weight of 56,000 and sodium chondroitin sulfate having a weight-average-molecular weight of 25,000 can be used in combination. When chondroitin sulfate and salts thereof which have a weight-average-molecular weight of 40,000 to 70,000 defined in this specification and chondroitin sulfate and salts thereof which have a weight-average-molecular weight outside the range defined in this specification are used in combination, chondroitin sulfate and salts thereof which have a weight-average-molecular weight of 40,000 to 70,000 may be contained as raw materials.

In this specification, the "weight-average-molecular weight" can be determined by using gel permeation chromatography in which a multi-angle light scattering detector (MALS detector) and a differential refractive index detector (RI detector) are connected online. Specifically, the following conditions are presented.

### <Preparation of standard sample>

A sample is obtained by adding 10 mL of a 0.1 M sodium nitrate aqueous solution to 5 mg of chondroitin sulfate or salts thereof, gently stirring it at room temperature, and completely dissolving it.

### <Conditions for weight-average-molecular weight measurement>

Device: gel permeation chromatograph-multi-angle light scatterometer
Detector: differential refractive index detector (Optilab rEX commercially available from Wyatt Technology)
Multi-angle light scattering detector (DAWN HELEOS commercially available from Wyatt Technology)
Column: Shodex OHpak SB-806M HQ 2 columns (ϕ7.8 mm×30 cm, commercially available from Showa Denko K.K.)
Solvent: 0.1 M sodium nitrate aqueous solution
Flow rate: 0.7 mL/min
Column temperature: 23°C
Detector temperature: 23°C
Injection volume: 0.2 mL
Data processing: commercially available from Wyatt Technology data processing system (ASTRA)

The weight-average-molecular weight of the chondroitin sulfate and salts thereof calculated by the above method is not particularly limited as long as it is in a range of 40,000 to 70,000. Examples of lower limit values of the weight-average-molecular weight include 41,000 or more, 42,000 or more, 43,000 or more, 44,000 or more, 45,000 or more, 46,000 or more, 47,000 or more, 48,000 or more, 49,000 or more, and 50,000 or more. Examples of upper limit values of the weight-average-molecular weight include 69,000 or less, 68,000 or less, 67,000 or less, 66,000 or less, 65,000 or less, 64,000 or less, 63,000 or less, 62,000 or less, 61,000 or less, and 60,000 or less. Examples of ranges of the weight-average-molecular weight include 41,000 to 69,000, 42,000 to 68,000, 43,000 to 67,000, 44,000 to 66,000, 45,000 to 65,000, 46,000 to 64,000, 47,000 to 63,000, 48,000 to 62,000, 49,000 to 61,000, and 50,000 to 60,000.

The content of the component (A) in the ophthalmic composition according to the present embodiment is not particularly limited, and is appropriately set depending on the types and contents of other formulation components, applications and formulation forms of the ophthalmic composition, and the like. In order to more significantly exhibit the effects of the first present invention, the content of the component (A) based on the total amount of the ophthalmic composition is, for example, generally 0.001 to 5 w/v%, preferably 0.005 to 5 w/v%, more preferably 0.008 to 4 w/v%, still more preferably 0.01 to 3 w/v%, yet more preferably 0.05 to 2 w/v%, particularly preferably 0.1 to 1 w/v%, and most preferably 0.3 to 1 w/v%.

The ophthalmic composition according to the present embodiment may further contain at least one selected from the group consisting of an anti-inflammatory agent, A vitamins, B vitamins, E vitamins, aminoethylsulfonic acid and salts thereof, aspartic acid and salts thereof, neostigmine and salts thereof, and a cellulose-based polymer compound (simply referred to as a "component (B)"). When the ophthalmic composition contains the component (B), the effects of the first present invention are more significantly exhibited.

In addition, as confirmed in test examples to be described below, the ophthalmic composition containing the component (A) and the component (B) according to the present embodiment exhibits an effect of reducing the amount of liquid remaining in pipes during filling of containers or during liquid transfer, an effect of increasing preservative efficacy, an effect of making it easier to blink regardless of the viscosity of a formulation when instilled in the eyes (even if it is a high-viscosity formulation), an effect of reducing discomfort during blinking regardless of the viscosity of a formulation when instilled in the eyes (even if it is a high-viscosity formulation), an effect of reducing a change in viscosity due to daylight, an effect of reducing precipitation (white residue) of contained components, an effect of suppressing coloring of a formulation due to daylight, an effect of suppressing coloring of a formulation due to ultraviolet rays, an effect of reducing a change in appearance (transparency) due to heat, an effect of increasing a cell survival percentage, an effect of reducing eye cell damage due to external stimuli (blinking, stimuli derived from contact lenses (when inserting and removing them, or while wearing them), rubbing the eyes, and contamination with foreign substances (pollen, air contaminants, eyelashes, eye makeup-related foreign substances, and other foreign substances, etc.)), and an effect of reducing the amount of liquid remaining in containers after use.

### [Anti-inflammatory agent]

Anti-inflammatory agents are compounds and salts thereof that have anti-inflammatory effects or antiphlogistic effects. The anti-inflammatory agents are not particularly limited as long as they are pharmaceutically, pharmacologically (medicinally) or physiologically acceptable.

Specific examples of anti-inflammatory agents include epsilon-aminocaproic acid, allantoin, berberine, azulenes (azulene, azulene sulfonic acid, chamazulene, guaiazulene, etc.), glycyrrhizic acid, zinc salts, lysozyme, lysozyme chloride, celecoxib, rofecoxib, indometacin, diclofenac, bromfenac, piroxicam, meloxicam, methyl salicylate, glycol salicylate, tranexamic acid, ibuprofen, ibuprofenpiconol, bufexamac, butyl fulfenamate, bendazac, ketoprofen, felbinac, pranoprofen, and salts thereof. The anti-inflammatory agents are preferably allantoin, glycyrrhizic acid and salts thereof, and zinc salts, and more preferably allantoin, glycyrrhizic acid and salts thereof. The glycyrrhizic acid and salts thereof are preferably alkali metal salts or ammonium salts of glycyrrhizic acid, more preferably dipotassium glycyrrhizinate and monoammonium glycyrrhizinate, and still more preferably dipotassium glycyrrhizinate. The zinc salts are preferably zinc sulfate or zinc lactate, and more preferably zinc sulfate. In addition, the zinc salts may be a hydrate (for example, zinc sulfate heptahydrate).

Commercially available anti-inflammatory agents can be used. The anti-inflammatory agents may be used alone or two or more thereof may be used in combination.

When an anti-inflammatory agent is used as the component (B), the content of the anti-inflammatory agent in the ophthalmic composition according to the present embodiment is not particularly limited, and is appropriately set depending on the type of the anti-inflammatory agent, the types and contents of other formulation components, applications and formulation forms of the ophthalmic composition, and the like. Regarding the content of the anti-inflammatory agent, in order to more significantly exhibit the effects of the first present invention, the total content of the anti-inflammatory agent based on the total amount of the ophthalmic composition is preferably 0.0001 to 10 w/v%, more preferably 0.001 to 5 w/v%, still more preferably 0.005 to 3 w/v%, yet more preferably 0.01 to 1 w/v%, and particularly preferably 0.03 to 0.5 w/v%. In addition, the total content of the anti-inflammatory agent may be 0.25 w/v%.

When an anti-inflammatory agent is used as the component (B), the content proportion of the anti-inflammatory agent with respect to the component (A) in the ophthalmic composition according to the present embodiment is not particularly limited, and is appropriately set depending on the types of the component (A) and the anti-inflammatory agent, the types and contents of other formulation components, applications and formulation forms of the ophthalmic composition, and the like. Regarding the content proportion of the anti-inflammatory agent with respect to the component (A), in order to further improve the effects of the first present invention, for example, the total content of the anti-inflammatory agent based on the total content of 1 part by mass of the component (A) contained in the ophthalmic composition according to the present embodiment is preferably 0.0002 to 1,000 parts by mass, more preferably 0.001 to 500 parts by mass, still more preferably 0.01 to 100 parts by mass, yet more preferably 0.05 to 50 parts by mass, yet more preferably 0.05 to 30 parts by mass, and particularly preferably 0.1 to 10 parts by mass. In addition, the total content of the anti-inflammatory agent based on the total content of 1 part by mass of the component (A) may be 0.5 parts by mass.

### [A vitamins]

A vitamins are not particularly limited as long as they are pharmaceutically, pharmacologically (medicinally) or physiologically acceptable. Specific examples of A vitamins include retinol, retinal, retinoic acid, derivatives thereof, and salts thereof.

Examples of derivatives of A vitamins include esters with monovalent carboxylic acids such as retinol palmitate, retinol acetate, retinol butyrate, retinol propionate, retinol octylate, retinol laurate, retinol oleate and retinol linoleate.

Examples of salts of A vitamins include organic acid salts [for example, monocarboxylates (acetate, trifluoroacetate, butyrate, palmitate, stearate, etc.), polycarboxylates (fumarate, maleate, succinate, malonate, etc.), oxycarboxylates (lactate, tartrate, citrate, etc.), organic sulfonates (methane sulfonate, toluene sulfonate, tosylate, etc.), etc.], inorganic acid salts (for example, hydrochloride, sulfate, nitrate, hydrobromide, phosphate, etc.), salts with organic bases (for example, salts with organic amines such as methylamine, triethylamine, triethanolamine, morpholine, piperazine, pyrrolidine, tripyridine, and picoline, etc.), salts with inorganic bases [for example, ammonium salts; salts with metals such as alkali metals (sodium, potassium, etc.), alkaline earth metals (calcium, magnesium, etc.), and aluminum, etc.], and the like.

The A vitamins are preferably derivatives of retinol, more preferably esters of retinol and monovalent carboxylic acids, still more preferably retinol palmitate and retinol acetate, and yet more preferably retinol palmitate.

As the A vitamins, synthetic products may be used or extracts obtained from natural products (for example, a vitamin A oil, etc.) may be used. The vitamin A oil is a fatty oil obtained from animal tissues containing retinol, or its concentrate, or one obtained by appropriately adding a vegetable oil thereto. Commercially available A vitamins can be used. The A vitamins may be used alone or two or more thereof may be used in combination.

Regarding the content of the A vitamins in the ophthalmic composition according to the present embodiment, the total content of the A vitamins based on the total amount of the ophthalmic composition is preferably 1,000 to 300,000 IU/100 mL, more preferably 5,000 to 300,000 IU/100 mL, still more preferably 10,000 to 100,000 IU/100 mL, yet more preferably 30,000 to 55,000 IU/100 mL, yet more preferably 35,000 to 55,000 IU/100 mL, and particularly preferably 45,000 to 55,000 IU/100 mL.

"IU" is an international unit determined by the method described in the 17th revised Japanese Pharmacopoeia, Vitamin A Assay Method, etc. For example, the pharmaceutical segments of the 17th revised Japanese Pharmacopoeia describe that retinol acetate contains at least 2,500,000 units of vitamin A per 1 g, and retinol palmitate contains at least 1,500,000 units of vitamin A per 1 g.

When A vitamins are used as the component (B), the content proportion of the A vitamins with respect to the component (A) in the ophthalmic composition according to the present embodiment is not particularly limited, and is appropriately set depending on the types of the component (A) and the A vitamins, the types and contents of other formulation components, applications and formulation forms of the ophthalmic composition, and the like. Regarding the content proportion of the A vitamins with respect to the component (A), in order to further improve the effects of the first present invention, for example, the total content of the A vitamins based on the total content of 1 part by mass of the component (A) contained in the ophthalmic composition according to the present embodiment is preferably 1,000 to 3,000,000 IU/g, more preferably 10,000 to 1,000,000 IU/g, still more preferably 35,000 to 550,000 IU/g, and yet more preferably 45,000 to 550,000 IU/g.

### [B vitamins]

B vitamins are not particularly limited as long as they are pharmaceutically, pharmacologically (medicinally) or physiologically acceptable.

Examples of specific examples of B vitamins include flavin adenine dinucleotide and salts thereof (flavin adenine dinucleotide sodium, etc.), cobalamins (cyanocobalamin, methylcobalamin, etc.), pantothenic acid and salts thereof (for example, sodium pantothenate, potassium pantothenate, calcium pantothenate, magnesium pantothenate, etc.), panthenol, pyridoxine or salts thereof (pyridoxine hydrochloride, etc.), and pyridoxal and salts thereof (pyridoxal phosphate, etc.). The B vitamins are preferably panthenol, pyridoxine or salts thereof.

Commercially available B vitamins can be used. The B vitamins may be used alone or two or more thereof may be used in combination.

When B vitamins are used as the component (B), the content of the B vitamins in the ophthalmic composition according to the present embodiment is not particularly limited, and is appropriately set depending on the type of the B vitamins, the types and contents of other formulation components, applications and formulation forms of the ophthalmic composition, and the like. Regarding the content of the B vitamins, in order to more significantly exhibit the effects of the first present invention, the total content of the B vitamins based on the total amount of the ophthalmic composition is preferably 0.0001 to 5 w/v%, more preferably 0.0005 to 1 w/v%, still more preferably 0.001 to 1 w/v%, yet more preferably 0.005 to 0.5 w/v%, and particularly preferably 0.01 to 0.1 w/v%.

When B vitamins are used as the component (B), the content proportion of the B vitamins with respect to the component (A) in the ophthalmic composition according to the present embodiment not particularly limited, and is appropriately set depending on the types of the component (A) and the B vitamins, the types and contents of other formulation components, applications and formulation forms of the ophthalmic composition, and the like. Regarding the content proportion of the B vitamins with respect to the component (A), in order to further improve the effects of the first present invention, for example, the total content of the B vitamins based on the total content of 1 part by mass of the component (A) contained in the ophthalmic composition according to the present embodiment is preferably 0.00002 to 1,000 parts by mass, more preferably 0.0001 to 500 parts by mass, still more preferably 0.001 to 100 parts by mass, yet more preferably 0.005 to 50 parts by mass, yet more preferably 0.01 to 30 parts by mass, and particularly preferably 0.05 to 1 part by mass.

### [E vitamins]

E vitamins are not particularly limited as long as they are pharmaceutically, pharmacologically (medicinally) or physiologically acceptable. Specific examples of E vitamins include tocopherol, tocotrienol, derivatives thereof, and salts thereof. Tocopherol and tocotrienol may be of any of α-, β-, γ-, and δ-, or may be a d-form or dl-form.

Examples of derivatives of E vitamins include esters with organic acids such as tocopherol acetate, tocopherol succinate, tocopherol nicotinate, and tocopherol linolenate.

Examples of salts of E vitamins include organic acid salts (lactate, acetate, butyrate, trifluoroacetate, fumarate, maleate, tartrate, citrate, succinate, malonate, methane sulfonate, toluene sulfonate, tosylate, palmitate, stearate, etc.), inorganic acid salts (for example, hydrochloride, sulfate, nitrate, hydrobromide, phosphate, etc.), salts with organic bases (for example, salts with organic amines such as methylamine, triethylamine, triethanolamine, morpholine, piperazine, pyrrolidine, amino acid, tripyridine, and picoline, etc.), and salts with inorganic bases (for example, ammonium salts, salts with metals such as alkali metals such as sodium and potassium, alkaline earth metals such as calcium, and magnesium, aluminum, etc.).

The E vitamins are preferably d-α-tocopherol, dl-α-tocopherol, β-tocopherol, γ-tocopherol, δ-tocopherol, vitamin E acetate (for example, tocopherol acetate), vitamin E nicotinate, vitamin E succinate, and vitamin E linolenate, and more preferably tocopherol acetate (for example, d-α-tocopherol acetate, dl-α-tocopherol acetate, etc.).

The E vitamins may be natural products or synthetic products. Commercially available E vitamins can be used. The E vitamins may be used alone or two or more thereof may be used in combination.

Regarding the content of the E vitamins in the ophthalmic composition according to the present embodiment, the total content of the E vitamins based on the total amount of the ophthalmic composition is preferably 0.0001 to 0.5 w/v%, more preferably 0.001 to 0.1 w/v%, still more preferably 0.005 to 0.05 w/v%, and yet more preferably 0.01 to 0.05 w/v%.

When E vitamins are used as the component (B), the content proportion of the E vitamins with respect to the component (A) in the ophthalmic composition according to the present embodiment is not particularly limited, and is appropriately set depending on the types of the component (A) and the E vitamins, the types and contents of other formulation components, applications and formulation forms of the ophthalmic composition, and the like. Regarding the content proportion of the E vitamins with respect to the component (A),in order to further improve the effects of the first present invention, for example, the total content of the E vitamins based on the total content of 1 part by mass of the component (A) contained in the ophthalmic composition according to the present embodiment is preferably 0.0001 to 50 parts by mass, more preferably 0.001 to 20 parts by mass, still more preferably 0.005 to 10 parts by mass, yet more preferably 0.01 to 5 parts by mass, and particularly preferably 0.01 to 0.5 parts by mass.

### [Aminoethylsulfonic acid and salts thereof]

Aminoethylsulfonic acid (taurine) and salts thereof are not particularly limited as long as they are pharmaceutically, pharmacologically (medicinally) or physiologically acceptable.

Examples of salts of aminoethylsulfonic acid include salts with organic bases (for example, salts with organic amines such as methylamine, triethylamine, triethanolamine, morpholine, piperazine, pyrrolidine, tripyridine, and picoline), salts with inorganic bases [for example, ammonium salts, and salts with metals such as alkali metals (sodium, potassium, etc.), alkaline earth metals (calcium, magnesium, etc.), and aluminum].

The aminoethylsulfonic acids and salts thereof are preferably aminoethylsulfonic acid.

Commercially available aminoethylsulfonic acids and salts thereof can be used. The aminoethylsulfonic acids and salts thereof may be used alone or two or more thereof may be used in combination.

Regarding the content of the aminoethylsulfonic acids and salts thereof in the ophthalmic composition according to the present embodiment, the total content of the aminoethylsulfonic acids and salts thereof based on the total amount of the ophthalmic composition is preferably 0.001 to 10 w/v%, more preferably 0.01 to 5 w/v%, still more preferably 0.05 to 3 w/v%, and yet more preferably 0.1 to 2 w/v%.

When aminoethylsulfonic acids and salts thereof are used as the component (B), the content proportion of the aminoethylsulfonic acids and salts thereof with respect to the component (A) in the ophthalmic composition according to the present embodiment is not particularly limited, and is appropriately set depending on the types of the component (A) and the aminoethylsulfonic acids and salts thereof, the types and contents of other formulation components, applications and formulation forms of the ophthalmic composition, and the like. Regarding the content proportion of the aminoethylsulfonic acids and salts thereof with respect to the component (A), in order to further improve the effects of the first present invention, for example, the total content of the aminoethylsulfonic acids and salts thereof based on the total content of 1 part by mass of the component (A) contained in the ophthalmic composition according to the present embodiment is preferably 0.001 to 1,000 parts by mass, more preferably 0.01 to 200 parts by mass, still more preferably 0.05 to 100 parts by mass, yet more preferably 0.1 to 20 parts by mass, and particularly preferably 0.1 to 10 parts by mass.

### [Aspartic acid and salts thereof]

Aspartic acid is a compound known as an acidic amino acid, also called 2-aminobutanedioic acid. The aspartic acids and salts thereof are not particularly limited as long as they are pharmaceutically, pharmacologically (medicinally) or physiologically acceptable. Aspartic acid may be any of L-form, D-form, and DL-form, and is preferably an L-form.

Examples of salts of aspartic acid include salts with inorganic bases (for example, ammonium salts; salts with metals such as alkali metals (sodium, potassium, etc.), alkaline earth metals (calcium, magnesium, etc.), and aluminum, etc.), and salts with organic bases (for example, salts with organic amines such as methylamine, triethylamine, triethanolamine, morpholine, piperazine, pyrrolidine, tripyridine, and picoline, etc.). The aspartic acids and salts thereof are preferably salts of aspartic acid with inorganic bases, more preferably alkali metal salts and alkaline earth metal salts of aspartic acid, and still more preferably potassium aspartate, magnesium aspartate and magnesium/potassium aspartate.

Commercially available aspartic acids and salts thereof can be used. The aspartic acids and salts thereof may be used alone or two or more thereof may be used in combination.

The content of the aspartic acids and salts thereof in the ophthalmic composition according to the present embodiment is not particularly limited, and is appropriately set depending on the types and contents of other formulation components, the formulation forms and the like. Regarding the content of the aspartic acids or salts thereof, for example, the total content of the aspartic acids or salts thereof based on the total amount of the ophthalmic composition is preferably 0.001 to 10 w/v%, more preferably 0.01 to 5 w/v%, still more preferably 0.05 to 3 w/v%, and yet more preferably 0.1 to 2 w/v%.

When aspartic acids and salts thereof are used as the component (B), the content proportion of the aspartic acids and salts thereof with respect to the component (A) in the ophthalmic composition according to the present embodiment is not particularly limited, and is appropriately set depending on the types of the component (A) and the aspartic acids and salts thereof, the types and contents of other formulation components, applications and formulation forms of the ophthalmic composition, and the like. Regarding the content proportion of the aspartic acids and salts thereof with respect to the component (A), in order to further improve the effects of the first present invention, for example, the total content of the aspartic acids and salts thereof based on the total content of 1 part by mass of the component (A) contained in the ophthalmic composition according to the present embodiment is preferably 0.001 to 1,000 parts by mass, more preferably 0.01 to 300 parts by mass, still more preferably 0.05 to 200 parts by mass, yet more preferably 0.1 to 50 parts by mass, and particularly preferably 0.1 to 20 parts by mass.

### [Neostigmine and salts thereof]

Neostigmine and salts thereof are not particularly limited as long as they are pharmaceutically, pharmacologically (medicinally) or physiologically acceptable. Examples of salts of neostigmine include neostigmine methyl sulfate. Neostigmine and salts thereof are preferably neostigmine methyl sulfate.

Commercially available neostigmine and salts thereof can be used. The neostigmine and salts thereof may be used alone or two or more thereof may be used in combination.

Regarding the content of the neostigmine and salts thereof in the ophthalmic composition according to the present embodiment, the total content of the neostigmine and salts thereof based on the total amount of the ophthalmic composition is preferably 0.0001 to 0.05 w/v%, more preferably 0.0005 to 0.01 w/v%, still more preferably 0.0008 to 0.008 w/v%, and yet more preferably 0.001 to 0.005 w/v%.

When neostigmine and salts thereof are used as the component (B), the content proportion of the neostigmine and salts thereof with respect to the component (A) in the ophthalmic composition according to the present embodiment is not particularly limited, and is appropriately set depending on the types of the component (A) and neostigmine and salts thereof, the types and contents of other formulation components, applications and formulation forms of the ophthalmic composition, and the like. Regarding the content proportion of the neostigmine and salts thereof with respect to the component (A), in order to further improve the effects of the first present invention, for example, the total content of the neostigmine and salts thereof based on the total content of 1 part by mass of the component (A) contained in the ophthalmic composition according to the present embodiment is preferably 0.0001 to 5 parts by mass, more preferably 0.0001 to 1 part by mass, still more preferably 0.0005 to 0.8 parts by mass, yet more preferably 0.001 to 0.5 parts by mass, and particularly preferably 0.001 to 0.05 parts by mass.

### [Cellulose-based polymer compound]

Cellulose-based polymer compounds are not particularly limited as long as they are pharmaceutically, pharmacologically (medicinally) or physiologically acceptable.

Examples of cellulose-based polymer compounds include methylcellulose, ethylcellulose, hydroxyethyl cellulose, hydroxymethylcellulose, hydroxypropyl cellulose, hydroxypropyl methylcellulose (hypromellose), carboxymethylcellulose, carboxyethylcellulose, and salts thereof. The cellulose-based polymer compounds are preferably hydroxyethyl cellulose, hydroxypropyl methylcellulose, and salts thereof, and more preferably hydroxypropyl methylcellulose and salts thereof. Examples of such examples include salts with organic bases (amine salts, basic ammonium salts such as arginine, etc.), salts with inorganic bases (alkali metal salts such as ammonium salts, sodium salts, and potassium salts, alkaline earth metal salts such as calcium salts and magnesium salts, aluminum salts, etc.) and the like, and among these, sodium salts, potassium salts, and calcium salts are more preferable, and sodium salts are particularly preferable.

Commercially available cellulose-based polymer compounds can be used. The cellulose-based polymer compounds may be used alone or two or more thereof may be used in combination.

When a cellulose-based polymer compound is used as the component (B), the content of the cellulose-based polymer compound in the ophthalmic composition according to the present embodiment is not particularly limited, and is appropriately set depending on the type of the cellulose-based polymer compound, the types and contents of other formulation components, applications and formulation forms of the ophthalmic composition, and the like. Regarding the content of the cellulose-based polymer compound, in order to more significantly exhibit the effects of the first present invention, the total content of the cellulose-based polymer compound based on the total amount of the ophthalmic composition is preferably 0.0001 to 10 w/v%, more preferably 0.001 to 5 w/v%, still more preferably 0.005 to 3 w/v%, and yet more preferably 0.01 to 2 w/v%.

When a cellulose-based polymer compound is used as the component (B), the content proportion of the cellulose-based polymer compound with respect to the component (A) in the ophthalmic composition according to the present embodiment is not particularly limited, and is appropriately set depending on the types of the component (A) and the cellulose-based polymer compound, the types and contents of other formulation components, applications and formulation forms of the ophthalmic composition, and the like. Regarding the content proportion of the cellulose-based polymer compound with respect to the component (A), in order to further improve the effects of the first present invention, for example, the total content of the cellulose-based polymer compound based on the total content of 1 part by mass of the component (A) contained in the ophthalmic composition according to the present embodiment is preferably 0.0001 to 500 parts by mass, more preferably 0.001 to 100 parts by mass, still more preferably 0.005 to 50 parts by mass, and yet more preferably 0.01 to 30 parts by mass.

The ophthalmic composition according to the present embodiment may further contain a surfactant (C) (also referred to as a "component (C)"). When the ophthalmic composition further contains the component (C), the effects of the first present invention are more significantly exhibited. The surfactants are not particularly limited as long as they are pharmaceutically, pharmacologically (medicinally) or physiologically acceptable, and may be any of nonionic surfactants, amphoteric surfactants, anionic surfactants, and cationic surfactants.

Examples of nonionic surfactants include POE sorbitan fatty acid esters such as monolaurate POE(20) sorbitan (polysorbate 20), monopalmitate POE(20) sorbitan (polysorbate 40), monostearate POE(20) sorbitan (polysorbate 60), tristearate POE(20) sorbitan (polysorbate 65), and monooleate POE(20) sorbitan (polysorbate 80); POE hydrogenated castor oils such as POE(40) hydrogenated castor oil (polyoxyethylene hydrogenated castor oil 40), and POE(60) hydrogenated castor oil (polyoxyethylene hydrogenated castor oil 60); POE castor oils such as POE(3) hydrogenated castor oil (polyoxyethylene castor oil 3), POE(10) castor oil (polyoxyethylene castor oil 10), and POE(35) castor oil (polyoxyethylene castor oil 35); POE alkyl ethers such as POE(9) lauryl ether; POE-POP alkyl ethers such as POE(20)POP(4) cetyl ether; polyoxyethylene/polyoxypropylene block copolymers such as POE(196)POP(67) glycol (poloxamer 407, Pluronic F127), and POE(200)POP(70) glycol, and monostearate polyethylene glycol such as polyoxyl stearate 40. Here, in the compounds exemplified above, POE indicates polyoxyethylene, POP indicates polyoxypropylene, and the number in parentheses indicates the number of moles added.

Examples of amphoteric surfactants include alkyldiaminoethylglycine and salts thereof (for example, hydrochloride, etc.).

Examples of anionic surfactants include alkylbenzene sulfonate, alkyl sulfate, polyoxyethylene alkyl sulfate, aliphatic α-sulfomethyl ester, and α-olefin sulfonic acid.

Examples of cationic surfactants include cetylpyridinium chloride, benzalkonium chloride, and benzethonium chloride.

Among these surfactants, nonionic surfactants are preferable, and POE sorbitan fatty acid esters, POE hydrogenated castor oils, POE castor oils, and POE/POP block copolymers are more preferable. Commercially available surfactants can be used. The surfactants may be used alone or two or more thereof may be used in combination.

The content of the component (C) in the ophthalmic composition according to the present embodiment is not particularly limited, and is appropriately set depending on the type of the component (C), applications and formulation forms of the ophthalmic composition, and the like. Regarding the content of the component (C), in order to more significantly exhibit the effects of the first present invention, for example, the total content of the component (C) based on the total amount of the ophthalmic composition is preferably 0.001 to 3 w/v%, more preferably 0.005 to 2 w/v%, still more preferably 0.01 to 1 w/v%, and particularly preferably 0.05 to 1 w/v%.

The content proportion of the component (C) with respect to the component (A) in the ophthalmic composition according to the present embodiment is not particularly limited, and is appropriately set depending on the types of the component (A) and the component (C), the types and contents of other formulation components, applications and formulation forms of the ophthalmic composition, and the like. Regarding the content proportion of the component (C) with respect to the component (A), in order to further improve the effects of the first present invention, for example, the total content of the component (C) based on the total content of 1 part by mass of the component (A) contained in the ophthalmic composition according to the present embodiment is more preferably 0.001 parts by mass to 30 parts by mass, more preferably 0.005 to 20 parts by mass, still more preferably 0.01 to 10 parts by mass, and particularly preferably 0.01 to 2 parts by mass.

The ophthalmic composition according to the present embodiment preferably further contains a buffer (D) (also referred to as a "component (D)"). When the ophthalmic composition further contains the component (D), the effects of the first present invention are more significantly exhibited. The buffers are not particularly limited as long as they are pharmaceutically, pharmacologically (medicinally) or physiologically acceptable. Examples of buffers include inorganic buffers that are buffers derived from inorganic acids and organic buffers that are buffers derived from organic acids or organic bases.

Examples of inorganic buffers include borate buffers, phosphate buffers, and carbonate buffers. Examples of borate buffers include boric acid or salts thereof (alkali metal borates, alkaline earth metal borates, etc.). Examples of phosphate buffers include phosphoric acid or salts thereof (alkali metal phosphates, alkaline earth metal phosphates, etc.). Examples of carbonate buffers include carbonic acid or salts thereof (alkali metal carbonates, alkaline earth metal carbonates, etc.). In addition, as the borate buffers, phosphate buffers or carbonate buffers, borate, phosphate or carbonate hydrates may be used. As more specific examples, examples of borate buffers include boric acid or salts thereof (sodium borate, potassium tetraborate, potassium metaborate, ammonium borate, borax, etc.); examples of phosphate buffers include phosphoric acid or salts thereof (disodium hydrogen phosphate, sodium dihydrogen phosphate, potassium dihydrogen phosphate, trisodium phosphate, tripotassium phosphate, calcium monohydrogen phosphate, calcium dihydrogen phosphate, etc.); and examples of carbonate buffers include carbonic acid or salts thereof (sodium bicarbonate, sodium carbonate, ammonium carbonate, potassium carbonate, calcium carbonate, potassium bicarbonate, magnesium carbonate, etc.).

Examples of organic buffers include citrate buffers, acetate buffers, lactate buffers, succinate buffers, tris buffers, and AMPD buffers. Examples of citrate buffers include citric acid or salts thereof (alkali metal citrate, alkaline earth metal citrate, etc.). Examples of acetate buffers include acetic acid or salts thereof (alkali metal acetate, alkaline earth metal acetate, etc.). Examples of lactate buffers include lactic acid or salts thereof (alkali metal lactate, alkaline earth metal lactate, etc.). Examples of succinate buffers include succinic acid or salts thereof (succinic acid alkali metal salt, etc.). In addition, as the citrate buffers, acetate buffers, lactate buffers or succinate buffers, citrate, acetate, lactate or succinate hydrates may be used. As more specific examples, examples of citrate buffers include citric acid or salts thereof (sodium citrate, potassium citrate, calcium citrate, sodium dihydrogen citrate, disodium citrate, etc.); examples of acetate buffers include acetic acid or salts thereof (ammonium acetate, sodium acetate, potassium acetate, calcium acetate, etc.); examples of lactate buffers include lactic acid or salts thereof (sodium lactate, potassium lactate, calcium lactate, etc.); and examples of succinate buffers include succinic acid or salts thereof (monosodium succinate, disodium succinate, etc.). Examples of tris buffers include trometamol or salts thereof (trometamol hydrochloride, etc.). Examples of AMPD buffers include 2-amino-2-methyl-1,3-propanediol or salts thereof.

The buffers are preferably borate buffers (for example, a combination of boric acid and borax, etc.), phosphate buffers (for example, a combination of disodium hydrogen phosphate and sodium dihydrogen phosphate, etc.), and tris buffers (for example, trometamol), more preferably borate buffers, still more preferably boric acids and salts thereof, and yet more preferably a combination of boric acid and borax.

Commercially available buffers may be used. The buffers may be used alone or two or more thereof may be used in combination.

The content of the component (D) in the ophthalmic composition according to the present embodiment is not particularly limited, and is appropriately set depending on the type of the component (D), the types and contents of other formulation components, applications and formulation forms of the ophthalmic composition, and the like. Regarding the content of the component (D), in order to more significantly exhibit the effects of the first present invention, for example, the total content of the component (D) based on the total amount of the ophthalmic composition is preferably 0.01 to 10 w/v%, more preferably 0.05 to 5 w/v%, and still more preferably 0.1 to 3 w/v%.

The content proportion of the component (D) with respect to the component (A) in the ophthalmic composition according to the present embodiment is not particularly limited, and is appropriately set depending on the types of the component (A) and the component (D), the types and contents of other formulation components, applications and formulation forms of the ophthalmic composition, and the like. Regarding the content proportion of the component (D) with respect to the component (A), in order to further improve the effects of the first present invention, for example, the total content of the component (D) based on the total content of 1 part by mass of the component (A) contained in the ophthalmic composition according to the present embodiment is preferably 0.01 to 100 parts by mass, more preferably 0.05 to 50 parts by mass, and still more preferably 0.1 to 30 parts by mass.

The pH of the ophthalmic composition according to the present embodiment is not particularly limited as long as it is within a pharmaceutically, pharmacologically (medicinally) or physiologically acceptable range. The pH of the ophthalmic composition according to the present embodiment may be, for example, 4.0 to 9.5, and is preferably 4.0 to 9.0, more preferably 4.5 to 9.0, still more preferably 4.5 to 8.5, yet more preferably 5.0 to 8.5, particularly preferably 5.0 to 8.0, more particularly preferably 5.3 to 7.5, and most preferably 5.3 to 7.0.

The osmotic pressure ratio of the ophthalmic composition according to the present embodiment can be adjusted, as necessary, to be within a range in which it is acceptable in living organisms. The appropriate osmotic pressure ratio can be appropriately set depending on applications, formulation forms, usage methods of the ophthalmic composition, and the like, and it may be, for example, 0.4 to 5.0, and is preferably 0.6 to 3.0, more preferably 0.8 to 2.2, and still more preferably 0.8 to 2.0. The osmotic pressure ratio is a ratio of the osmotic pressure of the sample to 286 mOsm (the osmotic pressure of a 0.9 w/v% sodium chloride aqueous solution) based on the 17th revised Japanese Pharmacopoeia, and the osmotic pressure is measured with reference to the osmotic pressure measurement method (freezing point depression method) described in the Japanese Pharmacopoeia. Here, a standard solution for osmotic pressure ratio measurement (0.9 w/v% sodium chloride aqueous solution) is prepared by drying sodium chloride (Japanese Pharmacopoeia standard reagent) at 500 to 650°C for 40 to 50 minutes and then cooling it in a dessicator (silica gel), and accurately weighing out 0.900 g of the sample and dissolving it in purified water to make exactly 100 mL, and a commercially available standard solution for osmotic pressure ratio measurement (0.9 w/v% sodium chloride aqueous solution) can be used.

The viscosity of the ophthalmic composition according to the present embodiment is not particularly limited as long as it is within a pharmaceutically, pharmacologically (medicinally) or physiologically acceptable range. Regarding the viscosity of the ophthalmic composition according to the present embodiment, for example, the viscosity measured at 20°C using a rotational viscometer (TV-20 type viscometer, commercially available from Toki Sangyo Co., Ltd., rotor; 1°34'×R24) is preferably 1 to 10,000 mPals, more preferably 1 to 8,000 mPa/s, still more preferably 1 to 1,000 mPa/s, yet more preferably 1 to 100 mPa/s, particularly preferably 1 to 20 mPa/s, and most preferably 1.5 to 10 mPals.

The ophthalmic composition according to the present embodiment may contain an appropriate amount of one or more components selected from among various pharmacologically active components and physiologically active components in addition to the above components as long as the effects of the first present invention are not impaired. The components are not particularly limited, and examples thereof include active components in ophthalmic drugs described in Guidance required/Over-the-Counter Drug Manufacturing and Sales Approval Standards, 2017 Edition (supervised by general incorporated association, the Society of Regulatory Science). Specific examples of components used in ophthalmic drugs include the following components.
Antiallergic agents: for example, sodium cromoglicate, tranilast, potassium pemirolast, acitazanolast, amlexanox, ibudilast, etc.
Antihistamines: for example, diphenhydramine or salts thereof (for example, diphenhydramine hydrochloride), iproheptine or salts thereof (for example, iproheptine hydrochloride), chlorpheniramine or salts thereof (for example, chlorpheniramine maleate), levocabastine or salts thereof (for example, levocabastine hydrochloride), ketotifen or salts thereof (for example, ketotifen fumarate), potassium pemirolast, olopatadine or salts thereof (for example, olopatadine hydrochloride), etc.
Steroids: for example, fluticasone propionate, fluticasone furoate, mometasone furoate, beclomethasone propionate, flunisolide, etc. Decongestants: for example, tetrahydrozoline hydrochloride, tetrahydrozoline nitrate, naphazoline hydrochloride, naphazoline nitrate, epinephrine, epinephrine hydrochloride, ephedrine hydrochloride, phenylephrine hydrochloride, dl-methylephedrine hydrochloride, etc. Eye muscle regulating agents: for example, cholinesterase inhibitors having an active center similar to acetylcholine, specifically tropicamide, helenien, atropine sulfate, pilocarpine hydrochloride, etc.
Vitamins: for example, ascorbic acid, sodium ascorbate, etc.
Amino acids: for example, L-arginine, glutamic acid, glycine, alanine, lysine, γ-aminobutyric acid, γ-aminovaleric acid, trimethylglycine and salts thereof, etc.
Astringents: for example, zinc oxide, etc.
Others: for example, sulfamethoxazole, sulfisoxazole, sulfisomidine and salts thereof, etc.

In the ophthalmic composition according to the present embodiment, as long as the effects of the first present invention are not impaired, depending on applications and formulation forms, various additives are appropriately selected using a general method, and one or a combination of two or more thereof may be contained in an appropriate amount. Examples of such additives include various additives described in Pharmaceutical Excipients Dictionary 2016 (edited by the Japan Pharmaceutical Excipients Association). Examples of typical components include the following additives.
Carriers: for example, aqueous solvent such as water or water-containing ethanol.
Chelating agents: for example, ethylenediaminediacetic acid (EDDA), ethylenediaminetriacetic acid, ethylenediaminetetraacetic acid (EDTA), N-(2-hydroxyethyl)ethylenediaminetriacetic acid (HEDTA), diethylenetriaminepentaacetic acid (DTPA), etc.
Bases: for example, octyldodecanol, titanium oxide, potassium bromide, plastibase, etc.
pH regulating agents: for example, hydrochloric acid, acetic acid, sodium hydroxide, potassium hydroxide, calcium hydroxide, magnesium hydroxide, triethanolamine, monoethanolamine, diisopropanolamine, etc.
Fragrances or cooling agents: for example, menthol, menthone, camphor, borneol, geraniol, cineole, citronellol, carvone, anethole, eugenol, limonene, linalol, linalyl acetate, thymol, cymene, terpineol, pinene, camphene, isoborneol, fenchone, nerol, myrcene, myrcenol, linalyl acetate, lavandulol, eucalyptus oil, bergamot oil, peppermint oil, cool mint oil, spearmint oil, mint oil, fennel oil, cinnamon oil, rose oil, camphor oil, etc. These may be any of a d-form, l-form and dl-form.
Thickeners: for example, polyvinyl-based polymer compounds such as polyvinylpyrrolidone and polyvinyl alcohol; carboxyvinyl polymers; guar gum; hydroxypropyl guar gum; gum arabic; karaya gum; xanthan gum; agar; alginic acid and salts thereof (sodium salts, etc.); heparin analogs, heparin, heparin sulfate, heparan sulfate, heparinoid, and mucopolysaccharides of hyaluronic acid and salts thereof (sodium salts, etc.); starch; chitin and derivatives thereof; chitosan and derivatives thereof; carrageenan; monosaccharides such as glucose, etc.
Stabilizers: for example, edetic acid, edetate salts (edetate disodium, calcium disodium edetate, trisodium edetate, and tetrasodium edetate), sodium formaldehyde sulfoxylate (rongalite), monostearate aluminum, monostearate glycerin, cyclodextrin, monoethanolamine, dibutyl hydroxy toluene, sodium bisulfite, sodium pyrosulfite, etc.
Preservatives: for example, alkyl polyaminoethylglycine quaternary ammonium salts (for example, benzalkonium chloride, benzethonium chloride, etc.), chlorhexidine gluconate, polydronium chloride, zinc chloride, sodium benzoate, ethanol, chlorobutanol, sorbic acid, potassium sorbate, sodium dehydroacetate, methyl paraoxybenzoate, ethyl paraoxybenzoate, propyl paraoxybenzoate, butyl paraoxybenzoate, oxyquinoline sulfate, phenethyl alcohol, benzyl alcohol, biguanide compounds (specifically, polyhexanide hydrochloride (polyhexamethylene biguanide), alexidine, etc.), Glokill (product name, commercially available from Rhodia), etc.
Isotonic agents: for example, potassium chloride, calcium chloride, sodium chloride, magnesium chloride, potassium acetate, sodium acetate, sodium bicarbonate, sodium carbonate, sodium thiosulfate, magnesium sulfate, glycerin, propylene glycol, sodium bisulfite, sodium sulfite, etc.
Sugar alcohols: for example, xylitol, sorbitol, mannitol, glycerin, etc. These may be any of d-form, l-form and dl-form.
Oils: for example, vegetable oils such as sesame oil, castor oil, soybean oil, and olive oil; animal oils such as squalane; and mineral oils such as liquid paraffin and petrolatum.

It is preferable that the ophthalmic composition according to the present embodiment not contain 0.01% or more of at least one selected from the group consisting of geraniol, linalyl acetate, limonene, citral and linalool, and it is more preferable that the ophthalmic composition not contain them because it is possible to significantly exhibit the effects of the first present invention

When the ophthalmic composition according to the present embodiment contains water, regarding the content of water, in order to more significantly exhibit the effects of the first present invention, for example, the content of water based on the total amount of the ophthalmic composition is preferably 80 w/v% or more and less than 100 w/v%, more preferably 85 w/v% or more and 99.5 w/v% or less, and still more preferably 90 w/v% or more and 99.2 w/v% or less.

The water used in the ophthalmic composition according to the present embodiment may be any water that is pharmaceutically, pharmacologically (medicinally) or physiologically acceptable. Examples of such water include distilled water, regular water, purified water, sterile purified water, water for injection and distilled water for injection. These definitions are based on the 17th revised Japanese Pharmacopoeia.

The ophthalmic composition according to the present embodiment can be prepared by adding and mixing a desired amount of the component (A), and as necessary, other components such as the component (B) to a desired concentration. For example, it can be prepared by dissolving or dispersing these components in purified water, adjusting the pH and osmotic pressure to a predetermined value, and performing a sterilization treatment by filtration sterilization or the like.

The ophthalmic composition according to the present embodiment can take various formulation forms depending on the purpose. Examples of formulation forms include liquid preparations, gel preparations, and semi-solid preparations (ointments, etc.).

The ophthalmic composition according to the present embodiment can be used as, for example, eye drops (eye lotions or ophthalmic preparations; here the eye drops include an eye drop that can be instilled in the eyes wearing contact lenses), artificial tears, eyewashes (also called eyewash liquids or eyewash preparations; here the eyewashes include an eyewash that can wash the eyes wearing contact lenses), and contact lens compositions [a contact lens wearing liquid, a contact lens care composition (a contact lens disinfectant, a contact lens preservative, a contact lens cleaning agent, a contact lens cleaning preservative), a contact lens packaging liquid, etc.]. Here, "contact lenses" include hard contact lenses and soft contact lenses (including both ionic and non-ionic lenses and both silicone hydrogel contact lenses and non-silicone hydrogel contact lenses).

The ophthalmic composition according to the present embodiment has an effect of reducing eye dryness, and therefore can be suitably used as an ophthalmic composition for reducing eye dryness. Therefore, one embodiment of the first present invention provides an ophthalmic composition for reducing eye dryness containing at least one selected from the group consisting of chondroitin sulfate and salts thereof which have a weight-average-molecular weight of 40,000 to 70,000. Here, in this specification, the "effect of reducing eye dryness" is thought to be due to the effect of alleviating eye cell damage caused by dryness, the effect of improving affinity for contact lenses or the like, rather than the effect of promoting lachrymal fluid secretion.

In addition, one embodiment of the first present invention provides a method of reducing eye dryness using at least one selected from the group consisting of chondroitin sulfate and salts thereof which have a weight-average-molecular weight of 40,000 to 70,000. In addition, one embodiment of the first present invention provides a use of at least one selected from the group consisting of chondroitin sulfate and salts thereof which have a weight-average-molecular weight of 40,000 to 70,000 for producing an ophthalmic composition for reducing eye dryness.

The ophthalmic composition according to the present embodiment can reduce dryness of the eyes and contact lenses. Therefore, it is possible to improve various symptoms caused by these symptoms. That is, the ophthalmic composition according to the present embodiment can reduce dryness of the eyes and contact lenses, and thus can alleviate symptoms such as friction during blinking due to dryness of the eyes and contact lenses, watery eyes, eye fatigue, bloodshot eyes, blurred vision, decreased visual function, inflammation, itching, a feeling of rumbling in the eyes, a feeling of foreign substance, pain, photophobia, eye discomfort (for example, discomfort when wearing hard contact lenses or soft contact lenses), damage to the eye surface due to drought stress, damage to the eye surface due to light and other light rays, dullness of the eyelids (heavy eyelids), and inability to concentrate and continue watching.

The ophthalmic composition according to the present embodiment has high affinity for contact lenses and has an effect of reducing dryness of contact lenses, and can be suitably used as an ophthalmic composition for reducing dryness of contact lenses. Here, in this embodiment, in order to more significantly exhibit the effects of the first present invention, the contact lenses are preferably soft contact lenses and more preferably silicone hydrogel contact lenses.

In addition, one embodiment of the first present invention provides a method of reducing dryness of contact lenses using at least one selected from the group consisting of chondroitin sulfate and salts thereof which have a weight-average-molecular weight of 40,000 to 70,000. In addition, one embodiment of the first present invention provides a use of at least one selected from the group consisting of chondroitin sulfate and salts thereof which have a weight-average-molecular weight of 40,000 to 70,000 for producing an ophthalmic composition for reducing dryness of contact lenses.

The ophthalmic composition according to the present embodiment is preferably an eye drop (including an eye drop that can be instilled in the eyes wearing contact lenses) because it can more significantly exhibit the effects of the first present invention. When the ophthalmic composition according to the present embodiment is an eye drop, the usage and dosage thereof are not particularly limited as long as they exhibit effects and have few side effects, and for example, for adults (aged 15 years or older) and children aged 7 years or older, a method of dropping 1 to 3 drops, 1 to 2 drops, or 2 to 3 drops at a time, 2 to 4 times or 5 to 6 times a day can be used.

The ophthalmic composition according to the present embodiment is provided after being stored in an arbitrary container. The container for storing the ophthalmic composition according to the present embodiment is not particularly limited, and may be made of, for example, glass or a plastic. The container is preferably made of a plastic. Examples of plastics include polyethylene terephthalate (PET), polyarylate, polyethylene naphthalate, polycarbonate, polyethylene, polypropylene, polyimide, copolymers of monomers constituting these, and mixtures of two or more of these. The container for storing the ophthalmic composition is preferably polyethylene terephthalate in order to further improve the effects of the first present invention. In addition, the container for storing the ophthalmic composition according to the present embodiment may be a transparent container that allows the inside of the container to be visually recognized or an opaque container that makes it difficult to visually recognize the inside of the container. A transparent container is preferable. Here, the "transparent container" includes both a colorless transparent container and a colored transparent container.

A nozzle may be attached to the container for storing the ophthalmic composition according to the present embodiment. The material of the nozzle is not particularly limited, and may be made of, for example, glass or a plastic. The container is preferably made of a plastic. Examples of plastics include polybutylene terephthalate, polyethylene, polypropylene, polyethylene terephthalate, polyethylene naphthalate, copolymers of monomers constituting these, and mixtures of two or more of these. In order to further improve the effects of the first present invention, the material of the nozzle is preferably polypropylene, polyethylene, polyethylene terephthalate, polybutylene terephthalate, or polyethylene naphthalate, and more preferably polyethylene.

The container for storing the ophthalmic composition according to the present embodiment may be a multi-dose type container in which a plurality of doses are stored or a unit-dose type container in which a single dose is stored.

The ophthalmic composition according to the present embodiment is preferably filled into a container having an internal volume of 4 to 30 mL, more preferably filled into a container having an internal volume of 5 to 20 mL, still more preferably filled into a container having an internal volume of 6 to 16 mL, and yet more preferably filled into a container having an internal volume of 10 to 15 mL. In addition, the ophthalmic composition may be filled into a container having an internal volume of 0.1 to 3 mL or may be filled into a container having an internal volume of 0.2 to 1 mL.

### [Examples]

Hereinafter, the first present invention will be described in detail with reference to test examples, but the first present invention is not limited thereto. In addition, the sodium chondroitin sulfate used in the following test examples is as follows, and sodium chondroitin sulfate having a weight-average-molecular weight of about 56,000 was derived from sharks.
sodium chondroitin sulfate
weight-average-molecular weight of about 56,000: commercially available from Seikagaku Corporation; Grade N-K
weight-average-molecular weight of about 28,000: commercially available from Seikagaku Corporation; Grade ND-K
weight-average-molecular weight of about 25,000: commercially available from Maruha Nichiro Corporation; over-the-counter sodium chondroitin sulfate

### [Test Example 1: drought stress test using rabbits]

Each test substance was prepared by dissolving sodium chondroitin sulfate having a weight-average-molecular weight of about 25,000 (commercially available from Maruha Nichiro Corporation; over-the-counter sodium chondroitin sulfate) or sodium chondroitin sulfate having a weight-average-molecular weight of about 56,000 (commercially available from Seikagaku Corporation; Grade N-K) so that the content was 0.5 w/v% or 3 w/v%, respectively, in a saline (commercially available from Otsuka Pharmaceutical Factory, Inc.).

According to the method used by Nagano et al. (New Ophthalmology, 13(2), 267-270, 1996), rabbits divided into 5 groups (n=5) (Japanese white rabbits (commercially available from Kitayama Labes Co. Ltd.)) were subjected to general anesthesia, and then gloves with ring-shaped fingers were put on their left and right eyeballs and the eyelids were forcibly opened. Immediately after the eyelids were opened, the test substance was instilled into both eyes of the rabbits in each group at 100 µL per eye, and the rabbits were left with their eyelids open for 3 hours. The ocular surface was stained by dropping 50 µL of a 1% methylene blue solution and washed with a saline. After the cornea was removed from the ocular surface, the methylene blue dye was extracted, and the absorbance was measured using a microplate reader Multiskan GO (commercially available from Thermo Fisher Scientific) at 660 nm. The results are shown in FIG. 1. Here, eye dryness caused corneal damage, and increased the amount of dye adhered to the cornea surface, and thus it could be evaluated that the higher the absorbance value, the drier the eyes were.

As shown in FIG. 1, it was confirmed that sodium chondroitin sulfate having a weight-average-molecular weight of about 56,000 had a significantly lower absorbance (**p<0.01, Tukey's multiple comparison test), and less corneal damage caused by eye dryness than sodium chondroitin sulfate having a weight-average-molecular weight of about 25,000.

### [Test Example 2: contact lens wettability test]

Ophthalmic compositions were prepared according to a general method using the formulations shown in Table 1 and used as test solutions. In Table 1, the units of the components are w/v%. Here, in Test Example 2 and the following test examples, sodium chondroitin sulfate having a weight-average-molecular weight of about 28,000 (commercially available from Seikagaku Corporation; Grade ND-K) and sodium chondroitin sulfate having a weight-average-molecular weight of about 56,000 (commercially available from Seikagaku Corporation; Grade N-K) were used.

4 mL of phosphate-buffered saline (sodium chloride 0.60%, dibasic sodium phosphate (12 hydrate) 0.60%, sodium dihydrogen phosphate (2 hydrate) 0.05%, pH 7.4±0.1) was dispensed into a 12-well plate (BD Falcon, No. 35-3043). One contact lens (Acuvue Oasys (commercially available from Johnson & Johnson Services, Inc.)) was immersed in each well and left at room temperature for 4 hours or longer. 4 mL of phosphate-buffered saline and each test solution were dispensed into a 12-well plate. Water on the contact lenses was gently wiped off with lint-free paper, and the contact lenses were immersed and left for 15 minutes. 100 mL of phosphate-buffered saline was dispensed into a beaker. The immersed contact lenses were gently rinsed with phosphate-buffered saline and water was removed using lint-free paper. The contact lenses were placed on a slide glass, and the contact angle 0.10 seconds after 3 µl of phosphate-buffered saline was added dropwise was measured using a contact angle measurement device (solid-liquid interface analysis system DropMaster500 (commercially available from Kyowa Interface Science Co., Ltd.)). The results are shown in Table 1. Here, it could be evaluated that the lower the contact angle, the better the wettability of the contact lenses and the higher the affinity for contact lenses.

**[Table 1]**

| | Test solution 1 | Test solution 2 | Test solution 3 |
|---|---|---|---|
| Boric acid | 1.7 | 1.7 | 1.7 |
| Borax | 0.2 | 0.2 | 0.2 |
| Sodium chondroitin sulfate (weight-average-molecular weight of about 28,000) | - | 0.5 | - |
| Sodium chondroitin sulfate (weight-average-molecular weight of about 56,000) | - | - | 0.5 |
| Purified water | Residual amount | Residual amount | Residual amount |
| Total amount | 100 mL | 100 mL | 100 mL |
| Contact angle (°) | 113.4 | 118.1 | 78.2 |

A test solution 2 containing sodium chondroitin sulfate having a weight-average-molecular weight of about 28,000 showed almost the same contact angle as a test solution 1 containing no sodium chondroitin sulfate. On the other hand, it was confirmed that a test solution 3 containing sodium chondroitin sulfate having a weight-average-molecular weight of about 56,000 had a significantly smaller contact angle, further significantly improved wettability, and higher affinity for contact lenses than the test solution 2 containing sodium chondroitin sulfate having a weight-average-molecular weight of about 28,000.

In addition, for a test solution 3-1 having the same composition as the test solution 3 except that the content of sodium chondroitin sulfate (a weight-average-molecular weight of about 56,000) of the test solution 3 was 1 w/v%, and a test solution 3-2 obtained by adding 50,000 units/100 mL of retinol palmitate, 0.35 w/v% of polyoxyethylene hydrogenated castor oil 60 and 0.2 w/v% of polyoxyethylene castor oil 10 to the test solution 3-1, and the contact angle (°) was measured in the same method as in Test Example 2, and was 67.4° for the test solution 3-1 and 44.2° for the test solution 3-2.

### [Test Example 3: contact lens dry test]

4 mL of phosphate-buffered saline (sodium chloride 0.60%, dibasic sodium phosphate (12 hydrate) 0.60%, sodium dihydrogen phosphate (2 hydrate) 0.05%, pH 7.4±0.1) was dispensed into a 12-well plate (BD Falcon, No. 35-3043). Two contact lenses (Acuvue Oasys (commercially available from Johnson & Johnson Services, Inc.)) were immersed in each well and left at room temperature for 4 hours or longer. After water on the contact lenses was gently wiped off with lint-free paper, the contact lenses were immersed in a 12-well plate to which 4 mL of phosphate-buffered saline and each test solution shown in Table 1 were dispensed, and left at room temperature for 24 hours. Water on the contact lenses was gently wiped off with lint-free paper, the contact lenses were immersed in a 12-well plate to which 2 mL of a cobalt(II) chloride color comparison stock solution (commercially available from Wako Pure Chemical Industries, Ltd., seller code: 031-19041) was dispensed and shaken under conditions of room temperature and 200 rpm for 5 minutes. Water on the contact lenses was gently wiped off with lint-free paper, and the coloring of the soft contact lenses was observed immediately (0 minutes) after they were placed on a cover glass and 5 minutes, 15 minutes and 30 minutes after they were placed on a cover glass. Here, in this test example, when the contact lenses were dried, they turned blue. Table 2 shows the results of evaluating the presence of coloring based on the following criteria.
A: no coloring
B: slightly colored (less than 30% of the entire contact lens surface)
C: clearly colored (30% or more of the entire contact lens surface)

**[Table 2]**

| | Test solution 1 | Test solution 2 | Test solution 3 |
|---|---|---|---|
| 0 minutes | A | A | A |
| 5 minutes | A | A | A |
| 15 minutes | B | B | A |
| 30 minutes | C | C | B |

In the test solution 1 containing no sodium chondroitin sulfate and the test solution 2 containing sodium chondroitin sulfate having a weight-average-molecular weight of about 28,000, slight coloring was observed after 15 minutes, but in the test solution 3 containing sodium chondroitin sulfate having a weight-average-molecular weight of about 560,000,000, no coloring was observed after 15 minutes, and slight coloring was observed after 30 minutes. Therefore, it was confirmed that sodium chondroitin sulfate having a weight-average-molecular weight of about 56,000 reduced dryness of the contact lenses more than sodium chondroitin sulfate having a weight-average-molecular weight of about 28,000.

### [Test Example 4: contact lens wettability test]

Ophthalmic compositions were prepared according to a general method using the formulations shown in Table 3 and used as test solutions. The units of the components in Table 3 are w/v% unless specified in the table. Here, in Test Example 4 and the following test examples, 1,740,000 IU/g of retinol palmitate was used. After water on the surface of contact lenses (Acuvue Advance (commercially available from Johnson & Johnson Services, Inc.)) immersed in 2 mL of a saline for 4 hours or longer was thoroughly wiped off with lint-free paper, droplets (about 1 µL) of each prepared ophthalmic composition were added dropwise, and the contact angle (static contact angle) with respect to the contact lens 0.1 seconds after dropwise addition was measured using an automatic contact angle meter (solid-liquid interface analysis system Drop Master, DM-A501 (commercially available from Kyowa Interface Science Co., Ltd.)). The contact angle was measured three times for each ophthalmic composition, and the average value thereof was calculated and used as the contact angle of each ophthalmic composition. The results are shown in Table 3. Here, it could be evaluated that the lower the contact angle, the better the wettability of the contact lenses, and the higher the affinity for contact lenses.

**[Table 3]**

| Component name | Test soluti on 4 | Test soluti on 5 | Test soluti on 6 | Test soluti on 7 | Test soluti on 8 | Test soluti on 9 | Test soluti on 10 | Test soluti on 11 | Test soluti on 12 | Test soluti on 13 | Test soluti on 14 | Test soluti on 15 |
|---|---|---|---|---|---|---|---|---|---|---|---|---|
| Sodium chondroitin sulfate (weight-aver age-molecula r weight of about 56,000) | 0.5 | 0.5 | 0.5 | 0.5 | 0.5 | 0.5 | 0.5 | 0.5 | 0.5 | 0.5 | 0.5 | 0.5 |
| Neostigmine methyl sulfate | - | - | 0.005 | - | - | - | - | - | - | - | - | - |
| Allantoin | - | - | - | 0.3 | - | - | - | - | - | - | - | - |
| Dipotassium glycyrrhizina te | - | - | - | | 0.25 | - | - | - | - | - | - | - |
| Pyridoxine hydrochlorid e | - | - | - | - | - | 0.1 | - | - | - | - | - | - |
| Panthenol | - | - | - | - | - | - | 0.1 | - | - | - | - | - |
| d-α-tocopher ol acetate | - | - | - | - | - | - | - | 0.05 | - | - | - | - |
| Retinol palmitate | - | - | - | - | - | - | - | - | 25,00 0 units | - | - | - |
| Potassium L-aspartate | - | - | - | - | - | - | - | - | - | 1 | - | - |
| Taurine | - | - | - | - | - | - | - | - | - | - | 1 | - |
| Hydroxyprop yl methylcellul ose | - | - | - | - | - | - | - | - | - | - | - | 0.1 |
| Boric acid | 1.2 | 1.2 | 1.2 | 1.2 | 1.2 | 1.2 | 1.2 | 1.2 | 1.2 | 1.2 | 1.2 | 1.2 |
| Borax | 0.1 | 0.1 | 0.1 | 0.1 | 0.1 | 0.1 | 0.1 | 0.1 | 0.1 | 0.1 | 0.1 | 0.1 |
| Polyoxyethyl ene hydrogenated castor oil (HCO-60) | - | 0.35 | - | - | - | - | - | 0.35 | 0.35 | - | - | - |
| Polyoxyethyl ene castor oil (CO-10) | - | 0.2 | - | - | - | - | - | 0.2 | 0.2 | - | - | - |
| Hydrochloric acid | Appr opriat e amou nt | Appr opriat e amou nt | Appr opriat e amou nt | Appr opriat e amou nt | Appr opriat e amou nt | Appr opriat e amou nt | Appr opriat e amou nt | Appr opriat e amou nt | Appr opriat e amou nt | Appr opriat e amou nt | Appr opriat e amou nt | Appr opriat e amou nt |
| Purified water | Resid ual amou nt | Resid ual amou nt | Resid ual amou nt | Resid ual amou nt | Resid ual amou nt | Resid ual amou nt | Resid ual amou nt | Resid ual amou nt | Resid ual amou nt | Resid ual amou nt | Resid ual amou nt | Resid ual amou nt |
| Total amount | 100 mL | 100 mL | 100 mL | 100 mL | 100 mL | 100 mL | 100 mL | 100 mL | 100 mL | 100 mL | 100 mL | 100 mL |
| pH | 5.5 | 5.5 | 5.5 | 5.5 | 5.5 | 5.5 | 5.5 | 5.5 | 5.5 | 5.5 | 5.5 | 5.5 |
| Contact anele (°) | 85.5 | 73.7 | 73.5 | 76.5 | 81.0 | 79.9 | 76.5 | 67.5 | 48.0 | 38.3 | 62.3 | 78.8 |

Test solutions 6 to 10 and 13 to 15 had a significantly smaller contact angle and further significantly improved wettability than a test solution 4. In addition, test solutions 11 and 12 had a significantly smaller contact angle and further significantly improved wettability than a test solution 5. That is, it was confirmed that the test solutions containing sodium chondroitin sulfate having a weight-average-molecular weight of about 56,000 and neostigmine methyl sulfate, allantoin, dipotassium glycyrrhizinate, pyridoxine hydrochloride, panthenol, d-α-tocopherol acetate, retinol palmitate, potassium L-aspartate, taurine or hydroxypropyl methylcellulose had a significantly smaller contact angle, further significantly improved wettability and higher affinity for contact lenses than the test solution containing only sodium chondroitin sulfate having a weight-average-molecular weight of about 56,000.

### [Test Example 5: measurement of contact angle in stainless steel tube]

Ophthalmic compositions were prepared according to a general method using the formulations shown in Table 4 and used as test solutions. In Table 4, the units of the components are w/v%. Droplets (about 1 µL) of each prepared ophthalmic composition were added dropwise to a metal plate made of stainless steel (Ferrule Cap TypeCLF-B), and the contact angle (static contact angle) with respect to the metal 0.1 seconds after dropwise addition was measured using an automatic contact angle meter (solid-liquid interface analysis system Drop Master, DM-A501 (commercially available from Kyowa Interface Science Co., Ltd.)). The contact angle was measured three times for each ophthalmic composition, and the average value thereof was calculated and used as the contact angle of each ophthalmic composition. The results are shown in Table 4.

**[Table 4]**

| Component name | Test solution 4 | Test solution 15 |
|---|---|---|
| Sodium chondroitin sulfate (weight-average-molecular weight of about 56,000) | 0.5 | 0.5 |
| Hydroxypropyl methylcellulose | - | 0.1 |
| Boric acid | 1.2 | 1.2 |
| Borax | 0.1 | 0.1 |
| Hydrochloric acid | Appropriate amount | Appropriate amount |
| Purified water | Residual amount | Residual amount |
| Total amount | 100 mL | 100 mL |
| pH | 5.5 | 5.5 |
| Contact angle (°) | 65.0 | 72.2 |

The test solution 15 had a significantly larger contact angle than the test solution 4. That is, it was confirmed that the test solution containing sodium chondroitin sulfate having a weight-average-molecular weight of about 56,000 and hydroxypropyl methylcellulose had significantly lower affinity for the stainless steel tube than the test solution containing only sodium chondroitin sulfate having a weight-average-molecular weight of about 56,000. It was found that, since the filling tubes on the production line were made of a metal such as stainless steel, when the ophthalmic composition was filled into the container, it was possible to reduce the amount of droplets adhered to the tips of the filling tubes, and the filling amount was easily uniformized.

### [Test Example 6: preservative efficacy test]

Ophthalmic compositions were prepared according to a general method using the formulations shown in Table 5 and used as test solutions. The units of the components in Table 5 are w/v% unless specified in the table. *Staphylococcus aureus* (ATCC6538) was inoculated on the surface of a soy bean/casein/digest slant medium and cultured at 33°C for 24 hours. The cultured bacterial cells were aseptically collected using a platinum loop and suspended in an appropriate amount of a sterile saline to prepare a bacterial suspension containing about 1×10⁷ CFU/mL of viable bacteria. Here, the number of viable bacteria in the suspension was measured by performing culturing separately. Next, 10 mL of each prepared ophthalmic composition was filled into a 15 mL conical tube made of PET (commercially available from CORNING). A *Staphylococcus aureus* bacterial solution (suspended in the saline) was inoculated into each of these ophthalmic compositions so that the number of viable bacteria (final concentration) was about 10⁵ CFU/mL, and stirred well to prepare a sample. The sample containing bacteria was stored at 23°C for 3 days under a light-shielded condition. Then, the sample containing bacteria was adjusted to an appropriate concentration for counting, 1 mL of the sample was seeded on a 3M^{™} Petrifilm ^{™} Rapid Aerobic Count Plate (RAC plate) and cultured at 33°C for 2 days, and the number of colonies observed was then counted to determine the number of viable bacteria. The number of viable bacteria immediately after inoculation was compared with the number of viable bacteria in the sample after storage for 3 days, and the amount of decrease in the number of bacteria was calculated as Log Reduction. In addition, regarding the calculated Log Reduction, it was determined whether the preservative efficacy was sufficient based on the following evaluation criteria. Here, culture of bacteria for initial bacteria count was performed at 33°C for 2 days. The results are shown in Table 5.

### Evaluation criteria

Log Reduction<0.7: D
0.7≤Log Reduction<0.8: C
0.8≤Log Reduction<0.9: B
0.9≤Log Reduction: A

**[Table 5]**

| Component name | Test solution 16 | Test solution 17 | Test solution 4 | Test solution 7 | Test solution 8 | Test solution 12 | Test solution 14 |
|---|---|---|---|---|---|---|---|
| Sodium chondroitin sulfate (weight-average-molecul ar weight of about 28,000) | 0.5 | 0.5 | - | - | - | - | - |
| Sodium chondroitin sulfate (weight-average-molecul ar weight of about 56,000) | - | - | 0.5 | 0.5 | 0.5 | 0.5 | 0.5 |
| Allantoin | - | - | - | 0.3 | - | - | - |
| Dipotassium glycyrrhizinate | - | - | - | - | 0.25 | - | - |
| Retinol palmitate | - | - | - | - | - | 25,000 units | - |
| Taurine | - | - | - | - | - | - | 1 |
| Boric acid | 1.2 | 1.2 | 1.2 | 1.2 | 1.2 | 1.2 | 1.2 |
| Borax | 0.1 | 0.1 | 0.1 | 0.1 | 0.1 | 0.1 | 0.1 |
| Polyoxyethylene hydrogenated castor oil 60 | - | 0.350 | - | - | - | 0.350 | - |
| Polyoxyethylene castor oil 10 | - | 0.200 | - | - | - | 0.200 | - |
| Hydrochloric acid | Appropriat e amount | Appropriat e amount | Appropriat e amount | Appropriat e amount | Appropriat e amount | Appropriat e amount | Appropriat e amount |
| Purified water | Residual amount | Residual amount | Residual amount | Residual amount | Residual amount | Residual amount | Residual amount |
| Total amount | 100 mL | 100 mL | 100 mL | 100 mL | 100 mL | 100 mL | 100 mL |
| pH | 5.5 | 5.5 | 5.5 | 5.5 | 5.5 | 5.5 | 5.5 |
| Evaluation Criteria (Log reduction) | D | D | B | A | A | A | A |

It was confirmed that the test solutions 7, 8, 12 and 14 containing sodium chondroitin sulfate having a weight-average-molecular weight of about 56,000, and allantoin, dipotassium glycyrrhizinate, retinol palmitate or taurine had further improved preservative efficacy than test solutions 16 and 17 containing sodium chondroitin sulfate having a weight-average-molecular weight of about 28,000. In addition, regarding the test solution 12, even if the same test was performed with the same formulation as the test solution 12 except that the concentration of retinol palmitate was 50,000 units/100 mL, the same preservative efficacy improving effect could be obtained.

### [Test Example 7: measurement of viscosity]

Ophthalmic compositions were prepared according to a general method using the formulations shown in Table 6 and used as test solutions. The units of the components in Table 6 are w/v% unless specified in the table. Regarding the viscosity of each ophthalmic composition (600 µL) after preparation, the viscosity against a shear rate at 34°C was measured using a cone plate type measurement jig (CP50-1, d: 0.102 mm) with a rheometer (MCR302 (commercially available from AntonPaar)). Regarding the viscosity (mPals) at a shear rate of 10,000 (1/s), according to the following formula, the viscosity reduction percentage of the test solution 12 or 13 with respect to the test solution 4 or 5 (formulation containing sodium chondroitin sulfate alone) was calculated. Here, a decrease in viscosity indicates that the viscosity decreased when stress was applied and the shear rate of 10,000 (1/s) was assumed to be the blink rate. When the viscosity at a shear rate of 10,000 (1/s) was measured, it was possible to evaluate a change in viscosity of the intraocular formulation during blinking. A decrease in viscosity during blinking indicates that it was easier to blink and it was less likely to cause discomfort during blinking. viscosity reduction percentage (%)=(1-viscosity of the test solution 12 or 13/viscosity of corresponding test solution)×100

Here, the corresponding test solution was the test solution 5 for the test solution 12 and the test solution 4 for the test solution 13.

The results are shown in Table 6.

**[Table 6]**

| Component name | Test solution 4 | Test solution 5 | Test solution 12 | Test solution 13 |
|---|---|---|---|---|
| Sodium chondroitin sulfate (weight-average-molecular weight of about 56,000) | 0.5 | 0.5 | 0.5 | 0.5 |
| Retinol palmitate | - | - | 25,000 units | - |
| Potassium L-aspartate | - | - | - | 1 |
| Boric acid | 1.2 | 1.2 | 1.2 | 1.2 |
| Borax | 0.1 | 0.1 | 0.1 | 0.1 |
| Polyoxyethylene hydrogenated castor oil 60 | - | 0.4 | 0.4 | - |
| Polyoxyethylene castor oil 10 | - | 0.2 | 0.2 | - |
| Hydrochloric acid | Appropriate amount | Appropriate amount | Appropriate amount | Appropriate amount |
| Purified water | Residual amount | Residual amount | Residual amount | Residual amount |
| Total amount | 100 mL | 100 mL | 100 mL | 100 mL |
| pH | 5.5 | 5.5 | 5.5 | 5.5 |
| Viscosity reduction percentage (%) | - | - | 17.4 | 29.9 |

The test solutions 12 and 13 containing retinol palmitate or potassium L-aspartate had a significantly lower viscosity than the test solution 5 and 4. Here, the inventors confirmed that the viscosity of the test solution 4 prepared using sodium chondroitin sulfate having a weight-average-molecular weight of about 56,000 measured with a rotational viscometer was higher than the viscosity of a test solution having the same composition as the test solution 4 except that sodium chondroitin sulfate having a weight-average-molecular weight of about 28,000 was used in place of sodium chondroitin sulfate having a weight-average-molecular weight of about 56,000. If the viscosity of the ophthalmic composition when instilled in the eyes is high, problems such as difficulty in blinking and easily causing discomfort may occur. Therefore, it can be said that, when the viscosity at a high shear rate is low, it is easy to blink after instillation and it is less likely to cause discomfort. Therefore, it was confirmed that the ophthalmic composition containing sodium chondroitin sulfate having a weight-average-molecular weight of about 56,000, and retinol palmitate or potassium L-aspartate made it easier to blink after instillation and was less likely to cause discomfort than the ophthalmic composition containing only sodium chondroitin sulfate having a weight-average-molecular weight of about 56,000.

### [Test Example 8: test of viscosity stability upon light emission]

Ophthalmic compositions were prepared according to a general method using the formulations shown in Table 7 and used as test solutions. The units of the components in Table 7 are w/v% unless specified in the table. A 10 mL glass headspace vial (commercially available from GL Sciences Inc.) was filled with 10 mL of each ophthalmic composition, and radiated with light in a photostability test device (LT-120A-WCD (commercially available from Nagano Science Co., Ltd.)) using a D65 fluorescent lamp as a light source at a room temperature of 25°C and an illuminance of 4,000 lx/h until the cumulative illuminance reached 1,200,000 lx. For each ophthalmic composition (600 µL) before and after radiation, the viscosity at a shear rate (1 to 10,000 (1/s)) at 34°C was measured using a cone plate type measurement jig (CP50-1, d: 0.102 mm) with a rheometer (MCR302 (commercially available from AntonPaar)). Then, using the viscosity (mPals) at a shear rate of 1,000 (1/s), according to the following formula, the viscosity stability before and after the test was evaluated. The results are shown in Table 7. Here, the smaller the value of the viscosity change percentage, the less the change in viscosity caused by light, and the more the ophthalmic composition is kept physically the same. viscosity change percentage (%)={(viscosity of each ophthalmic composition before light emission-viscosity of each ophthalmic composition after light emission)/viscosity of each ophthalmic composition before light emission}×100

**[Table 7]**

| Component name | Test solution 16 | Test solution 17 | Test solution 4 | Test solution 5 | Test solution 6 | Test solution 11 | Test solution 12 | Test solution 13 | Test solution 14 |
|---|---|---|---|---|---|---|---|---|---|
| Sodium chondroitin sulfate (weight-average-m olecular weight of about 28,000) | 0.5 | 0.5 | - | - | - | - | - | - | - |
| Sodium chondroitin sulfate (weight-average-m olecular weight of about 56,000) | - | - | 0.5 | 0.5 | 0.5 | 0.5 | 0.5 | 0.5 | 0.5 |
| Neostigmine methyl sulfate | - | - | - | - | 0.005 | - | - | - | - |
| d-α-tocopherol acetate | - | - | - | - | - | 0.05 | - | - | - |
| Retinol palmitate | - | - | - | - | - | - | 25,000 units | - | - |
| Potassium L-aspartate | - | - | - | - | - | - | - | 1 | - |
| Taurine | - | - | - | - | - | - | - | - | 1 |
| Boric acid | 1.2 | 1.2 | 1.2 | 1.2 | 1.2 | 1.2 | 1.2 | 1.2 | 1.2 |
| Borax | 0.1 | 0.1 | 0.1 | 0.1 | 0.1 | 0.1 | 0.1 | 0.1 | 0.1 |
| Polyoxyethylene hydrogenated castor oil 60 | - | 0.35 | - | 0.35 | - | 0.35 | 0.35 | - | - |
| Polyoxyethylene castor oil 10 | - | 0.2 | - | 0.2 | - | 0.2 | 0.2 | - | - |
| Hydrochloric acid | Appropr iate amount | Appropr iate amount | Appropr iate amount | Appropr iate amount | Appropr iate amount | Appropr iate amount | Appropr iate amount | Appropr iate amount | Appropr iate amount |
| Purified water | Residua l amount | Residua l amount | Residua l amount | Residua l amount | Residua l amount | Residua l amount | Residua l amount | Residua l amount | Residua l amount |
| Total amount | 100 mL | 100 mL | 100 mL | 100 mL | 100 mL | 100 mL | 100 mL | 100 mL | 100 mL |
| Viscosity change percentage (%) at a shear rate of 1,000 (1/s) | 0.4 | 5.8 | 26.5 | 23.9 | 0.8 | 2.6 | 4.3 | 1.4 | 0.5 |

In the test solutions 16 and 17 containing sodium chondroitin sulfate having a weight-average-molecular weight of about 28,000, the viscosity did not change due to light emission, but in the test solutions 4 and 5 containing sodium chondroitin sulfate having a weight-average-molecular weight of about 56,000, there was a problem of the viscosity changing due to light emission. However, it was confirmed that, in the test solutions 6 and 11 to 14 containing sodium chondroitin sulfate having a weight-average-molecular weight of about 56,000, and neostigmine methyl sulfate, d-α-tocopherol acetate, retinol palmitate, potassium L-aspartate or taurine, the viscosity change percentage was significantly lowered, and the stability of the ophthalmic composition upon light emission was improved. Here, the same tendency was obtained when the viscosity was measured at a shear rate of 100 (1/s). In addition, regarding the test solution 12, even if the same test was performed with the same formulation as the test solution 12 except that the concentration of retinol palmitate was 50,000 units/100 mL, the same stability improving effect of the ophthalmic composition upon light emission could be obtained.

### [Test Example 9: test for reducing precipitates]

Ophthalmic compositions were prepared according to a general method using the formulations shown in Table 8 and used as test solutions. In Table 8, the units of the components are w/v%. 1 mL of each ophthalmic composition was added to a 24-well plate (commercially available from Corning Inc.) and stored in a dry heat dryer (commercially available from Ikeda Scientific Co., Ltd.) at 60°C for 2 days, and the state of precipitates in each well in the plate was then visually observed, and the occurrence of precipitates was evaluated based on the following evaluation criteria. The results are shown in Table 8.

### <Evaluation criteria for occurrence of precipitate>

There were clearly visible precipitates on the bottom of the well, and the percentage of precipitates on the bottom was 1/2 or more: +++ There were clearly visible precipitates on the bottom of the well, and the percentage of precipitates on the bottom was 1/3 or more and less than 1/2: ++

There were clearly visible precipitates on the bottom of the well, and the percentage of precipitates on the bottom was less than 1/3: + No precipitates: -

**[Table 8]**

| Component name | Test solution 18 | Test solution 13 |
|---|---|---|
| Sodium chondroitin sulfate (weight-average-molecular weight of about 28,000) | 0.5 | - |
| Sodium chondroitin sulfate (weight-average-molecular weight of about 56,000) | - | 0.5 |
| Potassium L-aspartate | 1 | 1 |
| Boric acid | 1.2 | 1.2 |
| Borax | 0.1 | 0.1 |
| Hydrochloric acid | Appropriate amount | Appropriate amount |
| Purified water | Residual amount | Residual amount |
| Total amount | 100 mL | 100 mL |
| Precipitation | +++ | + |

Compared to a test solution 18 containing sodium chondroitin sulfate having a weight-average-molecular weight of about 28,000 and potassium aspartate, in the test solution 13 containing sodium chondroitin sulfate having a weight-average-molecular weight of about 56,000 and potassium aspartate, the occurrence of precipitates was significantly reduced.

### [Test Example 10: test for reducing change in appearance (color) upon light emission]

Ophthalmic compositions were prepared according to a general method using the formulations shown in Table 9 and used as test solutions. The units of the components in Table 9 are w/v% unless specified in the table. A 10 mL glass headspace vial was filled with 10 mL of each ophthalmic composition, and radiated with light in a photostability test device (LT-120A-WCD (commercially available from Nagano Science Co., Ltd.)) using a D65 fluorescent lamp as a light source at a room temperature of 25°C and an illuminance of 4,000 lx/h until the cumulative illuminance reached 1,200,000 lx. After the test, the color difference change (b* value) was measured for each ophthalmic composition before and after light emission using a spectrophotometer (CM3500d: commercially available from Konica Minolta, Inc.), and according to the following Formula 1, the change (color difference change degree; Δb* value) in appearance (color) of the ophthalmic composition before and after light emission was calculated, and, the color difference change reduction percentage was additionally calculated according to the following Formula 2. The results are shown in Table 9. Here, the smaller the Δb* value, the less the change (coloring) in appearance (color) of the ophthalmic composition. Δb* = b* value of each ophthalmic composition before light emission-b* value of each ophthalmic composition after light emission color difference change reduction percentage (%)={1-(Δb* of the test solution 12/Δb* of the test solution 19)}×100

**[Table 9]**

| Component name | Test solution 19 | Test solution 12 |
|---|---|---|
| Sodium chondroitin sulfate (weight-average-molecular weight of about 28,000) | 0.5 | - |
| Sodium chondroitin sulfate (weight-average-molecular weight of about 56,000) | - | 0.5 |
| Retinol palmitate | 25,000 units | 25,000 units |
| Boric acid | 1.2 | 1.2 |
| Borax | 0.1 | 0.1 |
| Polyoxyethylene hydrogenated castor oil 60 | 0.35 | 0.35 |
| Polyoxyethylene castor oil 10 | 0.2 | 0.2 |
| Hydrochloric acid | Appropriate amount | Appropriate amount |
| Purified water | Residual amount | Residual amount |
| Total amount | 100 mL | 100 mL |
| pH | 5.5 | 5.5 |
| Color difference change reduction percentage (%) | - | 32.3 |

It was confirmed that the test solution 12 containing sodium chondroitin sulfate having a weight-average-molecular weight of about 56,000 and retinol palmitate had a smaller color difference change degree (Δb*) upon light emission and less coloring upon light emission than the test solution 19 containing sodium chondroitin sulfate having a weight-average-molecular weight of about 28,000 and retinol palmitate. Here, a difference in the degree of coloring was also visually observed between the test solution 19 before and after light emission, and between the test solution 19 and the test solution 12 after light emission.

### [Test Example 11: test for reducing change in appearance (color) upon UV emission]

Ophthalmic compositions were prepared according to a general method using the formulations shown in Table 10 and used as test solutions. The units of the components in Table 10 are w/v% unless specified in the table. A glass bottle (10 mL) was filled with 10 mL of each ophthalmic composition and radiated with light at 35°C using SUNTESTER XLS+ (commercially available from Toyo Seiki Co., Ltd., 1,700 W xenon air-cooled lamp light source) at an UV illuminance of 765 (W/m²) for 96 hours. Then, each ophthalmic composition was sufficiently thermally uniformized at 25°C, the color difference change (b* value) of each ophthalmic composition before and after UV emission was measured using a spectrophotometer (CM3500d: commercially available from Konica Minolta, Inc.), and according to the following Formula 1, the change in appearance (color) of the ophthalmic composition before and after UV emission (color difference change degree; Δb* value) was calculated, and according to the following Formula 2, the color difference change reduction percentage was additionally calculated. The results are shown in Table 9. Here, the smaller the Δb* value, the less the change (coloring) in appearance (color) of the ophthalmic composition. Δb* = b* value before UV emission - b* value after UV emission color difference change reduction percentage (%)={1-(Δb* of the test solution 9 or 12/Δb* of corresponding test solution)}×100

Here, the corresponding test solution was the test solution 20 for the test solution 9 and the test solution 19 for the test solution 12.

**[Table 10]**

| Component name | Test solution 20 | Test solution 19 | Test solution 9 | Test solution 12 |
|---|---|---|---|---|
| Sodium chondroitin sulfate (weight-average-molecular weight of about 28,000) | 0.5 | 0.5 | - | - |
| Sodium chondroitin sulfate (weight-average-molecular | - | - | 0.5 | 0.5 |
| weight of about 56,000) | | | | |
| Pyridoxine hydrochloride | 0.1 | - | 0.1 | - |
| Retinol palmitate | - | 25,000 units | - | 25,000 units |
| Boric acid | 1.2 | 1.2 | 1.2 | 1.2 |
| Borax | 0.1 | 0.1 | 0.1 | 0.1 |
| Polyoxyethylene hydrogenated castor oil 60 | - | 0.35 | - | 0.35 |
| Polyoxyethylene castor oil 10 | - | 0.2 | - | 0.2 |
| Hydrochloric acid | Appropriate amount | Appropriate amount | Appropriate amount | Appropriate amount |
| Purified water | Residual amount | Residual amount | Residual amount | Residual amount |
| Total amount | 100 mL | 100 mL | 100 mL | 100 mL |
| pH | 5.5 | 5.5 | 5.5 | 5.5 |
| Color difference change reduction percentage (%) | - | - | 23.0 | 38.7 |

It was confirmed that the test solutions 9 and 12 containing sodium chondroitin sulfate having a weight-average-molecular weight of about 56,000, and pyridoxine hydrochloride or retinol palmitate has a smaller color difference change degree (Δb*) upon UV emission and less coloring upon UV emission than the test solutions 20 and 19 containing sodium chondroitin sulfate having a weight-average-molecular weight of about 28,000, and pyridoxine hydrochloride or retinol palmitate.

### [Test Example 12: test for reducing change in appearance (transparency) due to heat]

Ophthalmic compositions were prepared according to a general method using the formulations shown in Table 11 and used as test solutions. The units of the components in Table 11 are w/v% unless specified in the table. A 10 mL glass headspace vial was filled with 10 mL of each ophthalmic composition and left and stored in a constant-temperature vessel at 60°C for 3 weeks (thermal acceleration test). Then, each ophthalmic composition was sufficiently thermally uniformized at 25°C and the color difference change (L* value) of each ophthalmic composition before and after the thermal acceleration test was measured using a spectrophotometer (CM3500d: commercially available from Konica Minolta, Inc.), and according to the following Formula 1, the change (ΔL*) in appearance (transparency) of the ophthalmic composition before and after the thermal acceleration test was calculated, and according to the following Formula 2, the transparency change difference reduction percentage was additionally calculated. The results are shown in Table 11. Here, the L* value was used as an indicator of transparency. Therefore, the smaller the ΔL* value, the less the change in appearance (transparency) of the formulation. Δb* = b* value before thermal acceleration test-L* value after thermal acceleration test transparency change reduction percentage (%)={1-(Δb* of the test solution 11 or 12/Δb* of corresponding test solution)}×100

Here, the corresponding test solution was the test solution 21 for the test solution 11 and the test solution 19 for the test solution 12.

**[Table 11]**

| Component name | Test solution 21 | Test solution 19 | Test solution 11 | Test solution 12 |
|---|---|---|---|---|
| Sodium chondroitin sulfate (weight-average-molecular weight of about 28,000) | 0.5 | 0.5 | - | - |
| Sodium chondroitin sulfate (weight-average-molecular weight of about 56,000) | - | - | 0.5 | 0.5 |
| d-α-tocopherol acetate | 0.05 | - | 0.05 | - |
| Retinol palmitate | - | 25,000 units | - | 25,000 units |
| Boric acid | 1.2 | 1.2 | 1.2 | 1.2 |
| Borax | 0.1 | 0.1 | 0.1 | 0.1 |
| Polyoxyethylene | 0.35 | 0.35 | 0.35 | 0.35 |

| | | | | |
|---|---|---|---|---|
| hydrogenated castor oil 60 | | | | |
| Polyoxyethylene castor oil 10 | 0.2 | 0.2 | 0.2 | 0.2 |
| Hydrochloric acid | Appropriate amount | Appropriate amount | Appropriate amount | Appropriate amount |
| Purified water | Residual amount | Residual amount | Residual amount | Residual amount |
| Total amount | 100 mL | 100 mL | 100 mL | 100 mL |
| pH | 5.5 | 5.5 | 5.5 | 5.5 |
| Transparency change reduction percentage (%) | - | - | 82.1 | 61.5 |

It was confirmed that the test solutions 11 and 12 containing sodium chondroitin sulfate having a weight-average-molecular weight of about 56,000, and d-α-tocopherol acetate or retinol palmitate had a significantly smaller change in transparency due to heat and less of a change in appearance (transparency) due to heat than the test solutions 21 and 19 containing sodium chondroitin sulfate having a weight-average-molecular weight of about 28,000, and d-α-tocopherol acetate or retinol palmitate.

### [Test Example 13: cell dry test]

Ophthalmic compositions were prepared according to a general method using the formulations shown in Table 12 and used as test solutions. The units of the components in Table 12 are w/v% unless specified in the table. 100 µL of human corneal epithelial cell line HCE-T cells at a concentration of 1×10⁵ cells/mL was seeded in each well in a 96-well plate (commercially available from Corning Inc.), and cultured in a CO₂ incubator set at 37°C, a humidity of 90%, and a concentration of 5% CO₂ until the cells became confluent. As a growth medium, one obtained by adding 5% of FCS (commercially available from DS Pharma Co., Ltd.), 0.5% of DMSO (commercially available from Wako Pure Chemical Industries, Ltd.), 10 ng/mL of a recombinant human EGF (commercially available from R&D), and 5 µg/mL of an insulin solution human (commercially available from SIGMA) to DMEM/F12 (commercially available from ThermoFisher) was used. After 2 to 4 days, when the cells became confluent, the growth medium was removed by suction from each well, 50 µL of each ophthalmic composition was added to each well, and incubated for 15 minutes under conditions of 37°C and 5% CO₂. After each ophthalmic composition was removed by suction from each well, drought stress was applied by leaving it in a clean bench for 20 minutes, and the number of viable cells was then evaluated. In order to evaluate the number of viable cells, 10 µL of a cell number measurement reagent (Cell counting kit-8, commercially available from Dojindo Molecular Technologies, Inc.) per 100 µL of the medium was put into each well, and culturing was performed under conditions of 37°C, 5% CO₂, and a humidity of 90% for 2 to 3 hours, and measurement was then performed using an absorbance meter (commercially available from MOLECULAR DEVICES) at 450 nm. Here, the higher the value of the absorbance, the greater the number of viable cells. The results are shown in Table 12.

**[Table 12]**

| Component name | Test solution 4 | Test solution 5 | Test solution 8 | Test solution 11 | Test solution 12 | Test solution 13 | Test solution 14 | Test solution 22 |
|---|---|---|---|---|---|---|---|---|
| Sodium chondroitin sulfate (weight-average-mol ecular weight of about 56,000) | 0.5 | 0.5 | 0.5 | 0.5 | 0.5 | 0.5 | 0.5 | 1 |
| Dipotassium glycyrrhizinate | - | - | 0.25 | - | - | - | - | - |
| d-α-tocopherol acetate | - | - | - | 0.05 | - | - | - | - |
| Retinol palmitate | - | - | - | - | 25,000 units | - | - | - |
| Potassium L-aspartate | - | - | - | - | - | 1 | - | - |
| Taurine | - | - | - | - | - | - | 1 | - |
| Boric acid | 1.2 | 1.2 | 1.2 | 1.2 | 1.2 | 1.2 | 1.2 | 1.2 |
| Borax | 0.1 | 0.1 | 0.1 | 0.1 | 0.1 | 0.1 | 0.1 | 0.1 |
| Polyoxyethylene hydrogenated castor oil 60 | - | 0.35 | - | 0.35 | 0.35 | - | - | - |
| Polyoxyethylene castor oil 10 | - | 0.2 | - | 0.2 | 0.2 | - | - | - |
| Hydrochloric acid | Appropri ate amount | Appropri ate amount | Appropri ate amount | Appropri ate amount | Appropri ate amount | Appropri ate amount | Appropri ate amount | Appropri ate amount |
| Purified water | Residual amount | Residual amount | Residual amount | Residual amount | Residual amount | Residual amount | Residual amount | Residual amount |
| Total amount | 100 mL | 100 mL | 100 mL | 100 mL | 100 mL | 100 mL | 100 mL | 100 mL |
| pH | 5.5 | 5.5 | 5.5 | 5.5 | 5.5 | 5.5 | 5.5 | 5.5 |
| Absorbance | 0.50 | 0.52 | 0.74 | 0.63 | 0.67 | 0.70 | 0.64 | 0.95 |

It was confirmed that, compared to the test solution 4 containing sodium chondroitin sulfate, in the test solutions 8, 13 and 14 containing sodium chondroitin sulfate, and dipotassium glycyrrhizinate, magnesium aspartate, or taurine, the number of viable cells significantly increased, and cell death due to drought stress could be significantly reduced. In addition, it was confirmed that, compared to the test solution 5 containing sodium chondroitin sulfate, in the test solutions 11 and 12 containing d-α-tocopherol acetate or retinol palmitate, the number of viable cells significantly increased, and cell death due to drought stress could be significantly reduced. In addition, based on the results of the test solutions 4 and 22, it was confirmed that, with sodium chondroitin sulfate having a weight-average-molecular weight of about 56,000, the number of viable cells significantly increased at 1%. In addition, regarding the test solution 12, even if the same test was performed with the same formulation as the test solution 12 except that the concentration of retinol palmitate was 50,000 units/100 mL, the same effect of reducing cell death due to drought stress could be obtained.

### [Test Example 14: cell damage test]

Ophthalmic compositions were prepared according to a general method using the formulations shown in Table 13 and used as test solutions. In Table 13, the units of the components are w/v%. 500 µL of human corneal epithelial cell line HCE-T cells at a concentration of 1×10⁵ cells/mL was seeded in each well in a 24-well pate (commercially available from Corning Inc.), and cultured in a CO₂ incubator set at 37°C and a concentration of 5% CO₂. As a growth medium, one obtained by adding 5% of FCS (commercially available from DS Pharma Co., Ltd.), 0.5% of DMSO (commercially available from Wako Pure Chemical Industries, Ltd.), 10 ng/mL of a recombinant human EGF (commercially available from R&D), and 5 µg/mL of an insulin solution human (commercially available from SIGMA) to DMEM/F12 (commercially available from ThermoFisher) was used. After 2 to 4 days, when the cells became confluent, the growth medium was removed by suction from each well, 50 µL of each ophthalmic composition was added to each well, and incubated for 15 minutes under conditions of 37°C and 5% CO₂. Three or four glass beads (commercially available from As One Corporation) were put into each well and shaken for 1 minute at 450 rpm using a microplate shaker (commercially available from Heidlph Instruments GmbH & Co. KG). The supernatant and glass beads were removed, 500 µL of a medium in which a cell number measurement reagent (Cell counting kit-8, commercially available from Dojindo Molecular Technologies, Inc.) and a medium were mixed at 1:10 was added, culturing was performed in a CO₂ incubator for 2 hours, and the absorbance at 450 nm was then measured using an absorbance meter (commercially available from MOLECULAR DEVICES). The cell survival percentage was calculated according to the following formula. cell survival percentage (%)=(absorbance in each formulation/absorbance of control)×100

The results are shown in Table 13.

**[Table 13]**

| Component name | Control | Test solution 16 | Test solution 4 | Test solution 7 | Test solution 8 | Test solution 9 | Test solution 10 |
|---|---|---|---|---|---|---|---|
| Sodium chondroitin sulfate (weight-average-molecul ar weight of about 28,000) | - | 0.5 | - | - | - | - | - |
| Sodium chondroitin sulfate (weight-average-molecul ar weight of about 56,000) | - | - | 0.5 | 0.5 | 0.5 | 0.5 | 0.5 |
| Allantoin | - | - | - | 0.3 | - | - | - |
| Dipotassium glycyrrhizinate | - | - | - | - | 0.25 | - | - |
| Pyridoxine hydrochloride | - | - | - | - | - | 0.1 | - |
| Panthenol | - | - | - | - | - | - | 0.1 |
| Boric acid | 1.2 | 1.2 | 1.2 | 1.2 | 1.2 | 1.2 | 1.2 |
| Borax | 0.1 | 0.1 | 0.1 | 0.1 | 0.1 | 0.1 | 0.1 |
| Hydrochloric acid | Appropriat e amount | Appropriat e amount | Appropriat e amount | Appropriat e amount | Appropriat e amount | Appropriat e amount | Appropriat e amount |
| Purified water | Residual amount | Residual amount | Residual amount | Residual amount | Residual amount | Residual amount | Residual amount |
| Total amount | 100 mL | 100 mL | 100 mL | 100 mL | 100 mL | 100 mL | 100 mL |
| pH | 5.5 | 5.5 | 5.5 | 5.5 | 5.5 | 5.5 | 5.5 |
| Survival percentage (%) based on 100 of control | - | 96.0 | 113.7 | 277.4 | 159.5 | 133.4 | 122.3 |

It was confirmed that the test solution 4 containing sodium chondroitin sulfate having a weight-average-molecular weight of about 56,000 had a higher cell survival percentage than to the test solution 16 containing sodium chondroitin sulfate having a weight-average-molecular weight of about 28,000. In addition, it was confirmed that the test solutions 4, 7 and 8 to 10 containing sodium chondroitin sulfate having a weight-average-molecular weight of about 56,000, and allantoin, dipotassium glycyrrhizinate, pyridoxine hydrochloride or panthenol has a significantly higher cell survival percentage than the test solution 4. Therefore, after the ophthalmic composition containing sodium chondroitin sulfate having a weight-average-molecular weight of about 56,000, and allantoin, dipotassium glycyrrhizinate, pyridoxine hydrochloride or panthenol was instilled, if an external stimulus was applied to the eyes (for example, when rubbing the eyes with hands, blinking, inserting and removing contact lenses, friction with contact lenses, or contamination with foreign substances (pollen, air contaminants, eyelashes, eye makeup, or other foreign substances)), it was expected that eye cell damage could be reduced.

### [Test Example 15: test for measuring residual liquid amount in eye drop bottle]

Ophthalmic compositions were prepared according to a general method using the formulations shown in Table 14 and used as test solutions. In Table 14, the units of the components are w/v%. The tare weight of a 10 mL PET eye drop bottle was measured, and 5 mL of each ophthalmic composition was filled. Next, the weight of each eye drop bottle after each filled ophthalmic compositions was discharged was measured, and according to the following Formulae 1 and 2, the residual liquid amount (g) and the improvement percentage (%) of the residual liquid amount with respect to the residual liquid amount of the test solution 22 were calculated. residual liquid amount (g) = the weight of the eye drop bottle after the ophthalmic composition is discharged-the tare weight of the eye drop bottle improvement percentahe (%) of the residual liquid amount = {1-(the residual liquid amount of each test solution/the residual liquid amount of the test solution 22)}×100

**[Table 14]**

| Component name | Test solution 22 | Test solution 4 | Test solution 5 | Test solution 6 | Test solution 11 | Test solution 12 | Test solution 14 |
|---|---|---|---|---|---|---|---|
| Sodium chondroitin sulfate (weight-average-molecul ar weight of about 56,000) | - | 0.5 | 0.5 | 0.5 | 0.5 | 0.5 | 0.5 |
| Neostigmine methyl sulfate | - | - | - | 0.005 | - | - | - |
| Taurine | - | - | - | - | - | - | 1 |
| d-α-tocopherol acetate | - | - | - | - | 0.05 | - | - |
| Retinol palmitate | - | - | - | - | - | 25,000 units | - |
| Boric acid | 1.2 | 1.2 | 1.2 | 1.2 | 1.2 | 1.2 | 1.2 |
| Borax | 0.1 | 0.1 | 0.1 | 0.1 | 0.1 | 0.1 | 0.1 |
| Polyoxyethylene hydrogenated castor oil (HCO-60) | - | - | 0.35 | - | 0.35 | 0.35 | - |
| Polyoxyethylene castor oil (CO-10) | - | - | 0.2 | - | 0.2 | 0.2 | - |
| Hydrochloric acid | Appropriat e amount | Appropriat e amount | Appropriat e amount | Appropriat e amount | Appropriat e amount | Appropriat e amount | Appropriat e amount |
| Purified water | Residual amount | Residual amount | Residual amount | Residual amount | Residual amount | Residual amount | Residual amount |
| pH | 5.5 | 5.5 | 5.5 | 5.5 | 5.5 | 5.5 | 5.5 |
| Total amount | 100 mL | 100 mL | 100 mL | 100 mL | 100 mL | 100 mL | 100 mL |
| Improvement percentage (%) of residual liquid amount | - | 23.6 | 32.2 | 27.6 | 34.1 | 37.7 | 25.8 |

Compared to the test solution 22 containing no sodium chondroitin sulfate having a weight-average-molecular weight of about 56,000, in the test solutions 4 and 5 containing sodium chondroitin sulfate having a weight-average-molecular weight of about 56,000, the residual liquid amount decreased even though the viscosity increased. In addition, it was confirmed that, in the test solutions 6 and 14 containing neostigmine methyl sulfate or taurine in the test solution 4, and the test solutions 11 and 12 containing d-α-tocopherol acetate or retinol palmitate in the test solution 5, the residual liquid amount was significantly reduced, making it easier to use up the ophthalmic composition in the eye drop bottle.

### [Formulation Examples]

Eye drops were prepared according to a general method using the formulations shown in the following Tables 15 to 19. Here, the units of the component amounts in the following Tables 15 to 19 are w/v% unless specified in the tables.

**[Table 15]**

| Component name | Formul ation Examp le 1 | Formul ation Examp le 2 | Formul ation Examp le 3 | Formul ation Examp le 4 | Formul ation Examp le 5 | Formul ation Examp le 6 | Formul ation Examp le 7 | Formul ation Examp le 8 | Formul ation Examp le 9 | Formul ation Examp le 10 |
|---|---|---|---|---|---|---|---|---|---|---|
| Sodium chondroitin sulfate (weight-average -molecular weight of about 56,000) | 0.5 | 0.5 | 3.0 | 0.5 | 3.0 | 0.3 | 1 | 0.1 | 0.5 | 1 |
| Tetrahydrozolin e hydrochloride | 0.01 | - | - | - | - | - | - | - | 0.05 | - |
| Neostigmine methyl sulfate | - | 0.001 | - | - | - | - | - | - | 0.005 | - |
| Epsilon-aminoc aproic acid | - | - | - | - | 0.5 | - | - | - | 1 | 2 |
| Allantoin | - | - | 0.3 | - | - | - | - | - | - | 0.1 |
| Dipotassium glycyrrhizinate | 0.25 | - | - | - | - | - | - | - | 0.1 | 0.15 |
| Chlorphenirami ne maleate | 0.03 | 0.02 | - | - | 0.03 | - | - | - | - | 0.03 |
| Flavin adenine dinucleotide sodium | - | - | - | - | - | - | - | - | - | 0.05 |
| Retinol palmitate | - | 35,000 units | - | - | 50,000 units | - | - | - | 50,000 units | - |
| Pyridoxine hydrochloride | 0.1 | 0.05 | 0.05 | - | - | - | - | 0.1 | 0.05 | 0.1 |
| d-α-tocopherol acetate | - | 0.035 | - | - | 0.05 | - | - | 0.03 | - | 0.05 |
| Potassium L-aspartate | - | - | - | - | - | - | 1.000 | - | 1 | - |
| Magnesium/pota ssium L-aspartate | - | - | - | - | - | - | - | 0.2 | - | - |
| Taurine | - | 1 | 0.5 | - | - | 0.5 | - | 0.5 | 1 | - |
| Sodium chloride | - | - | - | 0.08 | - | 0.08 | 0.1 | - | - | - |
| Potassium chloride | - | - | - | 0.45 | - | 0.4 | 0.3 | - | - | - |
| 1-menthol | 0.01 | - | 0.04 | 0.015 | 0.02 | 0.008 | 0.05 | 0.002 | 0.002 | - |
| d-camphor | 0.005 | - | 0.01 | - | - | - | - | - | 0.003 | - |
| d-borneol | 0.003 | - | - | 0.015 | - | - | - | - | - | - |
| Geraniol | - | 0.005 | 0.003 | - | - | - | 0.002 | - | - | - |
| Eucalyptus oil | - | - | - | - | 0.001 | - | - | 0.002 | - | - |
| Bergamot oil | - | - | - | - | 0.001 | - | - | - | - | - |
| Mint oil | - | 0.001 | - | - | - | - | - | 0.002 | - | - |
| Polyoxyethylene hydrogenated castor oil | - | 0.2 | - | - | 0.5 | 0.3 | - | - | 0.1 | - |
| Polyoxyethylene castor oil | 0.1 | - | - | 0.3 | - | 0.1 | - | - | - | - |
| Polysorbate 80 | 0.5 | - | 0.3 | - | 0.2 | - | 0.3 | 0.5 | - | - |
| Polyoxyl stearate | - | - | - | - | - | 0.05 | - | - | - | - |
| Polyoxyethylene polyoxypropyle ne glycol | - | 0.1 | - | 0.1 | - | - | 0.05 | - | - | - |
| Benzalkonium chloride | - | - | 0.01 | - | - | - | - | 0.01 | 0.01 | - |
| Benzethonium chloride | - | - | - | - | 0.01 | - | - | - | - | - |
| Chlorhexidine gluconate | - | - | - | - | - | - | 0.02 | - | - | - |
| Polyhexanide hydrochloride | - | - | - | - | - | 1 ppm | - | - | - | 1.5 ppm |
| Chlorobutanol | - | - | 0.15 | - | - | - | - | - | - | 0.3 |
| Zinc chloride | - | 0.0002 5 | - | - | - | - | - | - | - | - |
| Edetate sodium | 0.01 | 0.05 | - | - | 0.05 | - | 0.01 | 0.01 | 0.05 | 0.05 |
| Boric acid | 1.5 | 1.8 | 1 | - | 1.5 | 1 | - | 1 | 0.4 | 1 |
| Borax | 0.5 | 0.35 | - | - | 0.3 | 0.2 | - | 0.1 | 0.1 | 0.2 |
| Dibasic sodium phosphate | - | - | - | - | - | - | 0.15 | - | - | - |
| Sodium dihydrogen phosphate | - | - | - | - | - | - | 0.2 | - | - | - |
| Citric acid | - | - | - | - | - | - | - | - | 0.1 | - |
| Sesame oil | - | - | - | 0.1 | - | 0.05 | 0.05 | - | - | - |
| Propylene glycol | - | - | - | - | - | 0.1 | - | - | - | - |
| Concentrated glycerin | - | - | - | 0.1 | - | - | - | - | - | - |
| Sodium hyaluronate | - | - | 0.1 | - | 0.02 | 0.3 | - | 0.1 | - | - |
| L-arginine | - | - | 1 | - | - | - | - | 0.5 | - | - |
| Polyvinylpyrroli done | - | - | - | - | - | - | - | - | 0.5 | - |
| Hydroxyethyl cellulose | - | - | - | 0.25 | - | 0.05 | 0.6 | - | - | 0.1 |
| Hypromellose | - | - | - | 0.25 | - | - | - | - | - | - |
| Carboxyvinyl polymer | - | - | - | - | - | - | 0.1 | - | - | - |
| Dibutyl hydroxy toluene | - | - | - | - | 0.01 | - | - | - | 0.005 | - |
| Hydrochloric acid | Approp riate amount | Approp riate amount | Approp riate amount | Approp riate amount | Approp riate amount | Approp riate amount | Approp riate amount | Approp riate amount | Approp riate amount | Approp riate amount |
| Sodium hydroxide | Approp riate amount | Approp riate amount | Approp riate amount | Approp riate amount | Approp riate amount | Approp riate amount | Approp riate amount | Approp riate amount | Approp riate amount | Approp riate amount |
| Purified water | Residu al amount | Residu al amount | Residu al amount | Residu al amount | Residu al amount | Residu al amount | Residu al amount | Residu al amount | Residu al amount | Residu al amount |
| Total amount | 100 mL | 100 mL | 100 mL | 100 mL | 100 mL | 100 mL | 100 mL | 100 mL | 100 mL | 100 mL |
| pH | 7.0 | 6.5 | 5.7 | 7.2 | 6.9 | 7.0 | 7.5 | 7.3 | 7.0 | 5.5 |

**[Table 16]**

| Component name | Formul ation Examp le 11 | Formul ation Examp le 12 | Formul ation Examp le 13 | Formul ation Examp le 14 | Formul ation Examp le 15 | Formul ation Examp le 16 | Formul ation Examp le 17 | Formul ation Examp le 18 | Formul ation Examp le 19 | Formul ation Examp le 20 |
|---|---|---|---|---|---|---|---|---|---|---|
| Sodium chondroitin sulfate (weight-average -molecular weight of about 56,000) | 0.5 | 1 | 0.1 | 0.25 | 0.1 | 0.5 | 0.25 | 0.4 | 0.25 | 0.15 |
| Tetrahydrozolin e hydrochloride | 0.01 | 0.03 | 0.1 | 0.01 | 0.01 | 0.02 | - | - | - | 0.02 |
| Neostigmine | - | 0.005 | - | 0.005 | 0.005 | - | - | - | - | 0.0025 |
| methyl sulfate | | | | | | | | | | |
| Epsilon-aminoc aproic acid | - | 1 | 1 | 1 | 1 | - | - | - | 1 | - |
| Allantoin | 0.1 | - | - | - | - | - | 0.1 | - | - | - |
| Dipotassium glycyrrhizinate | - | 0.1 | 0.25 | - | - | 0.25 | 0.1 | - | - | 0.25 |
| Sodium azulene sulfonate | - | - | - | - | - | - | - | 0.02 | - | - |
| Berberine chloride | - | - | - | - | - | 0.01 | - | 0.01 | - | - |
| Zinc sulfate | - | 0.05 | - | - | - | - | 0.1 | - | - | 0.1 |
| Chlorphenirami ne maleate | - | 0.03 | 0.03 | 0.03 | 0.03 | 0.02 | 0.01 | 0.03 | - | - |
| Flavin adenine dinucleotide sodium | - | - | 0.05 | - | - | - | - | 0.01 | 0.05 | - |
| Cyanocobalami n | - | 0.01 | - | - | - | - | - | - | - | - |
| Retinol palmitate | - | 50,000 units | - | 100,00 0 units | 50,000 units | 10,000 units | - | 150,00 0 units | - | - |
| Pyridoxine hydrochloride | - | 0.05 | 0.1 | - | 0.01 | 0.08 | - | - | 0.01 | - |
| d-α-tocopherol acetate | 0.03 | - | - | 0.05 | 0.03 | - | 0.025 | 0.01 | - | - |
| Potassium L-aspartate | 0.5 | 0.5 | 0.5 | - | 0.8 | - | - | 0.1 | 1 | - |
| Magnesium/pota ssium L-aspartate | - | - | - | 0.800 | - | - | 1 | - | 2 | - |
| Taurine | 0.5 | 0.5 | 1 | 0.5 | 1 | - | 0.5 | 0.1 | 1 | - |
| Sodium chloride | - | 0.008 | - | - | - | - | - | - | - | - |
| Potassium chloride | - | - | - | - | - | - | 0.1 | - | - | - |
| 1-menthol | 0.02 | 0.01 | - | 0.005 | 0.008 | 0.001 | 0.005 | - | 0.0045 | - |
| d-camphor | - | 0.001 | - | - | - | - | - | - | 0.001 | - |
| d-borneol | - | 0.001 | - | 0.001 | - | - | - | - | - | - |
| Geraniol | 0.001 | - | 0.002 | - | - | - | 0.0001 | 0.003 | - | - |
| Eucalyptus oil | 0.001 | - | - | 0.001 | - | - | - | 0.003 | - | - |
| Bergamot oil | - | - | - | 0.001 | - | 0.0005 | - | - | - | - |
| Mint oil | - | - | - | - | 0.001 | 0.0005 | - | - | - | 0.001 |
| Polyoxyethylene hydrogenated castor oil | - | - | 0.3 | 0.3 | 0.4 | - | - | - | 0.2 | - |
| Polyoxyethylene castor oil | - | - | - | - | - | - | 0.1 | - | - | - |
| Polysorbate 80 | 0.2 | - | - | 0.2 | 0.2 | - | 0.3 | 0.5 | 0.1 | 0.25 |
| Polyoxyl | - | - | - | - | - | - | 0.1 | - | - | - |
| stearate | | | | | | | | | | |
| Polyoxyethylene polyoxypropyle ne glycol | 0.05 | - | - | - | - | - | - | - | - | - |
| Benzalkonium chloride | - | 0.01 | - | - | - | - | - | - | - | - |
| Chlorhexidine gluconate | - | - | - | - | - | - | - | - | 0.005 | - |
| Polyhexanide hydrochloride | 1 ppm | - | 1 ppm | - | - | - | - | - | - | - |
| Chlorobutanol | 0.1 | 0.2 | 0.1 | - | - | - | 0.2 | - | - | - |
| Zinc chloride | - | - | - | - | - | - | - | - | - | 0.001 |
| Edetate sodium | 0.01 | 0.05 | 0.01 | 0.05 | 0.05 | - | 0.01 | 0.01 | 0.05 | 0.05 |
| Boric acid | 1.2 | 0.2 | 0.3 | 0.1 | 0.5 | 0.3 | 0.1 | 1 | 0.4 | 0.1 |
| Borax | 0.3 | 0.1 | 0.05 | 0.025 | - | - | 0.01 | 0.1 | 0.1 | 0.2 |
| Dibasic sodium phosphate | - | - | - | - | - | - | - | - | - | 0.15 |
| Sodium dihydrogen phosphate | - | - | - | - | - | - | - | - | - | 0.2 |
| Citric acid | - | - | - | - | - | - | - | - | 0.1 | - |
| Trometamol | - | - | - | - | 0.3 | - | - | - | - | - |
| Sesame oil | - | - | - | - | - | - | 0.001 | - | - | - |
| Propylene glycol | - | - | - | - | 0.2 | - | | - | - | - |
| Concentrated glycerin | 0.1 | - | - | - | - | - | - | - | - | - |
| Sodium hyaluronate | 0.005 | 0.002 | - | - | - | - | - | - | - | 0.01 |
| Dextran | - | - | - | 0.05 | - | - | - | - | - | - |
| L-arginine | - | - | - | - | - | - | - | - | - | 0.1 |
| Polyvinylpyrroli done | - | - | - | - | - | - | 0.1 | - | - | - |
| Hydroxyethyl cellulose | - | - | 0.1 | - | - | - | - | - | - | - |
| Hydroxypropyl methylcellulose | 0.1 | - | - | - | - | - | 0.2 | - | 0.01 | - |
| Dibutyl hydroxy toluene | - | - | - | 0.01 | 0.01 | - | - | - | - | - |
| Hydrochloric acid | Approp riate amount | Approp riate amount | Approp riate amount | Approp riate amount | Approp riate amount | Approp riate amount | Approp riate amount | Approp riate amount | Approp riate amount | Approp riate amount |
| Sodium hydroxide | Approp riate amount | Approp riate amount | Approp riate amount | Approp riate amount | Approp riate amount | Approp riate amount | Approp riate amount | Approp riate amount | Approp riate amount | Approp riate amount |
| Purified water | Residu al amount | Residu al amount | Residu al amount | Residu al amount | Residu al amount | Residu al amount | Residu al amount | Residu al amount | Residu al amount | Residu al amount |
| Total amount | 100 mL | 100 mL | 100 mL | 100 mL | 100 mL | 100 mL | 100 mL | 100 mL | 100 mL | 100 mL |
| pH | 5.8 | 6.5 | 5.7 | 6.2 | 5.8 | 7 | 5.3 | 6 | 7 | 5.5 |

**[Table 17]**

| Component name | Formulation Example 21 | Formulation Example 22 | Formulation Example 23 | Formulation Example 24 | Formulation Example 25 | Formulation Example 26 |
|---|---|---|---|---|---|---|
| Sodium chondroitin sulfate (weight-average-molecular weight of about 56,000) | 0.2 | 1 | 0.5 | 0.3 | 0.5 | 0.5 |
| Potassium L-aspartate | - | - | 1 | - | 0.5 | 0.5 |
| Magnesium/potassium L-aspartate | - | 1 | - | - | 0.5 | - |
| Taurine | - | 0.5 | - | 1 | - | 0.5 |
| Sodium chloride | - | - | 0.08 | - | - | 0.05 |
| Potassium chloride | 0.6 | 0.4 | 0.1 | 0.3 | 0.45 | 0.4 |
| 1-menthol | 0.04 | 0.002 | 0.005 | 0.015 | - | 0.005 |
| d-camphor | 0.01 | - | - | 0.003 | - | 0.003 |
| Geraniol | 0.003 | - | - | - | - | - |
| Eucalyptus oil | - | 0.002 | - | - | - | - |
| Bergamot oil | - | - | - | 0.001 | - | - |
| Mint oil | - | 0.002 | - | - | - | - |
| Polyoxyethylene hydrogenated castor oil | - | - | 0.1 | 0.1 | - | - |
| Polyoxyethylene castor oil | - | - | - | - | 0.3 | - |
| Polysorbate 80 | 0.3 | 0.2 | - | - | - | 0.2 |
| Polyoxyl stearate | - | - | - | - | 0.1 | - |
| Polyoxyethylene polyoxypropylene glycol | - | - | - | - | 0.05 | - |
| Benzalkonium chloride | - | 0.01 | 0.01 | - | - | - |
| Chlorhexidine gluconate | - | - | - | - | 0.02 | - |
| Polyhexanide hydrochloride | 1 ppm | - | - | 1 ppm | - | - |
| Chlorobutanol | 0.15 | - | - | - | - | - |
| Zinc chloride | - | - | - | - | - | 0.0001 |
| Edetate sodium | - | 0.005 | 0.05 | 0.005 | 0.01 | 0.05 |
| Boric acid | 1.5 | 1 | 0.4 | 1 | - | 1 |
| Borax | - | 0.1 | 0.1 | 0.2 | - | 0.2 |
| Dibasic sodium phosphate | - | - | - | - | 0.15 | - |
| Sodium dihydrogen phosphate | - | - | - | - | 0.1 | - |
| Citric acid | - | - | 0.1 | - | - | 0.1 |
| Sesame oil | - | - | - | - | 0.05 | - |
| Concentrated glycerin | - | - | - | - | - | 0.1 |
| Sodium hyaluronate | - | 0.02 | - | - | - | - |
| L-arginine | - | 0.5 | - | - | - | - |
| Polvvinvrrolidone | - | - | 0.5 | 0.1 | - | - |
| Hydroxyethyl cellulose | 0.07 | - | - | - | 0.2 | - |
| Hydroxypropyl methylcellulose | - | - | 0.1 | - | 0.2 | 0.3 |
| Hydrochloric acid | Appropriate | Appropriate | Appropriate | Appropriate | Appropriate | Appropriate |
| | amount | amount | amount | amount | amount | amount |
| Sodium hydroxide | Appropriate amount | Appropriate amount | Appropriate amount | Appropriate amount | Appropriate amount | Appropriate amount |
| Purified water | Residual amount | Residual amount | Residual amount | Residual amount | Residual amount | Residual amount |
| Total amount | 100 mL | 100 mL | 100 mL | 100 mL | 100 mL | 100 mL |
| pH | 6.5 | 7.2 | 6.8 | 7 | 7.5 | 6.2 |

**[Table 18]**

| Component name | Formulati on Example 27 | Formulati on Example 28 | Formulati on Example 29 | Formulati on Example 30 | Formulati on Example 31 | Formulati on Example 32 | Formulati on Example 33 | Formulati on Example 34 |
|---|---|---|---|---|---|---|---|---|
| Sodium chondroitin sulfate (MW of about 56,000) | - | 0.25 | 0.05 | 0.4 | - | 0.25 | 0.05 | 0.4 |
| Sodium chondroitin sulfate (MW of about 45,000) | 0.5 | - | - | - | 0.5 | - | - | - |
| Sodium chondroitin sulfate (MW of about 25,000) | - | 0.25 | 0.45 | 0.1 | - | 0.25 | 0.45 | 0.1 |
| Tetrahydrozoline hydrochloride | 0.01 | 0.01 | 0.01 | 0.01 | 0.05 | 0.05 | 0.05 | 0.05 |
| Neostigmine methyl sulfate | - | - | - | - | 0.005 | 0.005 | 0.005 | 0.005 |
| Epsilon-aminocap roic acid | - | - | - | - | 1 | 1 | 1 | 1 |
| Dipotassium glycyrrhizinate | 0.25 | 0.25 | 0.25 | 0.25 | 0.1 | 0.1 | 0.1 | 0.1 |
| Chlorpheniramine maleate | 0.03 | 0.03 | 0.03 | 0.03 | - | - | - | - |
| Retinol palmitate | - | - | - | - | 50,000 units | 50,000 units | 50,000 units | 50,000 units |
| Pyridoxine hydrochloride | 0.1 | 0.1 | 0.1 | 0.1 | 0.05 | 0.05 | 0.05 | 0.05 |
| Potassium L-aspartate | - | - | - | - | 1 | 1 | 1 | 1 |
| Taurine | - | - | - | - | 1 | 1 | 1 | 1 |
| 1-menthol | 0.01 | 0.01 | 0.01 | 0.01 | 0.002 | 0.002 | 0.002 | 0.002 |
| d-camphor | 0.005 | 0.005 | 0.005 | 0.005 | 0.003 | 0.003 | 0.003 | 0.003 |
| d-borneol | 0.003 | 0.003 | 0.003 | 0.003 | - | - | - | - |
| Polyoxyethylene hydrogenated castor oil | - | - | - | - | 0.1 | 0.1 | 0.1 | 0.1 |
| Polyoxyethylene castor oil | 0.1 | 0.1 | 0.1 | 0.1 | - | - | - | - |
| Polysorbate 80 | 0.5 | 0.5 | 0.5 | 0.5 | - | - | - | - |
| Benzalkonium chloride | - | - | - | - | 0.01 | 0.01 | 0.01 | 0.01 |
| Edetate sodium | 0.01 | 0.01 | 0.01 | 0.01 | 0.05 | 0.05 | 0.05 | 0.05 |
| Boric acid | 1.5 | 1.5 | 1.5 | 1.5 | 0.4 | 0.4 | 0.4 | 0.4 |
| Borax | 0.5 | 0.5 | 0.5 | 0.5 | 0.1 | 0.1 | 0.1 | 0.1 |
| Citric acid | - | - | - | - | 0.1 | 0.1 | 0.1 | 0.1 |
| Polyvinylpyrrolid one | - | - | - | - | 0.5 | 0.5 | 0.5 | 0.5 |
| Dibutyl hydroxy toluene | - | - | - | - | 0.005 | 0.005 | 0.005 | 0.005 |
| Hydrochloric acid | Appropri ate amount | Appropri ate amount | Appropri ate amount | Appropri ate amount | Appropri ate amount | Appropri ate amount | Appropri ate amount | Appropri ate amount |
| Sodium hydroxide | Appropri ate amount | Appropri ate amount | Appropri ate amount | Appropri ate amount | Appropri ate amount | Appropri ate amount | Appropri ate amount | Appropri ate amount |
| Purified water | Residual amount | Residual amount | Residual amount | Residual amount | Residual amount | Residual amount | Residual amount | Residual amount |
| Total amount | 100 mL | 100 mL | 100 mL | 100 mL | 100 mL | 100 mL | 100 mL | 100 mL |
| pH | 7.0 | 7.0 | 7.0 | 7.0 | 7.0 | 7.0 | 7.0 | 7.0 |

**[Table 19]**

| Component name | Formulati on Example 35 | Formulati on Example 36 | Formulati on Example 37 | Formulati on Example 38 | Formulati on Example 39 | Formulati on Example 40 | Formulati on Example 41 | Formulati on Example 42 |
|---|---|---|---|---|---|---|---|---|
| Sodium chondroitin sulfate (MW of about 56,000) | 0.5 | 0.5 | 0.5 | 0.5 | 0.5 | 0.5 | 0.5 | 0.5 |
| Neostigmine methyl sulfate | - | 0.005 | - | - | - | - | - | - |
| Epsilon-aminocap roic acid | - | 1 | - | - | - | - | - | - |
| Chlorpheniramine maleate | - | 0.03 | - | - | 0.03 | 0.03 | 0.03 | 0.03 |
| Retinol palmitate | 50,000 units | 50,000 units | 50,000 units | - | - | - | - | - |
| d-α-tocopherol acetate | 0.05 | 0.045 | 0.05 | - | 0.005 | - | 0.005 | - |
| Potassium L-aspartate | - | 0.8 | - | - | 0.2 | - | 0.2 | - |
| Taurine | - | 1 | - | - | 0.1 | - | 0.1 | - |
| Sodium chloride | 0.1 | - | - | 0.1 | - | - | - | - |
| Potassium chloride | 0.2 | - | - | 0.4 | - | - | - | - |
| 1-menthol | - | 0.002 | - | - | 0.03 | 0.015 | 0.03 | 0.015 |
| d-borneol | - | 0.01 | - | - | - | - | - | - |
| Polyoxyethylene hydrogenated castor oil | - | 0.3 | - | 0.5 | - | - | 0.2 | - |
| Polysorbate 80 | - | 0.3 | - | 0.5 | 0.3 | 0.3 | - | 0.3 |
| Polyoxyethylene polyoxypropylene glycol | 0.55 | - | 0.55 | - | - | - | - | - |
| Benzalkonium chloride | - | - | - | - | 0.01 | 0.01 | - | 0.01 |
| Sodium bisulfite | 0.4 | - | 0.4 | 0.2 | - | - | - | - |
| Polyhexanide hydrochloride | - | - | - | - | - | - | 1 ppm | - |
| Sodium cromoglicate | - | - | - | - | 1 | 1 | - | - |
| Tranilast | - | - | - | - | - | - | 0.5 | 0.5 |
| Pranoprofen | - | - | - | - | 0.05 | 0.05 | 0.05 | 0.05 |
| Edetate sodium | 0.05 | 0.1 | 0.05 | 0.1 | 0.05 | 0.05 | 0.05 | 0.05 |
| Boric acid | 1 | 1 | 1 | 0.5 | 1 | 0.8 | 1 | 0.8 |
| Borax | 0.02 | 0.02 | 0.02 | 0.05 | 0.1 | 0.05 | 0.1 | 0.05 |
| Trometamol | - | 0.1 | 0.1 | - | - | - | - | - |
| Liquid paraffin | - | - | - | 0.1 | - | - | - | - |
| Propylene glycol | - | 0.2 | 0.3 | - | - | - | - | - |
| Polyvinylpyrrolid one | - | - | 0.2 | 0.2 | - | - | 1.5 | 3 |
| Hydroxypropyl methylcellulose | - | - | 0.1 | 0.1 | - | - | - | - |
| Monoethanolami ne | - | - | - | - | - | - | 0.2 | 0.1 |
| Dibutyl hydroxy toluene | 0.01 | 0.01 | 0.01 | 0.01 | 0.01 | 0.01 | 0.01 | 0.01 |
| Hydrochloric acid | Appropri ate amount | Appropri ate amount | Appropri ate amount | Appropri ate amount | Appropri ate amount | Appropri ate amount | Appropri ate amount | Appropri ate amount |
| Sodium hydroxide | Appropri ate amount | Appropri ate amount | Appropri ate amount | Appropri ate amount | Appropri ate amount | Appropri ate amount | Appropri ate amount | Appropri ate amount |
| Purified water | Residual amount | Residual amount | Residual amount | Residual amount | Residual amount | Residual amount | Residual amount | Residual amount |
| Total amount | 100 mL | 100 mL | 100 mL | 100 mL | 100 mL | 100 mL | 100 mL | 100 mL |
| pH | 7.0 | 6.0 | 7.0 | 7.0 | 7.0 | 6.0 | 8.0 | 7.5 |

This concludes the description of the first present invention, and now the second present invention will be described.

The second present invention relates to an ophthalmic composition.

### Background Art

Chondroitin sulfate or salts thereof are a type of acidic mucopolysaccharide, and are added to the ophthalmic formulation for promoting energy metabolism, promoting metabolism and cell respiration to relieve eye fatigue, replenishing lachrymal fluid components, and the like (for example, Patent Literature 2-1).

### Citation List

### Patent Literature

[Patent Literature 2-1] Japanese Unexamined Patent Publication No. 2011-148791

### [Summary of Second Present Invention]

### [Technical Problem of Second Present Invention]

The inventors found a new problem in the ophthalmic composition containing chondroitin sulfate or salts thereof having a specific weight-average-molecular weight that its viscosity decreases and stability decreases when exposed to light. An object of the second present invention is to provide a stable ophthalmic composition which contains chondroitin sulfate or salts thereof having a specific weight-average-molecular weight and of which a change in viscosity due to light is reduced.

### [Solution to Problem of Second Present Invention]

Surprisingly, the inventors found that an ophthalmic composition containing sodium chondroitin sulfate having a weight-average-molecular weight of 40,000 to 70,000 and a specific component reduces a change in viscosity due to light.

The second present invention provides, for example, the following inventions.
[1] An ophthalmic composition containing at least one selected from the group consisting of chondroitin sulfate and salts thereof which have a weight-average-molecular weight of 40,000 to 70,000, and at least one selected from the group consisting of an antihistamine, a zinc salt, a decongestant, hydroxyethyl cellulose and salts thereof, and a polyvinyl-based polymer compound.
[2] The ophthalmic composition according to [1],
   wherein the antihistamine is at least one selected from the group consisting of chlorpheniramine and salts thereof, the decongestant is at least one selected from the group consisting of tetrahydrozoline and salts thereof, and the polyvinyl-based polymer compound is polyvinylpyrrolidone.

### [Effects of second present invention]

According to the second present invention, it is possible to provide a stable ophthalmic composition which contains chondroitin sulfate or salts thereof having a specific weight-average-molecular weight and of which a change in viscosity due to light.

### [Descriptions of Embodiments of Second Present Invention]

Hereinafter, embodiments for implementing a second present invention will be described in detail. However, the second present invention is not limited to the following embodiments.

In this specification, unless otherwise specified, the content unit "%" indicates "w/v%" and is synonymous with "g/100 mL."

The ophthalmic composition according to the present embodiment contains at least one selected from the group consisting of chondroitin sulfate and salts thereof which have a weight-average-molecular weight of 40,000 to 70,000 (simply referred to as a "component (A)") and at least one selected from the group consisting of an antihistamine, a zinc salt, a decongestant, hydroxyethyl cellulose and salts thereof, and a polyvinyl-based polymer compound (simply referred to as a "component (B)").

As confirmed in test examples to be described below, the ophthalmic composition according to the present embodiment exhibits, in addition to an effect of reducing a change in viscosity due to light, an effect of reducing the amount of liquid remaining in pipes during filling of containers or during liquid transfer, an effect of increasing preservative efficacy, an effect of making it easier to blink regardless of the viscosity of a formulation when instilled in the eyes (even if it is a high-viscosity formulation), an effect of reducing discomfort during blinking regardless of the viscosity of a formulation when instilled in the eyes (even if a high-viscosity formulation), an effect of suppressing coloring of a formulation due to ultraviolet rays, an effect of increasing a cell survival percentage, and an effect of reducing eye cell damage due to external stimuli (blinking, stimuli derived from contact lenses (when inserting and removing them, or while wearing them), rubbing the eyes, and contamination with foreign substances (pollen, air contaminants, eyelashes, eye makeup-related foreign substances, other foreign substances, etc.)).

### [Chondroitin sulfate and salts thereof]

Chondroitin sulfate and salts thereof are not particularly limited as long as they are pharmaceutically, pharmacologically (medicinally) or physiologically acceptable.

Examples of salts of chondroitin sulfate include alkali metal salts and alkaline earth metal salts. Examples of alkali metal salts include sodium salts and potassium salts. Examples of alkaline earth metal salts include magnesium salts and calcium salts.

The chondroitin sulfate and salts thereof are preferably chondroitin sulfate and alkali metal salts of chondroitin sulfate, more preferably chondroitin sulfate and sodium chondroitin sulfate, and still more preferably sodium chondroitin sulfate.

The chondroitin sulfate and salts thereof may be natural products or synthetic products, and generally, chondroitin sulfate and salts thereof derived from animals (preferably mammals, fish, mollusks, etc.; and more preferably cows, sharks, squids, rays, etc.), which are natural products, are preferably used, chondroitin sulfate and salts thereof derived from sharks and/or rays are more preferably used, and chondroitin sulfate and salts thereof derived from sharks are still more preferably used.

Commercially available chondroitin sulfates and salts thereof can be used. The chondroitin sulfates and salts thereof may be used alone or two or more thereof may be used in combination. In addition, as the chondroitin sulfate and salts thereof, chondroitin sulfate and salts thereof which have a weight-average-molecular weight of 40,000 to 70,000 defined in this specification and chondroitin sulfate and salts thereof which have a weight-average-molecular weight outside the range defined in this specification can be used in combination. For example, sodium chondroitin sulfate having a weight-average-molecular weight of 56,000 and sodium chondroitin sulfate having a weight-average-molecular weight of 25,000 can be used in combination. When chondroitin sulfate and salts thereof which have a weight-average-molecular weight of 40,000 to 70,000 defined in this specification and chondroitin sulfate and salts thereof which have a weight-average-molecular weight outside the range defined in this specification are used in combination, chondroitin sulfate and salts thereof which have a weight-average-molecular weight of 40,000 to 70,000 may be contained as raw materials.

In this specification, the "weight-average-molecular weight" can be determined by using gel permeation chromatography in which a multi-angle light scattering detector (MALS detector) and a differential refractive index detector (RI detector) are connected online. Specifically, the following conditions are presented.

### <Preparation of standard sample>

A sample is obtained by adding 10 mL of a 0.1 M sodium nitrate aqueous solution to 5 mg of chondroitin sulfate or salts thereof, gently stirring it at room temperature, and completely dissolving it.

### <Conditions for weight-average-molecular weight measurement>

Device: gel permeation chromatograph-multi-angle light scatterometer
Detector: differential refractive index detector (Optilab rEX commercially available from Wyatt Technology)
   Multi-angle light scattering detector (DAWN HELEOS commercially available from Wyatt Technology)
Column: Shodex OHpak SB-806M HQ 2 columns (ϕ7.8 mm×30 cm, commercially available from Showa Denko K.K.)
Solvent: 0.1 M sodium nitrate aqueous solution
Flow rate: 0.7 mL/min
Column temperature: 23°C
Detector temperature: 23°C
Injection volume: 0.2 mL
Data processing: commercially available from Wyatt Technology data processing system (ASTRA)

The weight-average-molecular weight of the chondroitin sulfate and salts thereof calculated by the above method is not particularly limited as long as it is in a range of 40,000 to 70,000. Examples of lower limit values of the weight-average-molecular weight include 41,000 or more, 42,000 or more, 43,000 or more, 44,000 or more, 45,000 or more, 46,000 or more, 47,000 or more, 48,000 or more, 49,000 or more, and 50,000 or more. Examples of upper limit values of the weight-average-molecular weight include 69,000 or less, 68,000 or less, 67,000 or less, 66,000 or less, 65,000 or less, 64,000 or less, 63,000 or less, 62,000 or less, 61,000 or less, and 60,000 or less. Examples of ranges of the weight-average-molecular weight include 41,000 to 69,000, 42,000 to 68,000, 43,000 to 67,000, 44,000 to 66,000, 45,000 to 65,000, 46,000 to 64,000, 47,000 to 63,000, 48,000 to 62,000, 49,000 to 61,000, and 50,000 to 60,000.

The content of the component (A) in the ophthalmic composition according to the present embodiment is not particularly limited, and is appropriately set depending on the types and contents of other formulation components, applications and formulation forms of the ophthalmic composition, and the like. In order to more significantly exhibit the effects of the second present invention, the content of the component (A) based on the total amount of the ophthalmic composition is, for example, generally 0.001 to 5 w/v%, preferably 0.005 to 5 w/v%, more preferably 0.008 to 4 w/v%, more preferably 0.01 to 3 w/v%, still more preferably 0.05 to 2 w/v%, particularly preferably 0.1 to 1 w/v%, and preferably 0.3 to 1 w/v%.

### [Antihistamines]

Antihistamines are compounds and salts thereof which have an antihistamine effect. The antihistamines are not particularly limited as long as they are pharmaceutically, pharmacologically (medicinally) or physiologically acceptable.

Specific examples of antihistamines include chlorpheniramine, iproheptine, diphenhydramine, ketotifen, olopatadine, levocabastine, and salts thereof. The antihistamines are preferably chlorpheniramine and salts thereof, and more preferably chlorpheniramine maleate.

Commercially available antihistamines can be used. The antihistamines may be used alone or two or more thereof may be used in combination.

When antihistamines are used as the component (B), content of the antihistamines in the ophthalmic composition according to the present embodiment is not particularly limited, and is appropriately set depending on the type of the antihistamines, the types and contents of other formulation components, applications and formulation forms of the ophthalmic composition, and the like. Regarding the content of the antihistamines, in order to more significantly exhibit the effects of the second present invention, the total content of the antihistamines based on the total amount of the ophthalmic composition is preferably 0.001 to 1 w/v%, more preferably 0.003 to 0.5 w/v%, still more preferably 0.005 to 0.1 w/v%, yet more preferably 0.01 to 0.05 w/v%, and particularly preferably 0.02 to 0.04 w/v% (for example, 0.03 w/v%).

When antihistamines are used as the component (B), the content proportion of the antihistamines with respect to the component (A) in the ophthalmic composition according to the present embodiment is not particularly limited, and is appropriately set depending on the types of the component (A) and the antihistamines, the types and contents of other formulation components, applications and formulation forms of the ophthalmic composition, and the like. Regarding the content proportion of the antihistamines with respect to the component (A), in order to further improve the effects of the second present invention, for example, the total content of the antihistamines based on the total content of 1 part by mass of the component (A) contained in the ophthalmic composition according to the present embodiment is preferably 0.0002 to 20 parts by mass, more preferably 0.001 to 10 parts by mass, and still more preferably 0.01 to 1 part by mass.

### [Zinc salt]

Zinc salts are not particularly limited as long as they are pharmaceutically, pharmacologically (medicinally) or physiologically acceptable.

The zinc salts are preferably zinc sulfate, zinc lactate or zinc chloride, and more preferably zinc sulfate. In addition, the zinc salts may be a hydrate (for example, zinc sulfate heptahydrate).

Commercially available zinc salts can be used. The zinc salts may be used alone or two or more thereof may be used in combination.

When zinc salts are used as the component (B), the content of the zinc salts in the ophthalmic composition according to the present embodiment is not particularly limited, and is appropriately set depending on the type of the zinc salts, the types and contents of other formulation components, applications and formulation forms of the ophthalmic composition, and the like. Regarding the content of the zinc salts, in order to more significantly exhibit the effects of the second present invention, the total content of the zinc salts based on the total amount of the ophthalmic composition is generally 0.00001 to 10 w/v%, preferably 0.0001 to 10 w/v%, more preferably 0.001 to 5 w/v%, still more preferably 0.005 to 3 w/v%, yet more preferably 0.01 to 1 w/v%, and particularly preferably 0.03 to 0.5 w/v%. In addition, the total content of the zinc salts may be 0.25 w/v%.

When zinc salts are used as the component (B), the content proportion of the zinc salts with respect to the component (A) in the ophthalmic composition according to the present embodiment is not particularly limited, and is appropriately set depending on the types of the component (A) and the zinc salts, the types and contents of other formulation components, applications and formulation forms of the ophthalmic composition, and the like. Regarding the content proportion of the zinc salts with respect to the component (A), in order to further improve the effects of the second present invention, for example, the total content of the zinc salts based on the total content of 1 part by mass of the component (A) contained in the ophthalmic composition according to the present embodiment is preferably 0.0002 to 1,000 parts by mass, more preferably 0.001 to 500 parts by mass, still more preferably 0.01 to 100 parts by mass, yet more preferably 0.05 to 50 parts by mass, yet more preferably 0.05 to 30 parts by mass, and particularly preferably 0.1 to 1 part by mass. In addition, the total content of the zinc salts based on the total content of 1 part by mass of the component (A) is 0.5 parts by mass.

### [Decongestant]

Decongestants are compounds and salts thereof that have an effect of decongesting the eyes. The decongestants are not particularly limited as long as they are pharmaceutically, pharmacologically (medicinally) or physiologically acceptable.

Specific examples of decongestants include imidazoline compounds and salts thereof such as tetrahydrozoline, naphazoline, and oxymetazoline (for example, hydrochloride, nitrate); and epinephrine, ephedrine, methylephedrine, phenylephrine and salts thereof (for example, hydrochloride). The decongestants are preferably imidazoline compounds and salts thereof, more preferably tetrahydrozoline, naphazoline and salts thereof, still more preferably tetrahydrozoline and salts thereof, and particularly preferably tetrahydrozoline hydrochloride (tetrahydrozoline hydrochloride).

Commercially available decongestants can be used. The decongestants may be used alone or two or more thereof may be used in combination.

When a decongestant is used as the component (B), the content of the decongestant in the ophthalmic composition according to the present embodiment is not particularly limited, and is appropriately set depending on the type of the decongestant, the types and contents of other formulation components, applications and formulation forms of the ophthalmic composition, and the like. Regarding the content of the decongestant, in order to more significantly exhibit the effects of the second present invention, the total content of the decongestant based on the total amount of the ophthalmic composition is preferably 0.0001 to 1 w/v%, more preferably 0.0005 to 0.5 w/v%, still more preferably 0.001 to 0.1 w/v%, and yet more preferably 0.002 to 0.1 w/v%.

When a decongestant is used as the component (B), the content proportion of the decongestant with respect to the component (A) in the ophthalmic composition according to the present embodiment is not particularly limited, and is appropriately set depending on the types of the component (A) and the decongestant, the types and contents of other formulation components, applications and formulation forms of the ophthalmic composition, and the like. Regarding the content proportion of the decongestant with respect to the component (A), in order to further improve the effects of the second present invention, for example, the total content of the decongestant based on the total content of 1 part by mass of the component (A) contained in the ophthalmic composition according to the present embodiment is preferably 0.0001 to 50 parts by mass, more preferably 0.0005 to 20 parts by mass, still more preferably 0.001 to 10 parts by mass, and yet more preferably 0.003 to 5 parts by mass.

### [Hydroxyethyl cellulose and salts thereof]

Hydroxyethyl cellulose and salts thereof are not particularly limited as long as they are pharmaceutically, pharmacologically (medicinally) or physiologically acceptable.

Examples of salts of hydroxyethyl cellulose include salts with organic bases (amine salts, basic ammonium salts such as arginine, etc.), salts with inorganic bases (alkali metal salts such as ammonium salts, sodium salts, and potassium salts, alkaline earth metal salts such as calcium salts and magnesium salts, aluminum salts, etc.), and the like, and among these, sodium salts, potassium salts, and calcium salts are more preferable, and sodium salts are particularly preferable.

Commercially available hydroxyethyl celluloses and salts thereof can be used. The hydroxyethyl celluloses and salts thereof may be used alone or two or more thereof may be used in combination.

When hydroxyethyl celluloses and salts thereof are used as the component (B), the content of the hydroxyethyl celluloses and salts thereof in the ophthalmic composition according to the present embodiment is not particularly limited, and appropriately set depending on the types of the hydroxyethyl celluloses and salts thereof, the types and contents of other formulation components, applications and formulation forms of the ophthalmic composition, and the like. Regarding the content of the hydroxyethyl celluloses and salts thereof, in order to more significantly exhibit the effects of the second present invention, the total content of the hydroxyethyl celluloses and salts thereof based on the total amount of the ophthalmic composition is preferably 0.0001 to 10 w/v%, more preferably 0.001 to 5 w/v%, still more preferably 0.005 to 3 w/v%, and yet more preferably 0.01 to 1 w/v%.

When hydroxyethyl celluloses and salts thereof are used as the component (B), the content proportion of the hydroxyethyl celluloses and salts thereof with respect to the component (A) in the ophthalmic composition according to the present embodiment is not particularly limited, and is appropriately set depending on the types of the component (A) and the hydroxyethyl celluloses and salts thereof, the types and contents of other formulation components, applications and formulation forms of the ophthalmic composition, and the like. Regarding the content proportion of the hydroxyethyl celluloses and salts thereof with respect to the component (A), in order to further improve the effects of the second present invention, for example, the total content of the hydroxyethyl celluloses and salts thereof based on the total content of 1 part by mass of the component (A) contained in the ophthalmic composition according to the present embodiment is preferably 0.0001 to 500 parts by mass, more preferably 0.001 to 100 parts by mass, still more preferably 0.005 to 50 parts by mass, and yet more preferably 0.01 to 30 parts by mass.

### [Polyvinyl-based polymer compound]

Polyvinyl-based polymer compounds are not particularly limited as long as they are pharmaceutically, pharmacologically (medicinally) or physiologically acceptable.

Examples of polyvinyl-based polymer compounds include polyvinyl alcohols (completely or partially saponified product), polyvinylpyrrolidone, and carboxyvinyl polymers. The molecular weight of the polyvinyl-based polymer that can be used in the second present invention is not limited, and for example, those having a weight-average-molecular weight of 5,000 to 5,000,000, preferably 10,000 to 3,000,000, and more preferably about 10,000 to 1,000,000 can be used. The K value (iscosity characteristic value) of polyvinylpyrrolidone is not particularly limited, and those having a K value of about 10 to 150 can be preferably used. Among these, in order to further improve the effects of the second present invention, polyvinylpyrrolidone is preferable, polyvinylpyrrolidone K25, polyvinylpyrrolidone K30, and polyvinylpyrrolidone K90 are more preferable, and polyvinylpyrrolidone K30 and polyvinylpyrrolidone K90 are still more preferable.

Commercially available polyvinyl-based polymer compounds can be used. The polyvinyl-based polymer compounds may be used alone or two or more thereof may be used in combination.

When a polyvinyl-based polymer compound is used as the component (B), the content of the polyvinyl-based polymer compound in the ophthalmic composition according to the present embodiment is not particularly limited, and is appropriately set depending on the type of the polyvinyl-based polymer compound, the types and contents of other formulation components, applications and formulation forms of the ophthalmic composition, and the like. Regarding the content of the polyvinyl-based polymer compound, in order to more significantly exhibit the effects of the second present invention, the total content of the polyvinyl-based polymer compound based on the total amount of the ophthalmic composition is preferably 0.0001 to 10 w/v%, more preferably 0.001 to 8 w/v%, still more preferably 0.005 to 5 w/v%, yet more preferably 0.01 to 3 w/v%, and particularly preferably 0.01 to 2 w/v%.

When a polyvinyl-based polymer compound is used as the component (B), the content proportion of the polyvinyl-based polymer compound with respect to the component (A) in the ophthalmic composition according to the present embodiment is not particularly limited, and is appropriately set depending on the types of the component (A) and the polyvinyl-based polymer compound, the types and contents of other formulation components, applications and formulation forms of the ophthalmic composition, and the like. Regarding the content proportion of the polyvinyl-based polymer compound with respect to the component (A), in order to further improve the effects of the second present invention, for example, the total content of the polyvinyl-based polymer compound based on the total content of 1 part by mass of the component (A) contained in the ophthalmic composition according to the present embodiment is preferably 0.0001 to 1,000 parts by mass, more preferably 0.001 to 500 parts by mass, still more preferably 0.005 to 100 parts by mass, and yet more preferably 0.01 to 50 parts by mass.

The ophthalmic composition according to the present embodiment may further contain a surfactant (C) (also referred to as a "component (C)"). When the ophthalmic composition further contains the component (C), the effects of the second present invention are more significantly exhibited. The surfactants are not particularly limited as long as they are pharmaceutically, pharmacologically (medicinally) or physiologically acceptable, and may be any of nonionic surfactants, amphoteric surfactants, anionic surfactants, and cationic surfactants.

Examples of nonionic surfactants include POE sorbitan fatty acid esters such as monolaurate POE(20) sorbitan (polysorbate 20), monopalmitate POE(20) sorbitan (polysorbate 40), monostearate POE(20) sorbitan (polysorbate 60), tristearate POE(20) sorbitan (polysorbate 65), and monooleate POE(20) sorbitan (polysorbate 80); POE hydrogenated castor oils such as POE(40) hydrogenated castor oil (polyoxyethylene hydrogenated castor oil 40), and POE(60) hydrogenated castor oil (polyoxyethylene hydrogenated castor oil 60); POE castor oils such as POE(3) hydrogenated castor oil (polyoxyethylene castor oil 3), POE(10) castor oil (polyoxyethylene castor oil 10), and POE(35) castor oil (polyoxyethylene castor oil 35); POE alkyl ethers such as POE(9) lauryl ether; POE-POP alkyl ethers such as POE(20)POP(4) cetyl ether; polyoxyethylene/polyoxypropylene block copolymers such as POE(196)POP(67) glycol (poloxamer 407, Pluronic F127), and POE(200)POP(70) glycol, and monostearate polyethylene glycol such as polyoxyl stearate 40. Here, in the compounds exemplified above, POE indicates polyoxyethylene, POP indicates polyoxypropylene, and the number in parentheses indicates the number of moles added.

Examples of amphoteric surfactants include alkyldiaminoethylglycine and salts thereof (for example, hydrochloride, etc.).

Examples of anionic surfactants include alkylbenzene sulfonate, alkyl sulfate, polyoxyethylene alkyl sulfate, aliphatic α-sulfomethyl ester, and α-olefin sulfonic acid.

Examples of cationic surfactants include cetylpyridinium chloride, benzalkonium chloride, and benzethonium chloride.

Among these surfactants, nonionic surfactants are preferable, and POE sorbitan fatty acid esters, POE hydrogenated castor oils, POE castor oils, and POE/POP block copolymers are more preferable. Commercially available surfactants can be used. The surfactants may be used alone or two or more thereof may be used in combination.

The content of the component (C) in the ophthalmic composition according to the present embodiment is not particularly limited, and is appropriately set depending on the type of the component (C), applications and formulation forms of the ophthalmic composition, and the like. Regarding the content of the component (C), in order to more significantly exhibit the effects of the second present invention, for example, the total content of the component (C) based on the total amount of the ophthalmic composition is preferably 0.001 to 3 w/v%, more preferably 0.005 to 2 w/v%, still more preferably 0.01 to 1 w/v%, and particularly preferably 0.05 to 1 w/v%.

The content proportion of the component (C) with respect to the component (A) in the ophthalmic composition according to the present embodiment is not particularly limited, and is appropriately set depending on the types of the component (A) and the component (C), the types and contents of other formulation components, applications and formulation forms of the ophthalmic composition, and the like. Regarding the content proportion of the component (C) with respect to the component (A), in order to further improve the effects of the second present invention, for example, the total content of the component (C) based on the total content of 1 part by mass of the component (A) contained in the ophthalmic composition according to the present embodiment is more preferably 0.001 parts by mass to 30 parts by mass, more preferably 0.005 to 20 parts by mass, still more preferably 0.01 to 10 parts by mass, and particularly preferably 0.01 to 2 parts by mass.

The ophthalmic composition according to the present embodiment preferably further contains a buffer (D) (also referred to as a "component (D)"). When the ophthalmic composition further contains the component (D), the effects of the second present invention are more significantly exhibited. The buffers are not particularly limited as long as they are pharmaceutically, pharmacologically (medicinally) or physiologically acceptable. Examples of buffers include inorganic buffers that are buffers derived from inorganic acids and organic buffers that are buffers derived from organic acids or organic bases.

Examples of inorganic buffers include borate buffers, phosphate buffers, and carbonate buffers. Examples of borate buffers include boric acid or salts thereof (alkali metal borates, alkaline earth metal borates, etc.). Examples of phosphate buffers include phosphoric acid or salts thereof (alkali metal phosphates, alkaline earth metal phosphates, etc.). Examples of carbonate buffers include carbonic acid or salts thereof (alkali metal carbonates, alkaline earth metal carbonates, etc.). In addition, as the borate buffers, phosphate buffers or carbonate buffers, borate, phosphate or carbonate hydrates may be used. As more specific examples, examples of borate buffers include boric acid or salts thereof (sodium borate, potassium tetraborate, potassium metaborate, ammonium borate, borax, etc.); examples of phosphate buffers include phosphoric acid or salts thereof (disodium hydrogen phosphate, sodium dihydrogen phosphate, potassium dihydrogen phosphate, trisodium phosphate, tripotassium phosphate, calcium monohydrogen phosphate, calcium dihydrogen phosphate, etc.); and examples of carbonate buffers include carbonic acid or salts thereof (sodium bicarbonate, sodium carbonate, ammonium carbonate, potassium carbonate, calcium carbonate, potassium bicarbonate, magnesium carbonate, etc.).

Examples of organic buffers include citrate buffers, acetate buffers, lactate buffers, succinate buffers, tris buffers, and AMPD buffers. Examples of citrate buffers include citric acid or salts thereof (alkali metal citrate, alkaline earth metal citrate, etc.). Examples of acetate buffers include acetic acid or salts thereof (alkali metal acetate, alkaline earth metal acetate, etc.). Examples of lactate buffers include lactic acid or salts thereof (alkali metal lactate, alkaline earth metal lactate, etc.). Examples of succinate buffers include succinic acid or salts thereof (succinic acid alkali metal salt, etc.). In addition, as the citrate buffers, acetate buffers, lactate buffers or succinate buffers, citrate, acetate, lactate or succinate hydrates may be used. As more specific examples, examples of citrate buffers include citric acid or salts thereof (sodium citrate, potassium citrate, calcium citrate, sodium dihydrogen citrate, disodium citrate, etc.); examples of acetate buffers include acetic acid or salts thereof (ammonium acetate, sodium acetate, potassium acetate, calcium acetate, etc.); examples of lactate buffers include lactic acid or salts thereof (sodium lactate, potassium lactate, calcium lactate, etc.); and examples of succinate buffers include succinic acid or salts thereof (monosodium succinate, disodium succinate, etc.). Examples of tris buffers include trometamol or salts thereof (trometamol hydrochloride, etc.). Examples of AMPD buffers include 2-amino-2-methyl-1,3-propanediol or salts thereof.

The buffers are preferably borate buffers (for example, a combination of boric acid and borax, etc.), phosphate buffers (for example, a combination of disodium hydrogen phosphate and sodium dihydrogen phosphate, etc.), and tris buffers (for example, trometamol), more preferably borate buffers, still more preferably boric acids and salts thereof, and yet more preferably a combination of boric acid and borax.

Commercially available buffers can be used. The buffers may be used alone or two or more thereof may be used in combination.

The content of the component (D) in the ophthalmic composition according to the present embodiment is not particularly limited, and is appropriately set depending on the type of the component (D), the types and contents of other formulation components, applications and formulation forms of the ophthalmic composition, and the like. Regarding the content of the component (D), in order to more significantly exhibit the effects of the second present invention, for example, the total content of the component (D) based on the total amount of the ophthalmic composition is preferably 0.01 to 10 w/v%, more preferably 0.05 to 5 w/v%, and still more preferably 0.1 to 3 w/v%.

The content proportion of the component (D) with respect to the component (A) in the ophthalmic composition according to the present embodiment is not particularly limited, and is appropriately set depending on the types of the component (A) and the component (D), the types and contents of other formulation components, applications and formulation forms of the ophthalmic composition, and the like. Regarding the content proportion of the component (D) with respect to the component (A), in order to further improve the effects of the second present invention, for example, the total content of the component (D) based on the total content of 1 part by mass of the component (A) contained in the ophthalmic composition according to the present embodiment is preferably 0.01 to 100 parts by mass, more preferably 0.05 to 50 parts by mass, and still more preferably 0.1 to 30 parts by mass.

The pH of the ophthalmic composition according to the present embodiment is not particularly limited as long as it is within a pharmaceutically, pharmacologically (medicinally) or physiologically acceptable range. The pH of the ophthalmic composition according to the present embodiment may be, for example, 4.0 to 9.5, and is preferably 4.0 to 9.0, more preferably 4.5 to 9.0, still more preferably 4.5 to 8.5, yet more preferably 5.0 to 8.5, particularly preferably 5.0 to 8.0, more particularly preferably 5.3 to 7.5, and most preferably 5.3 to 7.0.

The osmotic pressure ratio of the ophthalmic composition according to the present embodiment can be adjusted, as necessary, to be within a range in which it is acceptable in living organisms. The appropriate osmotic pressure ratio can be appropriately set depending on applications, formulation forms, usage methods of the ophthalmic composition, and the like, and it may be, for example, 0.4 to 5.0, and is preferably 0.6 to 3.0, more preferably 0.8 to 2.2, and still more preferably 0.8 to 2.0. The osmotic pressure ratio is a ratio of the osmotic pressure of the sample to 286 mOsm (the osmotic pressure of a 0.9 w/v% sodium chloride aqueous solution) based on the 17th revised Japanese Pharmacopoeia, and the osmotic pressure is measured with reference to the osmotic pressure measurement method (freezing point depression method) described in the Japanese Pharmacopoeia. Here, a standard solution for osmotic pressure ratio measurement (0.9 w/v% sodium chloride aqueous solution) is prepared by drying sodium chloride (Japanese Pharmacopoeia standard reagent) at 500 to 650°C for 40 to 50 minutes and then cooling it in a dessicator (silica gel), and accurately weighing out 0.900 g of the sample and dissolving it in purified water to make exactly 100 mL, and a commercially available standard solution for osmotic pressure ratio measurement (0.9 w/v% sodium chloride aqueous solution) can be used.

The viscosity of the ophthalmic composition according to the present embodiment is not particularly limited as long as it is within a pharmaceutically, pharmacologically (medicinally) or physiologically acceptable range. Regarding the viscosity of the ophthalmic composition according to the present embodiment, for example, the viscosity measured at 20°C using a rotational viscometer (TV-20 type viscometer, commercially available from Toki Sangyo Co., Ltd., rotor; 1°34'×R24) is preferably 1 to 10,000 mPals, more preferably 1 to 8,000 mPa/s, still more preferably 1 to 1,000 mPa/s, yet more preferably 1 to 100 mPa/s, particularly preferably 1 to 20 mPa/s, and most preferably 1.5 to 10 mPals.

The ophthalmic composition according to the present embodiment may contain an appropriate amount of one or more components selected from among various pharmacologically active components and physiologically active components in addition to the above components as long as the effects of the second present invention are not impaired. The components are not particularly limited, and examples thereof include active components in ophthalmic drugs described in Guidance required/Over-the-Counter Drug Manufacturing and Sales Approval Standards, 2017 Edition (supervised by general incorporated association, the Society of Regulatory Science). Specific examples of components used in ophthalmic drugs include the following components.
Antiallergic agents: for example, sodium cromoglicate, tranilast, potassium pemirolast, acitazanolast, amlexanox, ibudilast, etc.
Steroids: for example, fluticasone propionate, fluticasone furoate, mometasone furoate, beclomethasone propionate, flunisolide, etc.
Eye muscle regulating agent: for example, cholinesterase inhibitors having an active center similar to acetylcholine, specifically, neostigmine methyl sulfate, tropicamide, helenien, atropine sulfate, pilocarpine hydrochloride, etc.
Antiphlogistic agents: for example, methyl salicylate, glycol salicylate, allantoin, tranexamic acid, lysozyme, lysozyme chloride, indometacin, pranoprofen, ibuprofen, ibuprofenpiconol, ketoprofen, felbinac, bendazac, piroxicam, bufexamac, butyl fulfenamate, epsilon-aminocaproic acid, berberine chloride, berberine sulfate, sodium azulene sulfonate, glycyrrhizic acid or salts thereof (for example, dipotassium glycyrrhizinate, monoammonium glycyrrhizinate), etc.
Vitamins: for example, retinol acetate, retinol palmitate, tocopherol acetate, flavin adenine dinucleotide sodium, cyanocobalamin, pyridoxine hydrochloride, panthenol, calcium pantothenate, ascorbic acid, sodium ascorbate, etc.
Amino acids: for example, L-arginine, glutamic acid, glycine, alanine, lysine, γ-aminobutyric acid, γ-aminovaleric acid, trimethylglycine, taurine, aspartic acids and salts thereof, etc.
Astringents: for example, zinc oxide, etc.
Others: for example, sulfamethoxazole, sulfisoxazole, sulfisomidine and salts thereof, etc.

In the ophthalmic composition according to the present embodiment, as long as the effects of the second present invention are not impaired, depending on applications and formulation forms, various additives are appropriately selected using a general method, and one or a combination of two or more thereof may be contained in an appropriate amount. Examples of such additives include various additives described in Pharmaceutical Excipients Dictionary 2016 (edited by the Japan Pharmaceutical Excipients Association). Examples of typical components include the following additives.
Carriers: for example, aqueous solvent such as water or water-containing ethanol.
Chelating agents: for example, ethylenediaminediacetic acid (EDDA), ethylenediaminetriacetic acid, ethylenediaminetetraacetic acid (EDTA), N-(2-hydroxyethyl)ethylenediaminetriacetic acid (HEDTA), diethylenetriaminepentaacetic acid (DTPA), etc.
Bases: for example, octyldodecanol, titanium oxide, potassium bromide, plastibase, etc.
pH regulating agents: for example, hydrochloric acid, acetic acid, sodium hydroxide, potassium hydroxide, calcium hydroxide, magnesium hydroxide, triethanolamine, monoethanolamine, diisopropanolamine, etc.
Fragrances or cooling agents: for example, menthol, menthone, camphor, borneol, geraniol, cineole, citronellol, carvone, anethole, eugenol, limonene, linalol, linalyl acetate, thymol, cymene, terpineol, pinene, camphene, isoborneol, fenchone, nerol, myrcene, myrcenol, linalyl acetate, lavandulol, eucalyptus oil, bergamot oil, peppermint oil, cool mint oil, spearmint oil, mint oil, fennel oil, cinnamon oil, rose oil, camphor oil, etc. These may be any of d-form, 1-form and dl-form.
Thickeners: for example, cellulose-based polymer compounds such as methylcellulose, ethylcellulose, hydroxypropyl methylcellulose, and sodium carboxy methylcellulose; guar gum; hydroxypropyl guar gum; gum arabic; karaya gum; xanthan gum; agar; alginic acid and salts thereof (sodium salts, etc.); heparin analogs, heparin, heparin sulfate, heparan sulfate, heparinoid, and mucopolysaccharides of hyaluronic acid and salts thereof (sodium salts, etc.); starch; chitin and derivatives thereof; chitosan and derivatives thereof; carrageenan; and monosaccharides such as glucose, etc.
Stabilizers: for example, edetate, edetate salts (edetate disodium, calcium disodium edetate, trisodium edetate, and tetrasodium edetate), sodium formaldehyde sulfoxylate (rongalite), monostearate aluminum, monostearate glycerin, cyclodextrin, monoethanolamine, dibutyl hydroxy toluene, sodium bisulfite, sodium pyrosulfite, etc.
Preservatives: for example, alkyl polyaminoethylglycine quaternary ammonium salts (for example, benzalkonium chloride, benzethonium chloride, etc.), chlorhexidine gluconate, polydronium chloride, sodium benzoate, ethanol, chlorobutanol, sorbic acid, potassium sorbate, sodium dehydroacetate, methyl paraoxybenzoate, ethyl paraoxybenzoate, propyl paraoxybenzoate, butyl paraoxybenzoate, oxyquinoline sulfate, phenethyl alcohol, benzyl alcohol, biguanide compounds (specifically, polyhexanide hydrochloride (polyhexamethylene biguanide), alexidine, etc.), Glokill (product name, commercially available from Rhodia), etc. Isotonic agents: for example, potassium chloride, calcium chloride, sodium chloride, magnesium chloride, potassium acetate, sodium acetate, sodium bicarbonate, sodium carbonate, sodium thiosulfate, magnesium sulfate, glycerin, propylene glycol, sodium bisulfite, sodium sulfite, etc.
Sugar alcohols: for example, xylitol, sorbitol, mannitol, glycerin, etc. These may be any of d-form, l-form and dl-form.
Oils: for example, vegetable oils such as sesame oil, castor oil, soybean oil, and olive oil; animal oils such as squalane; and mineral oils such as liquid paraffin and petrolatum.

It is preferable that the ophthalmic composition according to the present embodiment not contain 0.01% or more of at least one selected from the group consisting of geraniol, linalyl acetate, limonene, citral and linalool and it is more preferable that the ophthalmic composition not contain them because it is possible to significantly exhibit the effects of the second present invention.

When the ophthalmic composition according to the present embodiment contains water, regarding the content of water, in order to more significantly exhibit the effects of the second present invention, for example, the content of water based on the total amount of the ophthalmic composition is preferably 80 w/v% or more and less than 100 w/v%, more preferably 85 w/v% or more and 99.5 w/v% or less, and still more preferably 90 w/v% or more and 99.2 w/v% or less.

The water used in the ophthalmic composition according to the present embodiment may be any water that is pharmaceutically, pharmacologically (medicinally) or physiologically acceptable. Examples of such water include distilled water, regular water, purified water, sterile purified water, water for injection and distilled water for injection. These definitions are based on the 17th revised Japanese Pharmacopoeia.

The ophthalmic composition according to the present embodiment can be prepared by adding and mixing desired amounts of the component (A) and the component (B), and as necessary, other components to a desired concentration. For example, it can be prepared by dissolving or dispersing these components in purified water, adjusting the pH and osmotic pressure to a predetermined value, and performing a sterilization treatment by filtration sterilization or the like.

The ophthalmic composition according to the present embodiment can take various formulation forms depending on the purpose. Examples of formulation forms include liquid preparations, gel preparations, and semi-solid preparations (ointments, etc.).

The ophthalmic composition according to the present embodiment can be used as, for example, eye drops (eye lotions or ophthalmic preparations; here the eye drops include an eye drop that can be instilled in the eyes wearing contact lenses), artificial tears, eyewashes (also called eyewash liquids or eyewash preparations; here the eyewashes include an eyewash that can wash the eyes wearing contact lenses), and contact lens compositions [a contact lens wearing liquid, a contact lens care composition (a contact lens disinfectant, a contact lens preservative, a contact lens cleaning agent, a contact lens cleaning preservative), a contact lens packaging liquid, etc.]. Here, "contact lenses" include hard contact lenses and soft contact lenses (including both ionic and non-ionic lenses and both silicone hydrogel contact lenses and non-silicone hydrogel contact lenses). In order to more significantly exhibit the effects of the second present invention, a silicone hydrogel contact lens ophthalmic composition is preferable, and an eye drop that can be instilled in the eyes wearing silicone hydrogel lenses is more preferable.

The ophthalmic composition according to the present embodiment is preferably an eye drop (including an eye drop that can be instilled in the eyes wearing contact lenses) because it can more significantly exhibit the effects of the second present invention. When the ophthalmic composition according to the present embodiment is an eye drop, the usage and dosage thereof are not particularly limited as long as they exhibit effects and have few side effects, and for example, for adults (aged 15 years or older) and children aged 7 years or older, a method of dropping 1 to 3 drops, 1 to 2 drops, or 2 to 3 drops at a time, 2 to 4 times or 5 to 6 times a day can be used.

The ophthalmic composition according to the present embodiment is provided after being stored in an arbitrary container. The container for storing the ophthalmic composition according to the present embodiment is not particularly limited, and may be made of, for example, glass or a plastic. The container is preferably made of a plastic. Examples of plastics include polyethylene terephthalate (PET), polyarylate, polyethylene naphthalate, polycarbonate, polyethylene, polypropylene, polyimide, copolymers of monomers constituting these, and mixtures of two or more of these. Polyethylene terephthalate is preferable. In addition, the container for storing the ophthalmic composition according to the present embodiment may be a transparent container that allows the inside of the container to be visually recognized or an opaque container that makes it difficult to visually recognize the inside of the container. A transparent container is preferable. Here, the "transparent container" includes both a colorless transparent container and a colored transparent container.

A nozzle may be attached to the container for storing the ophthalmic composition according to the present embodiment. The material of the nozzle is not particularly limited, and may be made of, for example, glass or a plastic. The container is preferably made of a plastic. Examples of plastics include polybutylene terephthalate, polyethylene, polypropylene, polyethylene terephthalate, polyethylene naphthalate, copolymers of monomers constituting these, and mixtures of two or more of these. In order to further improve the effects of the second present invention, the material of the nozzle is preferably polypropylene, polyethylene, polyethylene terephthalate, polybutylene terephthalate, or polyethylene naphthalate, and more preferably polyethylene.

The container for storing the ophthalmic composition according to the present embodiment may be a multi-dose type container in which a plurality of doses are stored or a unit-dose type container in which a single dose is stored.

The ophthalmic composition according to the present embodiment is preferably filled into a container having an internal volume of 4 to 30 mL, more preferably filled into a container having an internal volume of 5 to 20 mL, still more preferably filled into a container having an internal volume of 6 to 16 mL, and yet more preferably filled into a container having an internal volume of 10 to 15 mL. In addition, the ophthalmic composition may be filled into a container having an internal volume of 0.1 to 3 mL or may be filled into a container having an internal volume of 0.2 to 1 mL.

### [Examples of second present invention]

Hereinafter, the second present invention will be described in detail with reference to test examples, but the second present invention is not limited thereto. In addition, the sodium chondroitin sulfate used in the following test examples is as follows, and sodium chondroitin sulfate having a weight-average-molecular weight of 56,000 was derived from sharks.
Sodium chondroitin sulfate
weight-average-molecular weight of about 56,000: commercially available from Seikagaku Corporation; Grade N-K
weight-average-molecular weight of about 28,000: commercially available from Seikagaku Corporation; Grade ND-K
weight-average-molecular weight of about 25,000: commercially available from Maruha Nichiro Corporation; over-the-counter sodium chondroitin sulfate

### [Test Example 1: test of viscosity stability upon light emission]

Ophthalmic compositions were prepared according to a general method using the formulations shown in Table 20 and used as test solutions. In Table 20, the units of the components are w/v%. A 10 mL glass headspace vial (commercially available from GL Sciences Inc.) was filled with 10 mL of each ophthalmic composition, and radiated with light in a photostability test device (LT-120A-WCD (commercially available from Nagano Science Co., Ltd.)) using a D65 fluorescent lamp as a light source at a room temperature of 25°C and an illuminance of 4,000 lx/h until the cumulative illuminance reached 1,200,000 lx. For each ophthalmic composition (600 µL) before and after radiation, the viscosity at a shear rate (1 to 10,000 (1/s)) at 34°C was measured using a cone plate type measurement jig (CP50-1, d: 0.102 mm) with a rheometer (MCR302 (commercially available from AntonPaar)). Then, using the viscosity (mPals) at a shear rate of 1,000 (1/s), according to the following formula, the viscosity stability before and after the test was evaluated. The results are shown in Table 20. Here, the smaller the value of the viscosity change percentage, the less the change in viscosity caused by light, and the more the ophthalmic composition is kept physically the same. viscosity change percentage (%)={(viscosity of each ophthalmic composition before light emission-viscosity of each ophthalmic composition after light emission)/viscosity of each ophthalmic composition before light emission}×100

**[Table 20]**

| Component name | Reference Example 1 | Reference Example 2 | Example 1 | Example 2 | Example 3 | Example 4 | Example 5 | Example 6 |
|---|---|---|---|---|---|---|---|---|
| Sodium chondroitin sulfate (weight-average-mol ecular weight of about 28,000) | 0.5 | - | - | - | - | - | - | - |
| Sodium chondroitin sulfate (weight-average-mol ecular weight of about 56,000) | - | 0.5 | 0.5 | 0.5 | 0.5 | 0.5 | 0.5 | 0.5 |
| Tetrahydrozoline hydrochloride | - | - | 0.05 | - | - | - | - | - |
| Zinc sulfate hydrate | - | - | - | 0.25 | - | - | - | - |
| Chlorpheniramine maleate | - | - | - | - | 0.03 | - | - | - |
| Hydroxyethyl cellulose | - | - | - | - | - | 0.1 | - | - |
| Polyvinylpyrrolidone K30 | - | - | - | - | - | - | 0.1 | - |
| Polyvinylpyrrolidone K90 | - | - | - | - | - | - | 0.1 | 0.1 |
| Boric acid | 1.2 | 1.2 | 1.2 | 1.2 | 1.2 | 1.2 | 1.2 | 1.2 |
| Borax | 0.1 | 0.1 | 0.1 | 0.1 | 0.1 | 0.1 | 0.1 | 0.1 |
| Hydrochloric acid | Appropri ate amount | Appropri ate amount | Appropri ate amount | Appropri ate amount | Appropri ate amount | Appropri ate amount | Appropri ate amount | Appropri ate amount |
| Purified water | Residual amount | Residual amount | Residual amount | Residual amount | Residual amount | Residual amount | Residual amount | Residual amount |
| Total amount | 100 mL | 100 mL | 100 mL | 100 mL | 100 mL | 100 mL | 100 mL | 100 mL |
| pH | 5.5 | 5.5 | 5.5 | 5.5 | 5.5 | 5.5 | 5.5 | 5.5 |
| Viscosity change percentage (%) at a shear rate of 1,000 (1/s) | 0.4 | 26.5 | 23.7 | 1.8 | 0.3 | 3.8 | 3.5 | 3.8 |

In Reference Example 1 containing sodium chondroitin sulfate having a weight-average-molecular weight of about 28,000, the viscosity did not change due to light emission, but in Reference Example 2 containing sodium chondroitin sulfate having a weight-average-molecular weight of about 56,000, there was a problem of the viscosity changing due to light emission. However, it was confirmed that, in Examples 1 to 6 containing sodium chondroitin sulfate having a weight-average-molecular weight of about 56,000, and tetrahydrozoline hydrochloride, zinc sulfate hydrate, chlorpheniramine maleate, hydroxyethyl cellulose, polyvinylpyrrolidone K30 or polyvinylpyrrolidone K90, the viscosity change percentage was significantly lowered, and the stability of the ophthalmic composition upon light emission was improved. Here, the same tendency was obtained when the viscosity was measured at a shear rate of 100 (1/s).

### [Test Example 2: measurement of contact angle in stainless steel tube]

Ophthalmic compositions were prepared according to a general method using the formulations shown in Table 21 and used as test solutions. In Table 21, the units of the components are w/v%. Droplets (about 1 µL) of each prepared ophthalmic composition were added dropwise to a metal plate made of stainless steel (Ferrule Cap TypeCLF-B), and the contact angle (static contact angle) with respect to the metal 0.1 seconds after dropwise addition was measured using an automatic contact angle meter (solid-liquid interface analysis system Drop Master, DM-A501 (commercially available from Kyowa Interface Science Co., Ltd.)). The contact angle was measured three times for each ophthalmic composition, and the average value thereof was calculated and used as the contact angle of each ophthalmic composition. The results are shown in Table 21.

**[Table 21]**

| Component name | Reference Example 2 | Example 4 | Example 5 |
|---|---|---|---|
| Sodium chondroitin sulfate (weight-average-molecular weight of about 56,000) | 0.5 | 0.5 | 0.5 |
| Hydroxyethyl cellulose | - | 0.1 | - |
| PVPK30 | - | - | 0.1 |
| Boric acid | 1.2 | 1.2 | 1.2 |
| Borax | 0.1 | 0.1 | 0.1 |
| Hydrochloric acid | Appropriate amount | Appropriate amount | Appropriate amount |
| Purified water | Residual amount | Residual amount | Residual amount |
| Total amount | 100 mL | 100 mL | 100 mL |
| pH | 5.5 | 5.5 | 5.5 |
| Contact angle (°) | 65.0 | 71.2 | 74.1 |

The test solutions of Examples 4 and 5 had a significantly larger contact angle than the test solution of Reference Example 2. That is, it was confirmed that the test solution containing sodium chondroitin sulfate having a weight-average-molecular weight of about 56,000, and hydroxyethyl cellulose or polyvinylpyrrolidone had significantly lower affinity for the stainless steel tube than the test solution containing only sodium chondroitin sulfate having a weight-average-molecular weight of about 56,000. It was found that, since the filling tubes on the production line were made of a metal such as stainless steel, when the ophthalmic composition was filled into the container, it was possible to reduce the amount of droplets adhered to the tips of the filling tubes, and the filling amount was easily uniformized.

### [Test Example 3: preservative efficacy test]

Ophthalmic compositions were prepared according to a general method using the formulations shown in Table 22 and used as test solutions. In Table 22, the units of the components are w/v%. *Staphylococcus aureus* (ATCC6538) was inoculated on the surface of a soy bean/casein/digest slant medium and cultured at 33°C for 24 hours. The cultured bacterial cells were aseptically collected using a platinum loop and suspended in an appropriate amount of a sterile saline to prepare a bacterial suspension containing about 1×10⁷ CFU/mL of viable bacteria. Here, the number of viable bacteria in the suspension was measured by performing culturing separately. Next, 10 mL of each prepared ophthalmic composition was filled into a 15 mL conical tube made of PET (commercially available from CORNING). A *Staphylococcus aureus* bacterial solution (suspended in the saline) was inoculated into each of these ophthalmic compositions so that the number of viable bacteria (final concentration) was about 10⁵ CFU/mL, and stirred well to prepare a sample. The sample containing bacteria was stored at 23°C for 3 days under a light-shielded condition. Then, the sample containing bacteria was adjusted to an appropriate concentration for counting, 1 mL of the sample was seeded on a 3M^{™} Petrifilm ^{™} Rapid Aerobic Count Plate (RAC plate) and cultured at 33°C for 2 days, and the number of colonies observed was then counted to determine the number of viable bacteria. The number of viable bacteria immediately after inoculation was compared with the number of viable bacteria in the sample after storage for 3 days, and the amount of decrease in the number of bacteria was calculated as Log Reduction. In addition, regarding the calculated Log Reduction, it was determined whether the preservative efficacy was sufficient based on the following evaluation criteria. Here, culture of bacteria for initial bacteria count was performed at 33°C for 2 days. The results are shown in Table 22.

### Evaluation criteria

Log Reduction<0.7: D
0.7≤Log Reduction<0.8: C
0.8≤Log Reduction<0.9: B
0.9≤Log Reduction: A

**[Table 22]**

| Component name | Reference Example 1 | Example 2 | Example 6 |
|---|---|---|---|
| Sodium chondroitin sulfate (weight-average-molecular weight of about 28,000) | 0.5 | - | - |
| Sodium chondroitin sulfate (weight-average-molecular weight of about 56,000) | - | 0.5 | 0.5 |
| Zinc sulfate hydrate | - | 0.25 | - |
| Polyvinylpyrrolidone K90 | - | - | 0.1 |
| Boric acid | 1.2 | 1.2 | 1.2 |
| Borax | 0.1 | 0.1 | 0.1 |
| Hydrochloric acid | Appropriate amount | Appropriate amount | Appropriate amount |
| Purified water | Residual amount | Residual amount | Residual amount |
| Total amount | 100 mL | 100 mL | 100 mL |
| pH | 5.5 | 5.5 | 5.5 |
| Evaluation criteria | D | A | A |

Reference Example 1 had low preservative efficacy, but Examples 2 and 6 had improved preservative efficacy. That is, it was confirmed that the test solutions of Examples 2 and 6 containing sodium chondroitin sulfate having a weight-average-molecular weight of about 56,000, and zinc sulfate hydrate or polyvinylpyrrolidone had further improved preservative efficacy than the test solution of Reference Example 1 containing sodium chondroitin sulfate having a weight-average-molecular weight of about 28,000.

### [Test Example 4: measurement of viscosity]

Ophthalmic compositions were prepared according to a general method using the formulations shown in Table 23 and used as test solutions. In Table 21, the units of the components are w/v%. Regarding the viscosity of each ophthalmic composition (600 µL) after preparation, the viscosity against a shear rate at 34°C was measured using a cone plate type measurement jig (CP50-1, d: 0.102 mm) with a rheometer (MCR302 (commercially available from AntonPaar)). Regarding the viscosity (mPa/s) at a shear rate of 10,000 (1/s), according to the following formula, the viscosity reduction percentage of Example 2 with respect to Reference Example 2 (formulation containing sodium chondroitin sulfate alone) was calculated. Here, a decrease in viscosity indicates that the viscosity decreases when stress was applied, and the change in viscosity during blinking could be evaluated. Therefore, a decrease in viscosity during blinking indicates that it was easier to blink and it was less likely to cause discomfort during blinking. viscosity reduction percentage (%)=(1-viscosity of Example 2/viscosity of Refence Example 2)×100

The results are shown in Table 23.

**[Table 23]**

| Component name | Reference Example 2 | Example 2 |
|---|---|---|
| Sodium chondroitin sulfate (weight-average-molecular weight of about 56,000) | 0.5 | 0.5 |
| Zinc sulfate hydrate | - | 0.3 |
| Boric acid | 1.2 | 1.2 |
| Borax | 0.1 | 0.1 |
| Hydrochloric acid | Appropriate amount | Appropriate amount |
| Purified water | Residual amount | Residual amount |
| Total amount | 100 mL | 100 mL |
| pH | 5.5 | 5.5 |
| Viscosity reduction percentage (%) | - | 15.2 |

Example 2 containing zinc sulfate hydrate had a significantly lower viscosity than Reference Example 2. Here, the inventors confirmed that the viscosity of Reference Example 2 prepared using sodium chondroitin sulfate having a weight-average-molecular weight of about 56,000 measured with a rotational viscometer was higher than the viscosity of a test solution having the same composition as Reference Example 2 except that sodium chondroitin sulfate having a weight-average-molecular weight of about 28,000 was used in place of sodium chondroitin sulfate having a weight-average-molecular weight of about 56,000. If the viscosity of the ophthalmic composition when instilled in the eyes is high, problems such as difficulty in blinking and easily causing discomfort may occur. Therefore, it can be said that, when the viscosity at a high shear rate is low, it is easy to blink after instillation and it is less likely to cause discomfort. Therefore, it was confirmed that the ophthalmic composition containing sodium chondroitin sulfate having a weight-average-molecular weight of about 56,000 and zinc sulfate hydrate made it easier to blink after instillation and was less likely to cause discomfort than the ophthalmic composition containing only sodium chondroitin sulfate having a weight-average-molecular weight of about 56,000.

### [Test Example 5: test for reducing change in appearance (color) upon UV emission]

Ophthalmic compositions were prepared according to a general method using the formulations shown in Table 24 and used as test solutions. In Table 24, the units of the components are w/v%. A glass bottle (10 mL) was filled with 10 mL of each ophthalmic composition and radiated with light at 35°C using SUNTESTER XLS+ (1,700 W xenon air-cooled lamp light source commercially available from Toyo Seiki Co., Ltd.) at an UV illuminance of 765(W/m²) for 96 hours. Then, each ophthalmic composition was sufficiently thermally uniformized at 25°C, the color difference change (b* value) of each ophthalmic composition before and after UV emission was measured using a spectrophotometer (CM3500d: commercially available from Konica Minolta, Inc.), and according to the following Formula 1, the change in appearance of the ophthalmic composition before and after UV emission (color difference change degree; Δb* value) was calculated, and according to the following Formula 2, the color difference change reduction percentage was additionally calculated. The results are shown in Table 24. Here, the smaller the Δb* value, the less the change (coloring) in appearance (color) of the ophthalmic composition. Δb* = b* value before UV emission-b* value after UV emission color difference change reducton percentage (%)={1-(Δb* of Example 1/Δb * of Comparative Example 1)}×100

**[Table 24]**

| Component name | Comparative Example 1 | Example 1 |
|---|---|---|
| Sodium chondroitin sulfate (weight-average-molecular weight of about 28,000) | 0.5 | - |
| Sodium chondroitin sulfate (weight-average-molecular weight of about 56,000) | - | 0.5 |
| Tetrahydrozoline hydrochloride | 0.05 | 0.05 |
| Boric acid | 1.2 | 1.2 |
| Borax | 0.1 | 0.1 |
| Hydrochloric acid | Appropriate amount | Appropriate amount |
| Purified water | Residual amount | Residual amount |
| Total amount | 100 mL | 100 mL |
| pH | 5.5 | 5.5 |
| Color difference change reduction percentage (%) | - | 96.2 |

It was confirmed that Example 1 containing sodium chondroitin sulfate having a weight-average-molecular weight of about 56,000 and tetrahydrozoline hydrochloride had a smaller color difference change degree (Δb*) upon UV emission and less coloring of the formulation upon UV emission than Comparative Example 1 containing sodium chondroitin sulfate having a weight-average-molecular weight of about 28,000 and tetrahydrozoline hydrochloride.

### [Test Example 6: cell damage test]

Ophthalmic compositions were prepared according to a general method using the formulations shown in Table 25 and used as test solutions. In Table 25, the units of the components are w/v%. 500 µL of human corneal epithelial cell line HCE-T cells at a concentration of 1×10⁵ cells/mL was seeded in each well in a 24-well pate (commercially available from Corning Inc.), and cultured in a CO₂ incubator set at 37°C and a concentration of 5% CO₂. As a growth medium, one obtained by adding 5% of FCS (commercially available from DS Pharma Co., Ltd.), 0.5% of DMSO (commercially available from Wako Pure Chemical Industries, Ltd.), 10 ng/mL of a recombinant human EGF (commercially available from R&D), and 5 µg/mL of an insulin solution human (commercially available from SIGMA) to DMEM/F12 (commercially available from ThermoFisher) was used. After 2 to 4 days, when the cells became confluent, the growth medium was removed by suction from each well, 50 µL of each ophthalmic composition was added to each well, and incubated for 15 minutes under conditions of 37°C and 5% CO₂. Three or four glass beads (commercially available from As One Corporation) were put into each well and shaken for 1 minute at 450 rpm using a microplate shaker (commercially available from Heidlph Instruments GmbH & Co. KG). The supernatant and glass beads were removed, 500 µL of a medium in which a cell number measurement reagent (Cell counting kit-8, commercially available from Dojindo Molecular Technologies, Inc.) and a medium were mixed at 1:10 was added, culturing was performed in a CO₂ incubator for 2 hours, and the absorbance at 450 nm was then measured using an absorbance meter (commercially available from MOLECULAR DEVICES). The cell survival percentage was calculated according to the following formula. cell survival percentage (%)=(absorbance in each formulation/absorbance of control)×100

The results are shown in Table 25.

**[Table 25]**

| Component name | Control | Reference Example 1 | Example 4 |
|---|---|---|---|
| Sodium chondroitin sulfate (weight-average-molecular weight of about 28,000) | - | 0.5 | - |
| Sodium chondroitin sulfate (weight-average-molecular weight of about 56,000) | - | - | 0.5 |
| Hydroxyethyl cellulose | - | - | 0.1 |
| Boric acid | 1.2 | 1.2 | 1.2 |
| Borax | 0.1 | 0.1 | 0.1 |
| Hydrochloric acid | Appropriate amount | Appropriate amount | Appropriate amount |
| Purified water | Residual amount | Residual amount | Residual amount |
| Total amount | 100 mL | 100 mL | 100 mL |
| pH | 5.5 | 5.5 | 5.5 |
| Survival percentage (%) based on 100 of control | - | 96.0 | 120.0 |

It was confirmed that Example 4 containing sodium chondroitin sulfate having a weight-average-molecular weight of about 56,000 and hydroxyethyl cellulose had a significantly higher cell survival percentage than Reference Example 1 containing sodium chondroitin sulfate having a weight-average-molecular weight of about 28,000. Therefore, after the ophthalmic composition containing sodium chondroitin sulfate having a weight-average-molecular weight of about 56,000 and hydroxyethyl cellulose was instilled, if an external stimulus was applied to the eyes (for example, when rubbing the eyes with hands, blinking, inserting and removing contact lenses, friction with contact lenses, or contamination with foreign substances (pollen, air contaminants, eyelashes, eye makeup-related foreign substances, or other foreign substances)), it was expected that eye cell damage could be reduced.

### [Formulation Examples]

Eye drops were prepared according to a general method using the formulations shown in the following Tables 26 to 29. Here, the units of the component amounts in the following Tables 26 to 29 are w/v% unless specified in the tables.

**[Table 26]**

| Component name | Formul ation Examp le 1 | Formul ation Examp le 2 | Formul ation Examp le 3 | Formul ation Examp le 4 | Formul ation Examp le 5 | Formul ation Examp le 6 | Formul ation Examp le 7 | Formul ation Examp le 8 | Formul ation Examp le 9 | Formul ation Examp le 10 |
|---|---|---|---|---|---|---|---|---|---|---|
| Sodium chondroitin sulfate (weight-average -molecular weight of about 56,000) | 0.5 | 1 | 0.1 | 0.25 | 0.1 | 0.5 | 0.25 | 0.4 | 0.25 | 0.15 |
| Tetrahydrozolin e hydrochloride | 0.01 | 0.03 | 0.1 | 0.01 | 0.01 | 0.02 | - | - | - | 0.02 |
| Neostigmine methyl sulfate | - | 0.005 | - | 0.005 | 0.005 | - | - | - | - | 0.0025 |
| Epsilon-aminoc aproic acid | - | 1 | 1 | 1 | 1 | - | - | - | 1 | - |
| Allantoin | 0.1 | - | - | - | - | - | 0.1 | - | - | - |
| Dipotassium elycyrrhizinate | - | 0.1 | 0.25 | - | - | 0.25 | 0.1 | - | - | 0.25 |
| Sodium azulene sulfonate | - | - | - | - | - | - | - | 0.02 | - | - |
| Berberine chloride | - | - | - | - | - | 0.01 | - | 0.01 | - | - |
| Zinc sulfate | - | 0.05 | - | - | - | - | 0.1 | - | - | 0.1 |
| Chlorphenirami ne maleate | - | 0.03 | 0.03 | 0.03 | 0.03 | 0.02 | 0.01 | 0.03 | - | - |
| Flavin adenine dinucleotide sodium | - | - | 0.05 | - | - | - | - | 0.01 | 0.05 | - |
| Cyanocobalami n | - | 0.01 | - | - | - | - | - | - | - | - |
| Retinol palmitate | - | 50,000 units | - | 100,00 0 units | 50,000 units | 10,000 units | - | 150,00 0 units | - | - |
| Pyridoxine hydrochloride | - | 0.05 | 0.1 | - | 0.01 | 0.08 | - | - | 0.01 | - |
| d-α-tocopherol acetate | 0.03 | - | - | 0.05 | 0.03 | - | 0.025 | 0.01 | - | - |
| Potassium L-aspartate | 0.5 | 0.5 | 0.5 | - | 0.8 | - | - | 0.1 | 1 | - |
| Magnesium/pota ssium L-aspartate | - | - | - | 0.800 | - | - | 1 | - | 2 | - |
| Taurine | 0.5 | 0.5 | 1 | 0.5 | 1 | - | 0.5 | 0.1 | 1 | - |
| Sodium chloride | - | 0.008 | - | - | - | - | - | - | - | - |
| Potassium chloride | - | - | - | - | - | - | 0.1 | - | - | - |
| 1-menthol | 0.02 | 0.01 | - | 0.005 | 0.008 | 0.001 | 0.005 | - | 0.0045 | - |
| d-camphor | - | 0.001 | - | - | - | - | - | - | 0.001 | - |
| d-borneol | - | 0.001 | - | 0.001 | - | - | - | - | - | - |
| Geraniol | 0.001 | - | 0.002 | | - | - | 0.0001 | 0.003 | - | - |
| Eucalyptus oil | 0.001 | - | - | 0.001 | - | - | - | 0.003 | - | - |
| Bergamot oil | - | - | - | 0.001 | - | 0.0005 | - | - | - | - |
| Mint oil | - | - | - | - | 0.001 | 0.0005 | - | - | - | 0.0001 |
| Polyoxyethylene hydrogenated castor oil | - | - | 0.3 | 0.3 | 0.4 | - | - | - | 0.2 | - |
| Polyoxyethylene castor oil | - | - | - | - | - | - | 0.1 | - | - | - |
| Polysorbate 80 | 0.2 | - | - | 0.2 | 0.2 | - | 0.3 | 0.5 | 0.1 | 0.25 |
| Polyoxyl stearate | - | - | - | - | - | - | 0.1 | - | - | - |
| Polyoxyethylene polyoxypropyle ne glycol | 0.05 | - | - | - | - | - | - | - | - | - |
| Benzalkonium chloride | - | 0.01 | - | - | - | - | - | - | - | - |
| Chlorhexidine gluconate | - | - | - | - | - | - | - | - | 0.005 | - |
| Polyhexanide hydrochloride | 1 ppm | - | 1 ppm | - | - | - | - | - | - | - |
| Chlorobutanol | 0.1 | 0.2 | 0.1 | - | - | - | 0.2 | - | - | - |
| Zinc chloride | - | - | - | - | - | - | - | - | - | 0.001 |
| Edetate sodium | 0.01 | 0.05 | 0.01 | 0.05 | 0.05 | - | 0.01 | 0.01 | 0.05 | 0.05 |
| Boric acid | 1.2 | 0.2 | 0.3 | 0.1 | 0.5 | 0.3 | 0.1 | 1 | 0.4 | 0.1 |
| Borax | 0.3 | 0.1 | 0.05 | 0.025 | - | - | 0.01 | 0.1 | 0.1 | 0.2 |
| Dibasic sodium phosphate | | | | | | | | | | 0.15 |
| Sodium dihydrogen phosphate | - | - | - | - | - | - | - | - | - | 0.2 |
| Citric acid | - | - | - | - | - | - | - | - | 0.1 | |
| Trometamol | - | - | - | - | 0.3 | - | - | - | - | - |
| Sesame oil | - | - | - | - | - | - | 0.001 | - | - | - |
| Propylene glycol | - | - | - | - | 0.2 | - | - | - | - | - |
| Concentrated glycerin | 0.1 | - | - | - | - | - | - | - | - | - |
| Sodium hvaluronate | 0.005 | 0.002 | - | - | - | - | - | - | - | 0.01 |
| Dextran | - | - | - | 0.05 | - | - | - | - | - | - |
| L-arginine | - | - | - | - | - | - | - | - | - | 0.1 |
| Polyvinylpyrroli done | - | - | - | - | - | - | 0.1 | - | - | - |
| Hydroxyethyl cellulose | - | - | 0.1 | - | - | - | - | - | - | - |
| Hydroxypropyl methylcellulose | 0.1 | - | - | - | - | - | 0.2 | - | 0.01 | - |
| Dibutyl hydroxy toluene | - | - | - | 0.01 | 0.01 | - | - | - | - | - |
| Hydrochloric acid | Approp riate amount | Approp riate amount | Approp riate amount | Approp riate amount | Approp riate amount | Approp riate amount | Approp riate amount | Approp riate amount | Approp riate amount | Approp riate amount |
| Sodium hydroxide | Approp riate amount | Approp riate amount | Approp riate amount | Approp riate amount | Approp riate amount | Approp riate amount | Approp riate amount | Approp riate amount | Approp riate amount | Approp riate amount |
| Purified water | Residu al amount | Residu al amount | Residu al amount | Residu al amount | Residu al amount | Residu al amount | Residu al amount | Residu al amount | Residu al amount | Residu al amount |
| Total amount | 100 mL | 100 mL | 100 mL | 100 mL | 100 mL | 100 mL | 100 mL | 100 mL | 100 mL | 100 mL |
| pH | 5.8 | 6.5 | 5.7 | 6.2 | 5.8 | 7 | 5.3 | 6 | 7 | 5.5 |

**[Table 27]**

| Component name | Formulation Example 11 | Formulation Example 12 | Formulation Example 13 | Formulation Example 14 | Formulation Example 15 | Formulation Example 16 |
|---|---|---|---|---|---|---|
| Sodium chondroitin sulfate (weight-average-molecular weight of about 56,000) | 0.2 | 1 | 0.5 | 0.3 | 0.5 | 0.5 |
| Potassium L-aspartate | - | - | 1 | - | 0.5 | 0.5 |
| Magnesium/potassium L-aspartate | - | 1 | - | - | 0.5 | - |
| Taurine | - | 0.5 | - | 1 | - | 0.5 |
| Sodium chloride | - | - | 0.08 | - | - | 0.05 |
| Potassium chloride | 0.6 | 0.4 | 0.1 | 0.3 | 0.45 | 0.4 |
| 1-menthol | 0.04 | 0.002 | 0.005 | 0.015 | - | 0.005 |
| d-camphor | 0.01 | - | - | 0.003 | - | 0.003 |
| Geraniol | 0.003 | - | - | - | - | - |
| Eucalyptus oil | - | 0.002 | - | - | - | - |
| Bergamot oil | - | - | - | 0.001 | - | - |
| Mint oil | - | 0.002 | - | - | - | - |
| Polyoxyethylene hydrogenated castor oil | - | - | 0.1 | 0.1 | - | - |
| Polyoxyethylene castor oil | - | - | - | - | 0.3 | - |
| Polysorbate 80 | 0.3 | 0.2 | - | - | - | 0.2 |
| Polyoxyl stearate | - | - | - | - | 0.1 | - |
| Polyoxyethylene polyoxypropylene glycol | - | - | - | - | 0.05 | - |
| Benzalkonium chloride | - | 0.01 | 0.01 | - | - | - |
| Chlorhexidine gluconate | - | - | - | - | 0.02 | - |
| Polyhexanide hydrochloride | 1 ppm | - | - | 1 ppm | - | - |
| Chlorobutanol | 0.15 | - | - | - | - | - |
| Zinc chloride | - | - | - | - | - | 0.0001 |
| Edetate sodium | - | 0.005 | 0.05 | 0.005 | 0.01 | 0.05 |
| Boric acid | 1.5 | 1 | 0.4 | 1 | - | 1 |
| Borax | - | 0.1 | 0.1 | 0.2 | - | 0.2 |
| Dibasic sodium phosphate | - | - | - | - | 0.15 | - |
| Sodium dihydrogen phosphate | - | - | - | - | 0.1 | - |
| Citric acid | - | - | 0.1 | - | - | 0.1 |
| Sesame oil | - | - | - | - | 0.05 | - |
| Concentrated glycerin | - | - | - | - | - | 0.1 |
| Sodium hvaluronate | - | 0.02 | - | - | - | - |
| L-arginine | - | 0.5 | - | - | - | - |
| Polyvinylpyrrolidone | - | - | 0.5 | 0.1 | - | - |
| Hydroxyethyl cellulose | 0.07 | - | - | - | 0.2 | - |
| Hydroxypropyl methylcellulose | - | - | 0.1 | - | 0.2 | 0.3 |
| Hydrochloric acid | Appropriate amount | Appropriate amount | Appropriate amount | Appropriate amount | Appropriate amount | Appropriate amount |
| Sodium hydroxide | Appropriate amount | Appropriate amount | Appropriate amount | Appropriate amount | Appropriate amount | Appropriate amount |
| Purified water | Residual amount | Residual amount | Residual amount | Residual amount | Residual amount | Residual amount |
| Total amount | 100 mL | 100 mL | 100 mL | 100 mL | 100 mL | 100 mL |
| pH | 6.5 | 7.2 | 6.8 | 7 | 7.5 | 6.2 |

**[Table 28]**

| Component name | Formulati on Example 17 | Formulati on Example 18 | Formulati on Example 19 | Formulati on Example 20 | Formulati on Example 21 | Formulati on Example 22 | Formulati on Example 23 | Formulati on Example 24 |
|---|---|---|---|---|---|---|---|---|
| Sodium chondroitin sulfate (MW of about 56,000) | - | 0.25 | 0.05 | 0.4 | - | 0.25 | 0.05 | 0.4 |
| Sodium chondroitin sulfate (MW of about 45,000) | 0.5 | - | - | - | 0.5 | - | - | - |
| Sodium chondroitin sulfate (MW of about 25,000) | - | 0.25 | 0.45 | 0.1 | - | 0.25 | 0.45 | 0.1 |
| Tetrahydrozoline hydrochloride | 0.01 | 0.01 | 0.01 | 0.01 | 0.05 | 0.05 | 0.05 | 0.05 |
| Neostigmine methyl sulfate | - | - | - | - | 0.005 | 0.005 | 0.005 | 0.005 |
| Epsilon-aminocap roic acid | - | - | - | - | 1 | 1 | 1 | 1 |
| Dipotassium glycyrrhizinate | 0.25 | 0.25 | 0.25 | 0.25 | 0.1 | 0.1 | 0.1 | 0.1 |
| Chlorpheniramine maleate | 0.03 | 0.03 | 0.03 | 0.03 | - | - | - | - |
| Retinol palmitate | - | - | - | - | 50,000 units | 50,000 units | 50,000 units | 50,000 units |
| Pyridoxine hydrochloride | 0.1 | 0.1 | 0.1 | 0.1 | 0.05 | 0.05 | 0.05 | 0.05 |
| Potassium L-aspartate | - | - | - | - | 1 | 1 | 1 | 1 |
| Taurine | - | - | - | - | 1 | 1 | 1 | 1 |
| 1-menthol | 0.01 | 0.01 | 0.01 | 0.01 | 0.002 | 0.002 | 0.002 | 0.002 |
| d-camphor | 0.005 | 0.005 | 0.005 | 0.005 | 0.003 | 0.003 | 0.003 | 0.003 |
| d-borneol | 0.003 | 0.003 | 0.003 | 0.003 | - | - | - | - |
| Polyoxyethylene hydrogenated castor oil | - | - | - | - | 0.1 | 0.1 | 0.1 | 0.1 |
| Polyoxyethylene castor oil | 0.1 | 0.1 | 0.1 | 0.1 | - | - | - | - |
| Polysorbate 80 | 0.5 | 0.5 | 0.5 | 0.5 | - | - | - | - |
| Benzalkonium chloride | - | - | - | - | 0.01 | 0.01 | 0.01 | 0.01 |
| Edetate sodium | 0.01 | 0.01 | 0.01 | 0.01 | 0.05 | 0.05 | 0.05 | 0.05 |
| Boric acid | 1.5 | 1.5 | 1.5 | 1.5 | 0.4 | 0.4 | 0.4 | 0.4 |
| Borax | 0.5 | 0.5 | 0.5 | 0.5 | 0.1 | 0.1 | 0.1 | 0.1 |
| Citric acid | - | - | - | - | 0.1 | 0.1 | 0.1 | 0.1 |
| Polyvinylpyrrolid one | - | - | - | - | 0.5 | 0.5 | 0.5 | 0.5 |
| Dibutyl hydroxy toluene | - | - | - | - | 0.005 | 0.005 | 0.005 | 0.005 |
| Hydrochloric acid | Appropri ate amount | Appropri ate amount | Appropri ate amount | Appropri ate amount | Appropri ate amount | Appropri ate amount | Appropri ate amount | Appropri ate amount |
| Sodium hydroxide | Appropri ate amount | Appropri ate amount | Appropri ate amount | Appropri ate amount | Appropri ate amount | Appropri ate amount | Appropri ate amount | Appropri ate amount |
| Purified water | Residual amount | Residual amount | Residual amount | Residual amount | Residual amount | Residual amount | Residual amount | Residual amount |
| Total amount | 100 mL | 100 mL | 100 mL | 100 mL | 100 mL | 100 mL | 100 mL | 100 mL |
| pH | 7.0 | 7.0 | 7.0 | 7.0 | 7.0 | 7.0 | 7.0 | 7.0 |

**[Table 29]**

| Component name | Formulati on Example 25 | Formulati on Example 26 | Formulati on Example 27 | Formulati on Example 28 | Formulati on Example 29 | Formulati on Example 30 | Formulati on Example 31 | Formulati on Example 32 |
|---|---|---|---|---|---|---|---|---|
| Sodium chondroitin sulfate (MW of about 56,000) | 0.5 | 0.5 | 0.5 | 0.5 | 0.5 | 0.5 | 0.5 | 0.5 |
| Neostigmine methyl sulfate | - | 0.005 | - | - | - | - | - | - |
| Epsilon-aminocap roic acid | - | 1 | - | - | - | - | - | - |
| Chlorpheniramine maleate | - | 0.03 | - | - | 0.03 | 0.03 | 0.03 | 0.03 |
| Retinol palmitate | 50,000 units | 50,000 units | 50,000 units | - | - | - | - | - |
| d-α-tocopherol acetate | 0.05 | 0.045 | 0.05 | - | 0.005 | - | 0.005 | - |
| Potassium L-aspartate | - | 0.8 | - | - | 0.2 | - | 0.2 | - |
| Taurine | - | 1 | - | - | 0.1 | - | 0.1 | - |
| Sodium chloride | 0.1 | - | - | 0.1 | - | - | - | - |
| Potassium chloride | 0.2 | - | - | 0.4 | - | - | - | - |
| 1-menthol | - | 0.002 | - | - | 0.03 | 0.015 | 0.03 | 0.015 |
| d-borneol | - | 0.01 | - | - | - | - | - | - |
| Polyoxyethylene hydrogenated castor oil | - | 0.3 | - | 0.5 | - | - | 0.2 | - |
| Polysorbate 80 | - | 0.3 | - | 0.5 | 0.3 | 0.3 | - | 0.3 |
| Polyoxyethylene polyoxypropylene glycol | 0.55 | - | 0.55 | - | - | - | - | - |
| Benzalkonium chloride | - | - | - | - | 0.01 | 0.01 | - | 0.01 |
| Sodium bisulfite | 0.4 | - | 0.4 | 0.2 | - | - | - | - |
| Polyhexanide hydrochloride | - | - | - | - | - | - | 1 ppm | - |
| Sodium cromoglicate | - | - | - | - | 1 | 1 | - | - |
| Tranilast | - | - | - | - | - | - | 0.5 | 0.5 |
| Pranoprofen | - | - | - | - | 0.05 | 0.05 | 0.05 | 0.05 |
| Edetate sodium | 0.05 | 0.1 | 0.05 | 0.1 | 0.05 | 0.05 | 0.05 | 0.05 |
| Boric acid | 1 | 1 | 1 | 0.5 | 1 | 0.8 | 1 | 0.8 |
| Borax | 0.02 | 0.02 | 0.02 | 0.05 | 0.1 | 0.05 | 0.1 | 0.05 |
| Trometamol | - | 0.1 | 0.1 | - | - | - | - | - |
| Liquid paraffin | - | - | - | 0.1 | - | - | - | - |
| Propylene glycol | - | 0.2 | 0.3 | - | - | - | - | - |
| Polyvinylpyrrolid one | - | - | 0.2 | 0.2 | - | - | 1.5 | 3 |
| Hydroxypropyl methylcellulose | - | - | 0.1 | 0.1 | - | - | - | - |
| Monoethanolami ne | - | - | - | - | - | - | 0.2 | 0.1 |
| Dibutyl hydroxy toluene | 0.01 | 0.01 | 0.01 | 0.01 | 0.01 | 0.01 | 0.01 | 0.01 |
| Hydrochloric acid | Appropri ate amount | Appropri ate amount | Appropri ate amount | Appropri ate amount | Appropri ate amount | Appropri ate amount | Appropri ate amount | Appropri ate amount |
| Sodium hydroxide | Appropri ate amount | Appropri ate amount | Appropri ate amount | Appropri ate amount | Appropri ate amount | Appropri ate amount | Appropri ate amount | Appropri ate amount |
| Purified water | Residual amount | Residual amount | Residual amount | Residual amount | Residual amount | Residual amount | Residual amount | Residual amount |
| Total amount | 100 mL | 100 mL | 100 mL | 100 mL | 100 mL | 100 mL | 100 mL | 100 mL |
| pH | 7.0 | 6.0 | 7.0 | 7.0 | 7.0 | 6.0 | 8.0 | 7.5 |

This concludes the description of the second present invention, and now the third present invention will be described.

The third present invention relates to an ophthalmic composition.

### Background Art

Chondroitin sulfate or salts thereof are a type of acidic mucopolysaccharide, and are added to the ophthalmic formulation for promoting energy metabolism, promoting metabolism and cell respiration to relieve eye fatigue, replenishing lachrymal fluid components, and the like (for example, Patent Literature 3-1).

### Citation List

### Patent Literature

[Patent Literature 3-1] Japanese Unexamined Patent Publication No. 2011-148791

### [Summary of Third Present Invention]

### [Technical Problem of Third Present Invention]

An object of the third present invention is to provide a new ophthalmic composition containing chondroitin sulfate or salts thereof.

### [Solution to Problem of Third Present Invention]

The inventors found that an ophthalmic composition containing menthol may cause unpleasant stimuli or a burning sensation in the eyes, but these effects are surprisingly significantly reduced in an ophthalmic composition containing sodium chondroitin sulfate having a weight-average-molecular weight of about 56,000 and menthol.

The third present invention provides, for example, the following inventions.
[1] An ophthalmic composition containing at least one selected from the group consisting of chondroitin sulfate and salts thereof which have a weight-average-molecular weight of 40,000 to 70,000, and menthol.
[2] The ophthalmic composition according to [1], further containing at least one selected from the group consisting of boric acid and salts thereof.
[3] The ophthalmic composition according to [1] or [2], further containing chlorobutanol.
[4] The ophthalmic composition according to [1] or [2], further containing at least one selected from the group consisting of a decongestant, an eye muscle regulating agent, an anti-inflammatory agent, an antihistamine, A vitamins, B vitamins, E vitamins, amino acids and salts thereof, and a cellulose-based polymer compound.

### [Effects of third present invention]

According to the third present invention, it is possible to provide an ophthalmic composition that contains menthol and reduces unpleasant irritation.

### [Descriptions of Embodiments of Third Present Invention]

Hereinafter, embodiments for implementing a third present invention will be described in detail. However, the third present invention is not limited to the following embodiments.

In this specification, unless otherwise specified, the content unit "%" indicates "w/v%" and is synonymous with "g/100 mL."

The ophthalmic composition according to the present embodiment contains at least one selected from the group consisting of chondroitin sulfate and salts thereof which have a weight-average-molecular weight of 40,000 to 70,000 (simply referred to as a "component (A)"), and menthol (simply referred to as a "component (B)").

### [Chondroitin sulfate and salts thereof]

Chondroitin sulfate and salts thereof are not particularly limited as long as they are pharmaceutically, pharmacologically (medicinally) or physiologically acceptable.

Examples of salts of chondroitin sulfate include alkali metal salts and alkaline earth metal salts. Examples of alkali metal salts include sodium salts and potassium salts. Examples of alkaline earth metal salts include magnesium salts and calcium salts.

The chondroitin sulfate and salts thereof are preferably chondroitin sulfate and alkali metal salts of chondroitin sulfate, more preferably chondroitin sulfate and sodium chondroitin sulfate, and still more preferably sodium chondroitin sulfate.

The chondroitin sulfate and salts thereof may be natural products or synthetic products, and generally, chondroitin sulfate and salts thereof derived from animals (preferably mammals, fish, mollusks, etc.; and more preferably cows, sharks, squids, rays, etc.), which are natural products, are preferably used, chondroitin sulfate and salts thereof derived from sharks and/or rays are more preferably used, and chondroitin sulfate and salts thereof derived from sharks are still more preferably used.

Commercially available chondroitin sulfates and salts thereof can be used. The chondroitin sulfates and salts thereof may be used alone or two or more thereof may be used in combination. In addition, as the chondroitin sulfate and salts thereof, chondroitin sulfate and salts thereof which have a weight-average-molecular weight of 40,000 to 70,000 defined in this specification and chondroitin sulfate and salts thereof which have a weight-average-molecular weight outside the range defined in this specification can be used in combination. For example, sodium chondroitin sulfate having a weight-average-molecular weight of 56,000 and sodium chondroitin sulfate having a weight-average-molecular weight of 25,000 can be used in combination. When chondroitin sulfate and salts thereof which have a weight-average-molecular weight of 40,000 to 70,000 defined in this specification and chondroitin sulfate and salts thereof which have a weight-average-molecular weight outside the range defined in this specification are used in combination, chondroitin sulfate and salts thereof which have a weight-average-molecular weight of 40,000 to 70,000 may be contained as raw materials.

In this specification, the "weight-average-molecular weight" can be determined by using gel permeation chromatography in which a multi-angle light scattering detector (MALS detector) and a differential refractive index detector (RI detector) are connected online. Specifically, the following conditions are presented.

### <Preparation of standard sample>

A sample is obtained by adding 10 mL of a 0.1 M sodium nitrate aqueous solution to 5 mg of chondroitin sulfate or salts thereof, gently stirring it at room temperature, and completely dissolving it.

### <Conditions for weight-average-molecular weight measurement>

device: gel permeation chromatograph-multi-angle light scatterometer
detector: differential refractive index detector (Optilab rEX commercially available from Wyatt Technology)
multi-angle light scattering detector (DAWN HELEOS commercially available from Wyatt Technology)
Column: Shodex OHpak SB-806M HQ 2 columns (ϕ7.8 mm×30 cm, commercially available from Showa Denko K.K.)
Solvent: 0.1 M sodium nitrate aqueous solution
Flow rate: 0.7 mL/min
Column temperature: 23°C
Detector temperature: 23°C
Injection volume: 0.2 mL
Data processing: commercially available from Wyatt Technology data processing system (ASTRA)

The weight-average-molecular weight of the chondroitin sulfate and salts thereof calculated by the above method is not particularly limited as long as it is in a range of 40,000 to 70,000. Examples of lower limit values of the weight-average-molecular weight include 41,000 or more, 42,000 or more, 43,000 or more, 44,000 or more, 45,000 or more, 46,000 or more, 47,000 or more, 48,000 or more, 49,000 or more, and 50,000 or more. Examples of upper limit values of the weight-average-molecular weight include 69,000 or less, 68,000 or less, 67,000 or less, 66,000 or less, 65,000 or less, 64,000 or less, 63,000 or less, 62,000 or less, 61,000 or less, and 60,000 or less. Examples of ranges of the weight-average-molecular weight include 41,000 to 69,000, 42,000 to 68,000, 43,000 to 67,000, 44,000 to 66,000, 45,000 to 65,000, 46,000 to 64,000, 47,000 to 63,000, 48,000 to 62,000, 49,000 to 61,000, and 50,000 to 60,000.

The content of the component (A) in the ophthalmic composition according to the present embodiment is not particularly limited, and is appropriately set depending on the types and contents of other formulation components, applications and formulation forms of the ophthalmic composition, and the like. In order to more significantly exhibit the effects of the third present invention, the content of the component (A) based on the total amount of the ophthalmic composition may be, for example, 0.001 to 5 w/v%, 0.005 to 5 w/v%, 0.008 to 4 w/v%, 0.01 to 3 w/v%, 0.05 to 2 w/v%, 0.1 to 1 w/v%, or 0.3 to 1 w/v%.

### [Menthol]

Menthols are not particularly limited as long as they are pharmaceutically, pharmacologically (medicinally) or physiologically acceptable.

Menthol may be any of d-form, l-form and dl-form, and examples thereof include l-menthol, d-menthol, and dl-menthol. In addition, as the component (B), an essential oil containing menthol may be used. Examples of such essential oils include mint oil, cool mint oil, spearmint oil, and peppermint oil.

The content of the component (B) in the ophthalmic composition according to the present embodiment is not particularly limited, and is appropriately set depending on the types and contents of other formulation components, applications and formulation forms of the ophthalmic composition, and the like. In order to more significantly exhibit the effects of the third present invention, the content of menthol based on the total amount of the ophthalmic composition may be 0.00001 to 0.5 w/v%, 0.0001 to 0.1 w/v%, or 0.001 to 0.05 w/v%. Here, when an essential oil containing menthol is used as the component (B), the formulation proportion of the essential oil is set so that the menthol content in the formulated essential oil satisfies the above formulation proportion.

The content proportion of the component (B) with respect to the component (A) in the ophthalmic composition according to the present embodiment is not particularly limited, and is appropriately set depending on the type of the component (A), the types and contents of other formulation components, applications and formulation forms of the ophthalmic composition, and the like. Regarding the content proportion of the component (B) with respect to the component (A), in order to further improve the effects of the third present invention, for example, the total content of the component (B) based on the total content of 1 part by mass of the component (A) contained in the ophthalmic composition according to the present embodiment may be 0.00001 to 10 parts by mass, 0.0001 to 1 part by mass, or 0.001 to 0.1 parts by mass.

The ophthalmic composition according to the present embodiment may further contain at least one selected from the group consisting of boric acid and salts thereof (simply referred to as a "component (C)") in addition to the component (A) and the component (B). When the ophthalmic composition further contains the component (C), the effects of the third present invention are more significantly exhibited. The component (C) is not particularly limited as long as it is pharmaceutically, pharmacologically (medicinally) or physiologically acceptable.

Specific examples of boric acids and salts thereof include boric acid, sodium borate, potassium tetraborate, potassium metaborate, ammonium borate, and borax. As the boric acids and salts thereof, a combination of boric acid and borax is preferable.

The content of the component (C) in the ophthalmic composition according to the present embodiment is not particularly limited, and is appropriately set depending on the types and contents of other formulation components, applications and formulation forms of the ophthalmic composition, and the like. In order to more significantly exhibit the effects of the third present invention, the content of the component (C) based on the total amount of the ophthalmic composition may be 0.01 to 5 w/v%, 0.05 to 3 w/v%, or 0.1 to 2 w/v%.

The content proportion of the component (C) with respect to the component (A) in the ophthalmic composition according to the present embodiment is not particularly limited, and is appropriately set depending on the type of the component (A), the types and contents of other formulation components, applications and formulation forms of the ophthalmic composition, and the like. Regarding the content proportion of the component (C) with respect to the component (A), in order to further improve the effects of the third present invention, for example, the total content of the component (C) based on the total content of 1 part by mass of the component (A) contained in the ophthalmic composition according to the present embodiment may be 0.001 to 50 parts by mass, 0.01 to 10 parts by mass, or 0.1 to 5 parts by mass.

The ophthalmic composition according to the present embodiment may further contain chlorobutanol (simply referred to as a "component (D)") in addition to the component (A), the component (B) and as necessary, the component (C). When the ophthalmic composition further contains the component (D), the effects of the third present invention are more significantly exhibited. The component (D) is not particularly limited as long as it is pharmaceutically, pharmacologically (medicinally) or physiologically acceptable.

The content of the component (D) in the ophthalmic composition according to the present embodiment is not particularly limited, and is appropriately set depending on the types and contents of other formulation components, applications and formulation forms of the ophthalmic composition, and the like. In order to more significantly reduce unpleasant irritation and a burning sensation caused by menthol, the content of the component (D) based on the total amount of the ophthalmic composition may be 0.0005 to 1 w/v%, 0.001 to 0.5 w/v%, 0.005 to 0.3 w/v%, or 0.01 to 0.1 w/v%.

The content proportion of the component (D) with respect to the component (A) in the ophthalmic composition according to the present embodiment is not particularly limited, and is appropriately set depending on the type of the component (A), the types and contents of other formulation components, applications and formulation forms of the ophthalmic composition, and the like. Regarding the content proportion of the component (D) with respect to the component (A), in order to further improve the effects of the third present invention, for example, the total content of the component (D) based on the total content of 1 part by mass of the component (A) contained in the ophthalmic composition according to the present embodiment may be 0.0001 to 5 parts by mass, 0.001 to 1 part by mass, or 0.01 to 0.5 parts by mass.

An ophthalmic composition according to another embodiment of the third present invention may further contain, in addition to the component (A) and the component (B), at least one selected from the group consisting of decongestants, eye muscle regulating agents, anti-inflammatory agents, antihistamines, water-soluble vitamins and amino acids (simply referred to as a "component (E)"). As confirmed in test examples to be described below, when the ophthalmic composition further contains the component (E), it exhibits an effect of reducing the amount of liquid remaining in containers after use, an effect of making it easier to blink regardless of the viscosity of a formulation when instilled in the eyes (even if it is a high-viscosity formulation), an effect of reducing a change in viscosity due to ultraviolet rays, and/or an effect of reducing discomfort during blinking.

### [Decongestant]

Decongestants are compounds and salts thereof that have an effect of decongesting the eyes. Decongestants are not particularly limited as long as they are pharmaceutically, pharmacologically (medicinally) or physiologically acceptable.

Specific examples of decongestants include imidazoline compounds and salts thereof such as tetrahydrozoline, naphazoline, and oxymetazoline (for example, hydrochloride, nitrate); and epinephrine, ephedrine, methylephedrine, phenylephrine and salts thereof (for example, hydrochloride). The decongestants are preferably imidazoline compounds and salts thereof, more preferably tetrahydrozoline, naphazoline and salts thereof, still more preferably tetrahydrozoline and salts thereof, and particularly preferably tetrahydrozoline hydrochloride (tetrahydrozoline hydrochloride).

Commercially available decongestants can be used. The decongestants may be used alone or two or more thereof may be used in combination.

When a decongestant is used as the component (E), the content of the decongestant in the ophthalmic composition according to the present embodiment is not particularly limited, and is appropriately set depending on the type of the decongestant, the types and contents of other formulation components, applications and formulation forms of the ophthalmic composition, and the like. In order to more significantly exhibit the effects of the third present invention, the content of the decongestant based on the total amount of the ophthalmic composition may be 0.0001 to 0.5 w/v%, 0.0006 to 0.1 w/v%, or 0.0015 to 0.05 w/v%.

When tetrahydrozoline and salts thereof are used as the component (E), for example, the content of the component (E) based on the total amount of the ophthalmic composition may be 0.01 to 0.05 w/v%, or 0.025 to 0.05 w/v%.

When a decongestant is used as the component (E), the content proportion of the decongestant with respect to the component (A) in the ophthalmic composition according to the present embodiment is not particularly limited, and is appropriately set depending on the types of the component (A) and the decongestant, the types and contents of other formulation components, applications and formulation forms of the ophthalmic composition, and the like. Regarding the content proportion of the decongestant with respect to the component (A), in order to further improve the effects of the third present invention, for example, the total content of the decongestant based on the total content of 1 part by mass of the component (A) contained in the ophthalmic composition according to the present embodiment may be 0.0005 to 1 part by mass, 0.0015 to 0.5 parts by mass, or 0.025 to 0.25 parts by mass.

### [Eye muscle regulating agent]

Eye muscle regulating agents are compounds and salts thereof which have an effect of adjusting focus by tensioning or relaxing eye muscles (ciliary muscles). The eye muscle regulating agents are not particularly limited as long as they are pharmaceutically, pharmacologically (medicinally) or physiologically acceptable.

Specific examples of eye muscle regulating agents include cholinesterase inhibitors having an active center similar to acetylcholine, and more specific examples thereof include neostigmine, tropicamide, helenien, and atropine and salts thereof (for example, sulfate, methyl sulfate). The eye muscle regulating agents are preferably neostigmine and salts thereof and more preferably neostigmine methyl sulfate.

Commercially available eye muscle regulating agents can be used. The eye muscle regulating agents may be used alone or two or more thereof may be used in combination.

When an eye muscle regulating agent is used as the component (E), the content of the eye muscle regulating agent in the ophthalmic composition according to the present embodiment is not particularly limited, and is appropriately set depending on the type of the eye muscle regulating agent, the types and contents of other formulation components, applications and formulation forms of the ophthalmic composition, and the like. The content of the eye muscle regulating agent based on the total amount of the ophthalmic composition may be 0.0001 to 0.05 w/v%, 0.0005 to 0.01 w/v%, or 0.001 to 0.005 w/v%.

When an eye muscle regulating agent is used as the component (E), the content proportion of the eye muscle regulating agent with respect to the component (A) in the ophthalmic composition according to the present embodiment is not particularly limited, and is appropriately set depending on the types of the component (A) and the eye muscle regulating agent, the types and contents of other formulation components, applications and formulation forms of the ophthalmic composition, and the like. Regarding the content proportion of the eye muscle regulating agent with respect to the component (A), in order to further improve the effects of the third present invention, for example, the total content of the eye muscle regulating agent based on the total content of 1 part by mass of the component (A) contained in the ophthalmic composition according to the present embodiment may be 0.0001 to 0.5 parts by mass, 0.0005 to 0.1 parts by mass, or 0.001 to 0.05 parts by mass.

### [Anti-inflammatory agent]

Anti-inflammatory agents are compounds and salts thereof that have anti-inflammatory effects or antiphlogistic effects. The anti-inflammatory agents are not particularly limited as long as they are pharmaceutically, pharmacologically (medicinally) or physiologically acceptable.

Specific examples of anti-inflammatory agents include epsilon-aminocaproic acid, allantoin, berberine, azulenes (azulene, azulene sulfonic acid, chamazulene, guaiazulene, etc.), glycyrrhizic acid, zinc salts, lysozyme, celecoxib, rofecoxib, indometacin, diclofenac, bromfenac, piroxicam, meloxicam, methyl salicylate, ibuprofen, ibuprofenpiconol, bufexamac, butyl fulfenamate, bendazac, ketoprofen, felbinac, pranoprofen, and salts thereof. The anti-inflammatory agents are preferably allantoin, glycyrrhizic acid and salts thereof, and zinc salts. The glycyrrhizic acid and salts thereof are preferably alkali metal salts or ammonium salts of glycyrrhizic acid, more preferably dipotassium glycyrrhizinate and monoammonium glycyrrhizinate, and still more preferably dipotassium glycyrrhizinate. The zinc salts are preferably zinc sulfate or zinc lactate, and more preferably zinc sulfate. In addition, the zinc salts may be a hydrate (for example, zinc sulfate heptahydrate).

Commercially available anti-inflammatory agents can be used. The anti-inflammatory agents may be used alone or two or more thereof may be used in combination.

When an anti-inflammatory agent is used as the component (E), the content of the anti-inflammatory agent in the ophthalmic composition according to the present embodiment is not particularly limited, and is appropriately set depending on the type of the anti-inflammatory agent, the types and contents of other formulation components, applications and formulation forms of the ophthalmic composition, and the like. In order to more significantly exhibit the effects of the third present invention, the content of the anti-inflammatory agent based on the total amount of the ophthalmic composition may be 0.001 to 1 w/v%, 0.005 to 0.6 w/v%, or 0.05 to 0.3 w/v%.

When an anti-inflammatory agent is used as the component (E), the content proportion of the anti-inflammatory agent with respect to the component (A) in the ophthalmic composition according to the present embodiment is not particularly limited, and is appropriately set depending on the types of the component (A) and the anti-inflammatory agent, the types and contents of other formulation components, applications and formulation forms of the ophthalmic composition, and the like. Regarding the content proportion of the anti-inflammatory agent with respect to the component (A), in order to further improve the effects of the third present invention, for example, the total content of the anti-inflammatory agent based on the total content of 1 part by mass of the component (A) contained in the ophthalmic composition according to the present embodiment may be 0.001 to 10 parts by mass, 0.005 to 6 parts by mass, or 0.05 to 3 parts by mass.

### [Antihistamines]

Antihistamines are compounds and salts thereof which have an antihistamine effect. The antihistamines are not particularly limited as long as they are pharmaceutically, pharmacologically (medicinally) or physiologically acceptable.

Specific examples of antihistamines include chlorpheniramine, iproheptine, diphenhydramine, ketotifen, olopatadine, levocabastine, and salts thereof. The antihistamines are preferably chlorpheniramine and salts thereof, and more preferably chlorpheniramine maleate.

Commercially available antihistamines can be used. The antihistamines may be used alone or two or more thereof may be used in combination.

When antihistamines are used as the component (E), the content of the antihistamines in the ophthalmic composition according to the present embodiment is not particularly limited, and is appropriately set depending on the type of the antihistamines, the types and contents of other formulation components, applications and formulation forms of the ophthalmic composition, and the like. In order to more significantly exhibit the effects of the third present invention, the content of the antihistamines based on the total amount of the ophthalmic composition may be 0.0001 to 0.05 w/v%, 0.0006 to 0.05 w/v%, 0.006 to 0.04 w/v%, or 0.015 to 0.03 w/v%.

When antihistamines are used as the component (E), the content proportion of the antihistamines with respect to the component (A) in the ophthalmic composition according to the present embodiment is not particularly limited, and is appropriately set depending on the types of the component (A) and the antihistamines, the types and contents of other formulation components, applications and formulation forms of the ophthalmic composition, and the like. Regarding the content proportion of the antihistamines with respect to the component (A), in order to further improve the effects of the third present invention, for example, the total content of the antihistamines based on the total content of 1 part by mass of the component (A) contained in the ophthalmic composition according to the present embodiment may be 0.0001 to 0.5 parts by mass, 0.0006 to 0.5 parts by mass, 0.006 to 0.4 parts by mass, or 0.015 to 0.3 parts by mass.

### [A vitamins]

A vitamins are not particularly limited as long as they are pharmaceutically, pharmacologically (medicinally) or physiologically acceptable. Specific examples of A vitamins include retinol, retinal, retinoic acid, derivatives thereof, and salts thereof.

Examples of derivatives of A vitamins include esters with monovalent carboxylic acids such as retinol palmitate, retinol acetate, retinol butyrate, retinol propionate, retinol octylate, retinol laurate, retinol oleate and retinol linoleate.

Examples of salts of A vitamins include organic acid salts [for example, monocarboxylates (acetate, trifluoroacetate, butyrate, palmitate, stearate, etc.), polycarboxylates (fumarate, maleate, succinate, malonate, etc.), oxycarboxylates (lactate, tartrate, citrate, etc.), organic sulfonates (methane sulfonate, toluene sulfonate, tosylate, etc.), etc.], inorganic acid salts (for example, hydrochloride, sulfate, nitrate, hydrobromide, phosphate, etc.), salts with organic bases (for example, salts with organic amines such as methylamine, triethylamine, triethanolamine, morpholine, piperazine, pyrrolidine, tripyridine, and picoline, etc.), salts with inorganic bases [for example, ammonium salts; salts with metals such as alkali metals (sodium, potassium, etc.), alkaline earth metals (calcium, magnesium, etc.), and aluminum, etc.], and the like.

The A vitamins are preferably derivatives of retinol, more preferably esters of retinol and monovalent carboxylic acids, still more preferably retinol palmitate and retinol acetate, and yet more preferably retinol palmitate.

As the A vitamins, synthetic products may be used or extracts obtained from natural products (for example, a vitamin A oil, etc.) may be used. The vitamin A oil is a fatty oil obtained from animal tissues containing retinol, or its concentrate, or one obtained by appropriately adding a vegetable oil thereto. Commercially available A vitamins can be used. The A vitamins may be used alone or two or more thereof may be used in combination.

When A vitamins are used as the component (E), the content of the A vitamins in the ophthalmic composition according to the present embodiment based on the total amount of the ophthalmic composition may be 1,000 to 100,000 IU/100 mL, 5,000 to 75,000 IU/100 mL, or 10,000 to 50,000 IU/100 mL.

"IU" is an international unit determined by the method described in the 17th revised Japanese Pharmacopoeia, Vitamin A Assay Method, etc. For example, the pharmaceutical segments of the 17th revised Japanese Pharmacopoeia describe that retinol acetate contains at least 2,500,000 units of vitamin A per 1 g, and retinol palmitate contains at least 1,500,000 units of vitamin A per 1 g.

When A vitamins are used as the component (E), the content proportion of the A vitamins with respect to the component (A) in the ophthalmic composition according to the present embodiment is not particularly limited, and is appropriately set depending on the types of the component (A) and the A vitamins, the types and contents of other formulation components, applications and formulation forms of the ophthalmic composition, and the like. Regarding the content proportion of the A vitamins with respect to the component (A), in order to further improve the effects of the third present invention, for example, the total content of the A vitamins based on the total content of 1 part by mass of the component (A) contained in the ophthalmic composition according to the present embodiment may be 100 to 1,000,000 IU/g, 500 to 750,000 IU/g, or 10,000 to 500,000 IU/g.

### [B vitamins]

B vitamins are not particularly limited as long as they are pharmaceutically, pharmacologically (medicinally) or physiologically acceptable.

Examples of specific examples of B vitamins include flavin adenine dinucleotide and salts thereof (flavin adenine dinucleotide sodium, etc.), cobalamins (cyanocobalamin, methylcobalamin, etc.), pantothenic acid and salts thereof (for example, sodium pantothenate, potassium pantothenate, calcium pantothenate, magnesium pantothenate, etc.), panthenol, pyridoxine or salts thereof (pyridoxine hydrochloride, etc.), and pyridoxal and salts thereof (pyridoxal phosphate, etc.). The B vitamins are more preferably panthenol, pyridoxine or salts thereof.

Commercially available B vitamins can be used. The B vitamins may be used alone or two or more thereof may be used in combination.

When B vitamins are used as the component (E), the content of the B vitamins in the ophthalmic composition according to the present embodiment is not particularly limited, and is appropriately set depending on the type of the B vitamins, the types and contents of other formulation components, applications and formulation forms of the ophthalmic composition, and the like. In order to more significantly exhibit the effects of the third present invention, the content of the B vitamins based on the total amount of the ophthalmic composition may be 0.0005 to 0.5 w/v%, 0.001 to 0.25 w/v%, or 0.01 to 0.1 w/v%.

When B vitamins are used as the component (E), the content proportion of water-soluble vitamins with respect to the component (A) in the ophthalmic composition according to the present embodiment is not particularly limited, and is appropriately set depending on the types of the component (A) and the B vitamins, the types and contents of other formulation components, applications and formulation forms of the ophthalmic composition, and the like. Regarding the content proportion of the B vitamins with respect to the component (A), in order to further improve the effects of the third present invention, for example, the total content of the B vitamins based on the total content of 1 part by mass of the component (A) contained in the ophthalmic composition according to the present embodiment may be 0.0005 to 5 parts by mass, 0.001 to 2.5 parts by mass, or 0.01 to 1 part by mass.

### [E vitamins]

E vitamins are not particularly limited as long as they are pharmaceutically, pharmacologically (medicinally) or physiologically acceptable. Specific examples of E vitamins include tocopherol, tocotrienol, derivatives thereof, and salts thereof. Tocopherol and tocotrienol may be of α-, β-, γ-, or δ-, or may be any of d-form and dl-form.

Examples of derivatives of E vitamins include esters with organic acids such as tocopherol acetate, tocopherol succinate, tocopherol nicotinate, and tocopherol linolenate.

Examples of salts of E vitamins include organic acid salts (lactate, acetate, butyrate, trifluoroacetate, fumarate, maleate, tartrate, citrate, succinate, malonate, methane sulfonate, toluene sulfonate, tosylate, palmitate, stearate, etc.), inorganic acid salts (for example, hydrochloride, sulfate, nitrate, hydrobromide, phosphate, etc.), salts with organic bases (for example, salts with organic amines such as methylamine, triethylamine, triethanolamine, morpholine, piperazine, pyrrolidine, amino acid, tripyridine, and picoline, etc.), and salts with inorganic bases (for example, ammonium salts, salts with metals such as alkali metals such as sodium and potassium, alkaline earth metals such as calcium, and magnesium, aluminum, etc.).

The E vitamins are preferably d-α-tocopherol, dl-α-tocopherol, β-tocopherol, γ-tocopherol, δ-tocopherol, vitamin E acetate (for example, tocopherol acetate), vitamin E nicotinate, vitamin E succinate, and vitamin E linolenate, and more preferably tocopherol acetate (for example, d-α-tocopherol acetate, dl-α-tocopherol acetate, etc.).

The E vitamins may be natural products or synthetic products. Commercially available E vitamins can be used. The E vitamins may be used alone or two or more thereof may be used in combination.

When E vitamins are used as the component (E), the content of the E vitamins in the ophthalmic composition according to the present embodiment is not particularly limited, and is appropriately set depending on the type of the E vitamins, the types and contents of other formulation components, applications and formulation forms of the ophthalmic composition, and the like. The content of the E vitamins based on the total amount of the ophthalmic composition may be 0.0001 to 0.5 w/v%, 0.0005 to 0.1 w/v%, or 0.005 to 0.05 w/v%.

When E vitamins are used as the component (E), the content proportion of the E vitamins with respect to the component (A) in the ophthalmic composition according to the present embodiment is not particularly limited, and is appropriately set depending on the types of the component (A) and the E vitamins, the types and contents of other formulation components, applications and formulation forms of the ophthalmic composition, and the like. Regarding the content proportion of the E vitamins with respect to the component (A), in order to further improve the effects of the third present invention, for example, the total content of the E vitamins based on the total content of 1 part by mass of the component (A) contained in the ophthalmic composition according to the present embodiment may be 0.0001 to 5 parts by mass, 0.0005 to 1 part by mass, or 0.005 to 0.5 parts by mass.

### [Amino acids and salts thereof]

Amino acids and salts thereof are not particularly limited as long as they are pharmaceutically, pharmacologically (medicinally) or physiologically acceptable.

Examples of amino acids and salts thereof include L-aspartic acids or salts thereof (for example, potassium L-aspartate, sodium L-aspartate, magnesium L-aspartate, calcium L-aspartate, magnesium/potassium L-aspartate (mixture of equal amounts of magnesium L-aspartate and potassium L-aspartate)), aminoethylsulfonic acid or salts thereof, L-arginine, glutamic acid, glycine, alanine, lysine, γ-aminobutyric acid, y-aminovaleric acid, and trimethylglycine and salts thereof. Commercially available amino acids and salts thereof can be used. Amino acids and salts thereof may be any of L-form, D-form, and DL-form, and examples thereof include potassium L-aspartate, magnesium L-aspartate and mixtures of equal amounts of magnesium/potassium L-aspartate. Among amino acids and salts thereof, in order to further improve the effects of the third present invention, aspartic acid, aminoethylsulfonic acid and salts thereof are preferable, and aminoethylsulfonic acid is particularly preferable.

When amino acids and salts thereof are used as the component (E), in order to more significantly exhibit the effects of the third present invention, for example, the content of the amino acids and salts thereof in the ophthalmic composition according to the present embodiment based on the total amount of the ophthalmic composition may be 0.001 to 5 w/v%, 0.01 to 3 w/v%, or 0.1 to 2 w/v%.

When amino acids and salts thereof are used as the component (E), the content proportion of the amino acids and salts thereof with respect to the component (A) in the ophthalmic composition according to the present embodiment is not particularly limited, and is appropriately set depending on the types of the component (A) and the amino acids and salts thereof, the types and contents of other formulation components, applications and formulation forms of the ophthalmic composition, and the like. Regarding the content proportion of the amino acids and salts thereof with respect to the component (A), in order to further improve the effects of the third present invention, for example, the total content of the amino acids and salts thereof based on the total content of 1 part by mass of the component (A) contained in the ophthalmic composition according to the present embodiment may be 0.001 to 50 parts by mass, 0.01 to 30 parts by mass, or 0.1 to 20 parts by mass.

### [Cellulose-based polymer compound]

Cellulose-based polymer compounds are not particularly limited as long as they are pharmaceutically, pharmacologically (medicinally) or physiologically acceptable.

Examples of cellulose-based polymer compounds include methylcellulose, ethylcellulose, hydroxyethyl cellulose, hydroxymethylcellulose, hydroxypropyl cellulose, hydroxypropyl methylcellulose (hypromellose), carboxymethylcellulose, carboxyethylcellulose, and salts thereof. The cellulose-based polymer compounds are preferably hydroxyethyl cellulose, hydroxypropyl methylcellulose, and salts thereof, and more preferably hydroxypropyl methylcellulose and salts thereof. Examples of such examples include salts with organic bases (amine salts, basic ammonium salts such as arginine, etc.), salts with inorganic bases (alkali metal salts such as ammonium salts, sodium salts, and potassium salts, alkaline earth metal salts such as calcium salts and magnesium salts, aluminum salts, etc.) and the like, and among these, sodium salts, potassium salts, and calcium salts are more preferable, and sodium salts are particularly preferable.

Commercially available cellulose-based polymer compounds can be used. The cellulose-based polymer compounds may be used alone or two or more thereof may be used in combination.

When a cellulose-based polymer compound is used as the component (E), the content of the cellulose-based polymer compound in the ophthalmic composition according to the present embodiment is not particularly limited, and is appropriately set depending on the type of the cellulose-based polymer compound, the types and contents of other formulation components, applications and formulation forms of the ophthalmic composition, and the like. In order to more significantly exhibit the effects of the third present invention, the content of the cellulose-based polymer compound based on the total amount of the ophthalmic composition may be 0.001 to 3 w/v%, 0.005 to 1 w/v%, or 0.01 to 0.6 w/v%.

When a cellulose-based polymer compound is used as the component (E), the content proportion of the cellulose-based polymer compound with respect to the component (A) in the ophthalmic composition according to the present embodiment is not particularly limited, and is appropriately set depending on the types of the component (A) and the cellulose-based polymer compound, the types and contents of other formulation components, applications and formulation forms of the ophthalmic composition, and the like. Regarding the content proportion of the cellulose-based polymer compound with respect to the component (A), in order to further improve the effects of the third present invention, for example, the total content of the cellulose-based polymer compound based on the total content of 1 part by mass of the component (A) contained in the ophthalmic composition according to the present embodiment may be 0.001 to 30 parts by mass, 0.005 to 10 parts by mass, or 0.01 to 6 parts by mass.

An ophthalmic composition according to another embodiment of the third present invention may further contain, in addition to the component (A), the component (B), and the component (E), a component (C) (at least one selected from the group consisting of boric acid and salts thereof). When the ophthalmic composition further contains the component (C), an effect of reducing the amount of liquid remaining in containers after use, an effect of reducing a change in viscosity due to ultraviolet rays, an effect of making it easier to blink regardless of the viscosity of a formulation when instilled in the eyes (even if it is a high-viscosity formulation) and/or an effect of reducing discomfort during blinking are more significantly exhibited. Here, the type, content and the like of the component (C) in the present embodiment are as described above.

The ophthalmic composition according to the present embodiment may further contain a surfactant. When the ophthalmic composition further contains the surfactant, the effects of the third present invention are more significantly exhibited. The surfactants are not particularly limited as long as they are pharmaceutically, pharmacologically (medicinally) or physiologically acceptable, and may be any of nonionic surfactants, amphoteric surfactants, anionic surfactants, and cationic surfactants.

Examples of nonionic surfactants include POE sorbitan fatty acid esters such as monolaurate POE(20) sorbitan (polysorbate 20), monopalmitate POE(20) sorbitan (polysorbate 40), monostearate POE(20) sorbitan (polysorbate 60), tristearate POE(20) sorbitan (polysorbate 65), and monooleate POE(20) sorbitan (polysorbate 80); POE hydrogenated castor oils such as POE(40) hydrogenated castor oil (polyoxyethylene hydrogenated castor oil 40), and POE(60) hydrogenated castor oil (polyoxyethylene hydrogenated castor oil 60); POE castor oils such as POE(3) hydrogenated castor oil (polyoxyethylene castor oil 3), and POE(10) castor oil (polyoxyethylene castor oil 10); POE alkyl ethers such as POE(9) lauryl ether; POE-POP alkyl ethers such as POE(20)POP(4) cetyl ether; polyoxyethylene/polyoxypropylene block copolymers such as POE(196)POP(67) glycol (poloxamer 407, Pluronic F127), and POE(200)POP(70) glycol; and polyethylene glycol fatty acid esters such as monostearate polyethylene glycol (for example, monostearate polyethylene glycol (25E.O.), monostearate polyethylene glycol (40E.O.) (polyoxyl stearate 40)). Here, in the compounds exemplified above, POE indicates polyoxyethylene, POP indicates polyoxypropylene, and the number in parentheses indicates the number of moles added.

Examples of amphoteric surfactants include alkyldiaminoethylglycine and salts thereof (for example, hydrochloride, etc.).

Examples of anionic surfactants include alkylbenzene sulfonate, alkyl sulfate, polyoxyethylene alkyl sulfate, aliphatic α-sulfomethyl ester, and α-olefin sulfonic acid.

Examples of cationic surfactants include cetylpyridinium chloride, benzalkonium chloride, and benzethonium chloride.

Among these surfactants, nonionic surfactants are preferable, POE sorbitan fatty acid esters, POE hydrogenated castor oils, POE castor oils, POE/POP block copolymers, and polyethylene glycol fatty acid esters are more preferable, and POE sorbitan fatty acid esters, POE(40) hydrogenated castor oils, POE(60) hydrogenated castor oils, POE(3) castor oils, POE(10) castor oils, POE/POP block copolymers, and polyethylene glycol fatty acid esters are still more preferable. Commercially available surfactants can be used. The surfactants may be used alone or two or more thereof may be used in combination.

The content of the surfactant in the ophthalmic composition according to the present embodiment is not particularly limited, and is appropriately set depending on the type of the surfactant, applications and formulation forms of the ophthalmic composition, and the like. In order to more significantly exhibit the effects of the third present invention, for example, the content of the surfactant based on the total amount of the ophthalmic composition may be 0.001 to 3 w/v%, 0.005 to 1 w/v%, or 0.01 to 0.5 w/v%.

The content proportion of the surfactant with respect to the component (A) in the ophthalmic composition according to the present embodiment is not particularly limited, and is appropriately set depending on the types of the component (A) and the surfactant, the types and contents of other formulation components, applications and formulation forms of the ophthalmic composition, and the like. Regarding the content proportion of the surfactant with respect to the component (A), in order to further improve the effects of the third present invention, for example, the total content of the surfactant based on the total content of 1 part by mass of the component (A) contained in the ophthalmic composition according to the present embodiment may be 0.001 to 30 parts by mass, 0.005 to 10 parts by mass, or 0.01 to 5 parts by mass.

The pH of the ophthalmic composition according to the present embodiment is not particularly limited as long as it is within a pharmaceutically, pharmacologically (medicinally) or physiologically acceptable range. The pH of the ophthalmic composition according to the present embodiment may be, for example, 4.0 to 9.5, and is preferably 4.0 to 9.0, more preferably 4.5 to 9.0, still more preferably 4.5 to 8.5, yet more preferably 5.0 to 8.5, particularly preferably 5.0 to 8.0, more particularly preferably 5.3 to 7.5, and most preferably 5.5 to 7.0.

The osmotic pressure ratio of the ophthalmic composition according to the present embodiment can be adjusted, as necessary, to be within a range in which it is acceptable in living organisms. The appropriate osmotic pressure ratio can be appropriately set depending on applications, formulation forms, usage methods of the ophthalmic composition, and the like, and it may be, for example, 0.4 to 5.0, and is preferably 0.6 to 3.0, more preferably 0.8 to 2.2, and still more preferably 0.8 to 2.0. The osmotic pressure ratio is a ratio of the osmotic pressure of the sample to 286 mOsm (the osmotic pressure of a 0.9 w/v% sodium chloride aqueous solution) based on the 17th revised Japanese Pharmacopoeia, and the osmotic pressure is measured with reference to the osmotic pressure measurement method (freezing point depression method) described in the Japanese Pharmacopoeia. Here, a standard solution for osmotic pressure ratio measurement (0.9 w/v% sodium chloride aqueous solution) is prepared by drying sodium chloride (Japanese Pharmacopoeia standard reagent) at 500 to 650°C for 40 to 50 minutes and then cooling it in a dessicator (silica gel), and accurately weighing out 0.900 g of the sample and dissolving it in purified water to make exactly 100 mL, and a commercially available standard solution for osmotic pressure ratio measurement (0.9 w/v% sodium chloride aqueous solution) can be used.

The viscosity of the ophthalmic composition according to the present embodiment is not particularly limited as long as it is within a pharmaceutically, pharmacologically (medicinally) or physiologically acceptable range. Regarding the viscosity of the ophthalmic composition according to the present embodiment, for example, the viscosity measured at 20°C using a rotational viscometer (TV-20 type viscometer, commercially available from Toki Sangyo Co., Ltd., rotor; 1°34'×R24) is preferably 1 to 10,000 mPals, more preferably 1 to 8,000 mPa/s, still more preferably 1 to 1,000 mPa/s, yet more preferably 1 to 100 mPa/s, particularly preferably 1 to 20 mPa/s, and most preferably 1.5 to 10 mPals.

The ophthalmic composition according to the present embodiment may contain an appropriate amount of one or more components selected from among various pharmacologically active components and physiologically active components in addition to the above components as long as the effects of the third present invention are not impaired. The components are not particularly limited, and examples thereof include active components in ophthalmic drugs described in Guidance required/Over-the-Counter Drug Manufacturing and Sales Approval Standards, 2017 Edition (supervised by general incorporated association, the Society of Regulatory Science). Specific examples of components used in ophthalmic drugs include the following components.
Antiallergic agents: for example, sodium cromoglicate, tranilast, potassium pemirolast, acitazanolast, amlexanox, ibudilast, etc.
Steroids: for example, fluticasone propionate, fluticasone furoate, mometasone furoate, beclomethasone propionate, flunisolide, etc.
Vitamins: for example, ascorbic acid, sodium ascorbate, etc.
Astringents: for example, zinc oxide, etc.
Others: for example, sulfamethoxazole, sulfisoxazole, sulfisomidine and salts thereof, etc.

In the ophthalmic composition according to the present embodiment, as long as the effects of the third present invention are not impaired, depending on applications and formulation forms, various additives are appropriately selected using a general method, and one or a combination of two or more thereof may be contained in an appropriate amount. Examples of such additives include various additives described in Pharmaceutical Excipients Dictionary 2016 (edited by the Japan Pharmaceutical Excipients Association). Examples of typical components include the following additives.
Carriers: for example, aqueous solvent such as water or water-containing ethanol.
Chelating agents: for example, ethylenediaminediacetic acid (EDDA), ethylenediaminetriacetic acid, ethylenediaminetetraacetic acid (EDTA), N-(2-hydroxyethyl)ethylenediaminetriacetic acid (HEDTA), diethylenetriaminepentaacetic acid (DTPA), etc.
Bases: for example, octyldodecanol, titanium oxide, potassium bromide, plastibase, etc.
pH regulating agents: for example, hydrochloric acid, acetic acid, sodium hydroxide, potassium hydroxide, calcium hydroxide, magnesium hydroxide, triethanolamine, monoethanolamine, diisopropanolamine, etc.
Fragrances or cooling agents other than component (B): for example, menthone, camphor, borneol, geraniol, cineole, citronellol, carvone, anethole, eugenol, limonene, linalol, linalyl acetate, thymol, cymene, terpineol, pinene, camphene, isoborneol, fenchone, nerol, myrcene, myrcenol, linalyl acetate, lavandulol, eucalyptus oil, bergamot oil, fennel oil, cinnamon oil, rose oil, camphor oil, etc. These may be any of d-form, l-form and dl-form.
Thickeners other than cellulose-based polymer compounds: for example, guar gum; hydroxypropyl guar gum; gum arabic; karaya gum; xanthan gum; agar; alginic acid and salts thereof (sodium salts, etc.); mucopolysaccharides (other than the component (A)) of heparin analogs, heparin, heparin sulfate, heparan sulfate, heparinoid, hyaluronic acid and salts thereof (sodium salts, etc.); starch; chitin and derivatives thereof; chitosan and derivatives thereof; carrageenan; and monosaccharides such as glucose.
Buffers other than component (C): for example, phosphate buffer, carbonate buffer, acetate buffer, lactate buffer, succinate buffer, citrate buffer, tris buffer, AMPD buffer, etc.
Stabilizers: for example, edetate, edetate salts (edetate disodium, calcium disodium edetate, trisodium edetate, and tetrasodium edetate), sodium formaldehyde sulfoxylate (rongalite), monostearate aluminum, monostearate glycerin, cyclodextrin, monoethanolamine, dibutyl hydroxy toluene, sodium bisulfite, sodium pyrosulfite, etc.
Preservatives: for example, alkyl polyaminoethylglycine quaternary ammonium salts (for example, benzalkonium chloride, benzethonium chloride, etc.), chlorhexidine gluconate, polydronium chloride, sodium benzoate, ethanol, chlorobutanol, sorbic acid, potassium sorbate, sodium dehydroacetate, methyl paraoxybenzoate, ethyl paraoxybenzoate, propyl paraoxybenzoate, butyl paraoxybenzoate, oxyquinoline sulfate, phenethyl alcohol, benzyl alcohol, biguanide compounds (specifically, polyhexanide hydrochloride (polyhexamethylene biguanide), alexidine, etc.), Glokill (product name, commercially available from Rhodia), etc. Isotonic agents: for example, potassium chloride, calcium chloride, sodium chloride, magnesium chloride, potassium acetate, sodium acetate, sodium bicarbonate, sodium carbonate, sodium thiosulfate, magnesium sulfate, glycerin, propylene glycol, sodium bisulfite, sodium sulfite, etc.
Sugar alcohols: for example, xylitol, sorbitol, mannitol, glycerin, etc. These may be any of d-form, l-form and dl-form.
Oils: for example, vegetable oils such as sesame oil, castor oil, soybean oil, and olive oil; animal oils such as squalane; and mineral oils such as liquid paraffin and petrolatum.

When the ophthalmic composition according to the present embodiment contains water, regarding the content of water, in order to more significantly exhibit the effects of the third present invention, for example, the content of water based on the total amount of the ophthalmic composition is preferably 80 w/v% or more and less than 100 w/v%, more preferably 85 w/v% or more and 99.5 w/v% or less, and still more preferably 90 w/v% or more and 99.2 w/v% or less.

The water used in the ophthalmic composition according to the present embodiment may be any water that is pharmaceutically, pharmacologically (medicinally) or physiologically acceptable. Examples of such water include distilled water, regular water, purified water, sterile purified water, water for injection and distilled water for injection. These definitions are based on the 17th revised Japanese Pharmacopoeia.

The ophthalmic composition according to the present embodiment can be prepared by adding and mixing desired amounts of the component (A) and the component (B), and as necessary, other components to a desired concentration. For example, it can be prepared by dissolving or dispersing these components in purified water, adjusting the pH and osmotic pressure to a predetermined value, and performing a sterilization treatment by filtration sterilization or the like.

The ophthalmic composition according to the present embodiment can take various formulation forms depending on the purpose. Examples of formulation forms include liquid preparations, gel preparations, and semi-solid preparations (ointments, etc.), and in order to more significantly exhibit the effects of the third present invention, liquid preparations are preferable.

The ophthalmic composition according to the present embodiment can be used as, for example, eye drops (eye lotions or ophthalmic preparations; here the eye drops include an eye drop that can be instilled in the eyes wearing contact lenses), artificial tears, eyewashes (also called eyewash liquids or eyewash preparations; here the eyewashes include an eyewash that can wash the eyes wearing contact lenses), and contact lens compositions [a contact lens wearing liquid, a contact lens care composition (a contact lens disinfectant, a contact lens preservative, a contact lens cleaning agent, a contact lens cleaning preservative), a contact lens packaging liquid, etc.]. Here, "contact lenses" include hard contact lenses and soft contact lenses (including both ionic and non-ionic lenses and both silicone hydrogel contact lenses and non-silicone hydrogel contact lenses).

The ophthalmic composition according to the present embodiment is preferably an eye drop (including an eye drop that can be instilled in the eyes wearing contact lenses) because it can more significantly exhibit the effects of the third present invention. When the ophthalmic composition according to the present embodiment is an eye drop, the usage and dosage thereof are not particularly limited as long as they exhibit effects and have few side effects, and for example, for adults (aged 15 years or older) and children aged 7 years or older, a method of dropping 1 to 3 drops, 1 to 2 drops, or 2 to 3 drops at a time, 2 to 4 times or 5 to 6 times a day can be used.

The ophthalmic composition according to the present embodiment is provided after being stored in an arbitrary container. The container for storing the ophthalmic composition according to the present embodiment is not particularly limited, and may be made of, for example, glass or a plastic. The container is preferably made of a plastic. Examples of plastics include polyethylene terephthalate (PET), polyarylate, polyethylene naphthalate, polycarbonate, polyethylene, polypropylene, polyimide, copolymers of monomers constituting these, and mixtures of two or more of these. Polyethylene terephthalate is preferable. In addition, the container for storing the ophthalmic composition according to the present embodiment may be a transparent container that allows the inside of the container to be visually recognized or an opaque container that makes it difficult to visually recognize the inside of the container. A transparent container is preferable. Here, the "transparent container" includes both a colorless transparent container and a colored transparent container.

A nozzle may be attached to the container for storing the ophthalmic composition according to the present embodiment. The material of the nozzle is not particularly limited, and may be made of, for example, glass or a plastic. The container is preferably made of a plastic. Examples of plastics include polybutylene terephthalate, polyethylene, polypropylene, polyethylene terephthalate, polyethylene naphthalate, copolymers of monomers constituting these, and mixtures of two or more of these. In order to further improve the effects of the third present invention, the material of the nozzle is preferably polypropylene, polyethylene, polyethylene terephthalate, polybutylene terephthalate, or polyethylene naphthalate, and more preferably polyethylene.

The container for storing the ophthalmic composition according to the present embodiment may be a multi-dose type container in which a plurality of doses are stored or a unit-dose type container in which a single dose is stored.

The ophthalmic composition according to the present embodiment is preferably filled into a container having an internal volume of 4 to 30 mL, more preferably filled into a container having an internal volume of 5 to 20 mL, still more preferably filled into a container having an internal volume of 6 to 16 mL, and yet more preferably filled into a container having an internal volume of 10 to 15 mL. In addition, the ophthalmic composition may be filled into a container having an internal volume of 0.1 to 3 mL or may be filled into a container having an internal volume of 0.2 to 1 mL.

### [Examples of third present invention]

Hereinafter, the third present invention will be described in detail with reference to test examples, but the third present invention is not limited thereto. In addition, the sodium chondroitin sulfate used in the following test examples is as follows, and sodium chondroitin sulfate having a weight-average-molecular weight of about 56,000 was derived from sharks.
sodium chondroitin sulfate
weight-average-molecular weight of about 56,000: commercially available from Seikagaku Corporation; Grade N-K
weight-average-molecular weight of about 28,000: commercially available from Seikagaku Corporation; Grade ND-K
weight-average-molecular weight of about 25,000: commercially available from Maruha Nichiro Corporation; over-the-counter sodium chondroitin sulfate

### [Test Example 1: feeling of use test (1)]

Ophthalmic compositions of Comparative Examples 1-1 and 1-2 and Examples 1-1 and 1-2 were prepared according to a general method with the compositions shown in Table 30. In Table 30, the units of the components are w/v%. Each prepared ophthalmic composition was filtered through a 0.2 µm membrane filter, and filled into a sterilized PET eye drop container (volume of 15 mL) to prepare sterile eye drops. These eye drops were used to evaluate "unpleasant irritation" and "burning sensation" according to a visual analog scale (VAS) method. Specifically, one of the eye drops was instilled in the left and right eyes of six subjects with naked eyes, and regarding "unpleasant irritation" and "burning sensation" felt when instilled, the subjects were asked to check the degree of "unpleasant irritation" and "burning sensation" they felt using subjective symptom survey sheets with 10 cm lines drawn such as 0 cm when "unpleasant irritation" and "burning sensation" were not felt at all and 10 cm when "unpleasant irritation" and "burning sensation" were very strongly felt, and the length (mm) was measured as the degree of the subjective symptom intensity, and this was used to score each item. Each item was evaluated by calculating the score average value of the six subjects. Based on the obtained values, the improvement percentage of the VAS value was calculated using the following Formula (I). The comparative example corresponding to Example 1-1 was Comparative Example 1-1, and the comparative example corresponding to Example 1-2 was Comparative Example 1-2. VAS improvement percentage (%)={(VAS value of corresponding comparative example-VAS value of example)/VAS value of corresponding comparative example}×100

The results are also shown in Table 30.

**[Table 30]**

| | | Comparative Example 1-1 | Example 1-1 | Comparative Example 1-2 | Example 1-2 |
|---|---|---|---|---|---|
| Sodium chondroitin sulfate (weight-average-molecular weight of about 28,000) | | 0.5 | - | 1 | - |
| Sodium chondroitin sulfate (weight-average-molecular weight of about 56,000) | | - | 0.5 | - | 1 |
| 1-menthol | | 0.005 | 0.005 | 0.005 | 0.005 |
| Chlorobutanol | | 0.08 | 0.08 | 0.08 | 0.08 |
| Boric acid | | 0.5 | 0.5 | 0.5 | 0.5 |
| Borax | | 0.1 | 0.1 | 0.1 | 0.1 |
| Polysorbate 80 | | 0.1 | 0.1 | 0.017 | 0.017 |
| Polyoxyethylene hydrogenated castor oil 60 | | 0.1 | 0.1 | - | - |
| Sodium chloride | | 0.5 | 0.5 | 0.5 | 0.5 |
| Hydrochloric acid/sodium hydroxide | | Appropriate amount | Appropriate amount | Appropriate amount | Appropriate amount |
| Purified water | | Residual amount | Residual amount | Residual amount | Residual amount |
| Total amount | | 100 mL | 100 mL | 100 mL | 100 mL |
| pH | | 5.5 | 5.5 | 5.5 | 5.5 |
| VAS improvement percentage (%) | Unpleasant irritation | - | 70 | - | 95.1 |
| | Burning sensation | - | 87.5 | - | 100.0 |

It was confirmed that, compared to the eye drops containing menthol and sodium chondroitin sulfate having a weight-average-molecular weight of about 28,000 (Comparative Example 1-1 and Comparative Example 1-2), in the eye drops in which sodium chondroitin sulfate having a weight-average-molecular weight of about 56,000 was combined in place of sodium chondroitin sulfate having a weight-average-molecular weight of about 28,000 (Example 1-1 and Example 1-2), "unpleasant irritation" and "burning sensation" were significantly improved. Here, when soft contact lens wearers were asked to evaluate "unpleasant irritation" and "burning sensation" using the same method as above, the same tendency as in the case of the naked eyes was confirmed.

### [Test Example 2: test for measuring residual liquid amount in eye drop bottle]

Ophthalmic compositions were prepared according to a general method using the formulations shown in Tables 31 and 32 and used as test solutions. The units of the components in Tables 31 and 32 are w/v% unless specified in the tables. The tare weight of a 10 mL PET eye drop bottle was measured and 5 mL of each test solution was filled. Next, the weight of each eye drop bottle after each filled test solution was discharged was measured, and according to the following Formula (II), the residual liquid amount (g) was calculated, and regarding the residual liquid amount of each formulation, the improvement percentage of the residual liquid amount with respect to the residual liquid amount of the test solution 2-1 was calculated using the following Formula (III). residual liquid amount (g)=the weight of the eye drop bottle after the test solution is discharged (g)=the weight of the eye drop bottle (g) the improvement percentage (%) of the residual liquid amount with respect to the test solution 2-1=(1-the residual liquid amont of each test solution (g)/the resual liquid amount of the test solution 2-1 (g))×100

The results are also shown in Tables 31 and 32.

**[Table 31]**

| | Test solution 2-1 | Test solution 2-2 |
|---|---|---|
| Sodium chondroitin sulfate (weight-average-molecular weight of about 56,000) | - | 0.5 |
| 1-menthol | 0.008 | 0.008 |
| Boric acid | 1.2 | 1.2 |
| Borax | 0.1 | 0.1 |
| Polyoxyethylene hydrogenated castor oil 60 | 0.35 | 0.35 |
| Polyoxyethylene castor oil 10 | 0.2 | 0.2 |
| Hydrochloric acid/sodium hydroxide | Appropriate amount | Appropriate amount |
| Purified water | Residual amount | Residual amount |
| Total amount | 100 mL | 100 mL |
| pH | 5.8 | 5.8 |
| Improvement percentage (%) of residual liquid amount with respect to test solution 2-1 | - | 23.6 |

**[Table 32]**

| | Test solution 2-3 | Test solution 2-4 | Test solution 2-5 | Test solution 2-6 | Test solution 2-7 | Test solution 2-8 | Test solution 2-9 |
|---|---|---|---|---|---|---|---|
| Sodium chondroitin sulfate (weight-average-molecul ar weight of about 56,000) | 0.5 | 0.5 | 0.5 | 0.5 | 0.5 | 0.5 | 0.5 |
| Neostigmine methyl sulfate | 0.005 | - | - | - | - | - | - |
| Allantoin | - | 0.3 | - | - | - | - | - |
| Dipotassium glycyrrhizinate | - | - | 0.25 | - | - | - | - |
| Tocopherol acetate | - | - | - | 0.05 | - | - | - |
| Retinol palmitate | - | - | - | - | 25,000 units | - | - |
| Aminoethylsulfonic acid | - | - | - | - | - | 1 | - |
| Hydroxyethyl cellulose | - | - | - | - | - | - | 0.1 |
| 1-menthol | 0.008 | 0.008 | 0.008 | 0.008 | 0.008 | 0.008 | 0.008 |
| Boric acid | 1.2 | 1.2 | 1.2 | 1.2 | 1.2 | 1.2 | 1.2 |
| Borax | 0.1 | 0.1 | 0.1 | 0.1 | 0.1 | 0.1 | 0.1 |
| Polyoxyethylene hydrogenated castor oil 60 | 0.35 | 0.35 | 0.35 | 0.35 | 0.35 | 0.35 | 0.35 |
| Polyoxyethylene castor oil 10 | 0.2 | 0.2 | 0.2 | 0.2 | 0.2 | 0.2 | 0.2 |
| Hydrochloric acid/sodium hydroxide | Appropriat e amount | Appropriat e amount | Appropriat e amount | Appropriat e amount | Appropriat e amount | Appropriat e amount | Appropriat e amount |
| Purified water | Residual amount | Residual amount | Residual amount | Residual amount | Residual amount | Residual amount | Residual amount |
| Total amount | 100 mL | 100 mL | 100 mL | 100 mL | 100 mL | 100 mL | 100 mL |
| pH | 5.8 | 5.8 | 5.8 | 5.8 | 5.8 | 5.8 | 5.8 |
| Improvement percentage (%) of residual liquid amount with respect to test solution 2-1 | 27.6 | 30.0 | 25.5 | 34.1 | 37.7 | 25.8 | 28.5 |

It was confirmed that, when sodium chondroitin sulfate having a weight-average-molecular weight of about 56,000 was additionally added to the test solution 2-1 containing menthol, the residual liquid amount decreased, making it easier to use up the ophthalmic composition in the eye drop bottle (test solution 2-2). In addition, it was confirmed that, when neostigmine methyl sulfate, allantoin, dipotassium glycyrrhizinate, tocopherol acetate, retinol palmitate, aminoethylsulfonic acid or hydroxyethyl cellulose was additionally added to the test solution 2-2 containing menthol and sodium chondroitin sulfate having a weight-average-molecular weight of about 56,000, the residual liquid amount was further reduced, making it easier to use up the ophthalmic composition in the eye drop bottle (test solutions 2-3 to 2-9).

### [Test Example 3: test of viscosity stability upon light emission]

Ophthalmic compositions were prepared according to a general method with the composition shown in Tables 33 and 34 and used as test solutions. The units of the components in Tables 33 and 34 are w/v% unless specified in the tables. A glass bottle (10 mL) was filled with 10 mL of each test solution and radiated with light at 35°C using SUNTESTER XLS+ (1,700 W xenon air-cooled lamp light source commercially available from Toyo Seiki Co., Ltd.) at an UV illuminance of 765 (W/m²) for 96 hours. For each test solution (600 µL) before and after radiation, the viscosity at a shear rate (1 to 1,000 (1/s)) at 34°C was measured using a cone plate type measurement jig (CP50-1, d: 0.102 mm) with a rheometer (MCR302 (commercially available from AntonPaar)). Then, using the viscosity (mPals) at a shear rate of 100 (1/s), according to the following Formula (IV), the viscosity reduction percentage before and after the test was calculated. The ratio of the viscosity reduction percentage of the test solution 3-2 was calculated when the viscosity reduction percentage of the test solution 3-1 was set to 1. For the test solutions 3-3 to 3-8, regarding the viscosity reduction percentage of each formulation, the improvement percentage of the viscosity reduction percentage with respect to the test solution 3-2 was calculated using the following Formula (V). viscosity reduction percentage (%)={(the viscosity of each test solution before light emission (mPa/s)-the viscosity of each test solution after light emission (mPa/s))/the viscosity of each test solution before light emission (mPa/s)}×100 viscosity reduction improvement percentage (%)={(the viscosity reduction percentage of the test solution 3-2-the viscosity reduction percentage of each formulation example)/the viscosity reduction percentage of the test solution 3-2}×100

The results are also shown in Tables 33 and 34. Here, the smaller the value of the viscosity reduction percentage, the less the change in viscosity caused by light, and the more the ophthalmic composition is kept physically the same.

**[Table 33]**

| | Test solution 3-1 | Test solution 3-2 |
|---|---|---|
| Sodium chondroitin sulfate (weight-average-molecular weight of about 56,000) | - | 0.5 |
| 1-menthol | 0.008 | 0.008 |
| Boric acid | 1.2 | 1.2 |
| Borax | 0.1 | 0.1 |
| Polyoxyethylene hydrogenated castor oil 60 | 0.35 | 0.35 |
| Polyoxyethylene castor oil 10 | 0.2 | 0.2 |
| Hydrochloric acid/sodium hydroxide | Appropriate amount | Appropriate amount |
| Purified water | Residual amount | Residual amount |
| Total amount | 100 mL | 100 mL |
| pH | 5.8 | 5.8 |
| Ratio of viscosity reduction percentage when viscosity reduction percentage of test solution 3-1 is set to 1 (at a shear rate of 100 (1/s)) | 1 | 10.6 |

**[Table 34]**

| | Test solution 3-2 | Test solution 3-3 | Test solution 3-4 | Test solution 3-5 | Test solution 3-6 | Test solution 3-7 | Test solution 3-8 |
|---|---|---|---|---|---|---|---|
| Sodium chondroitin sulfate (weight-average-molecul ar weight of about 56,000) | 0.5 | 0.5 | 0.5 | 0.5 | 0.5 | 0.5 | 0.5 |
| Neostigmine methyl sulfate | - | 0.005 | - | - | - | - | - |
| Allantoin | - | - | 0.3 | - | - | - | - |
| Zinc sulfate | - | - | - | 0.25 | - | - | - |
| Panthenol | - | - | - | - | 0.1 | - | - |
| Potassium aspartate | - | - | - | - | - | 1 | - |
| Aminoethylsulfonic acid | - | - | - | - | - | - | 1 |
| 1-menthol | 0.008 | 0.008 | 0.008 | 0.008 | 0.008 | 0.008 | 0.008 |
| Boric acid | 1.2 | 1.2 | 1.2 | 1.2 | 1.2 | 1.2 | 1.2 |
| Borax | 0.1 | 0.1 | 0.1 | 0.1 | 0.1 | 0.1 | 0.1 |
| Polyoxyethylene hydrogenated castor oil 60 | 0.35 | 0.35 | 0.35 | 0.35 | 0.35 | 0.35 | 0.35 |
| Polyoxyethylene castor oil 10 | 0.2 | 0.2 | 0.2 | 0.2 | 0.2 | 0.2 | 0.2 |
| Hydrochloric acid/sodium hydroxide | Appropriat e amount | Appropriat e amount | Appropriat e amount | Appropriat e amount | Appropriat e amount | Appropriat e amount | Appropriat e amount |
| Purified water | Residual amount | Residual amount | Residual amount | Residual amount | Residual amount | Residual amount | Residual amount |
| Total amount | 100 mL | 100 mL | 100 mL | 100 mL | 100 mL | 100 mL | 100 mL |
| pH | 5.8 | 5.8 | 5.8 | 5.8 | 5.8 | 5.8 | 5.8 |
| Viscosity reduction improvement percentage (%) (at a shear rate of 100 (1/s)) | - | 32.8 | 26.7 | 55.9 | 35.7 | 21.6 | 22.2 |

It was confirmed that, when sodium chondroitin sulfate having a weight-average-molecular weight of about 56,000 was additionally added to the test solution 3-1 containing menthol, the viscosity tended to decrease significantly (test solution 3-2). On the other hand, it was confirmed that, in test solutions 3-3 to 3-8 in which neostigmine methyl sulfate, allantoin, zinc sulfate, panthenol, potassium aspartate or aminoethylsulfonic acid was additionally added to the test solution 3-2 containing sodium chondroitin sulfate having a weight-average-molecular weight of about 56,000 and menthol together, a decrease in viscosity was significantly improved, and the stability of the ophthalmic composition upon UV emission was improved.

### [Test Example 4: measurement of viscosity]

Ophthalmic compositions were prepared according to a general method with the composition shown in Tables 35 and 36 and used as test solutions. The units of the components in Tables 35 and 36 are w/v% unless specified in the tables. Regarding the viscosity of each test solution (600 µL) after preparation, the viscosity against a shear rate at 34°C was measured using a cone plate type measurement jig (CP50-1, d: 0.102 mm) with a rheometer (MCR302 (commercially available from AntonPaar)). Regarding the viscosity (mPals) at a shear rate of 10,000 (1/s), the ratio of the viscosity of the test solution 4-2 was calculated when the viscosity of the test solution 4-1 was set to 1. For test solutions 4-3 to 4-11, the viscosity reduction percentage (%) with respect to the test solution 4-2 was calculated using the following Formula (VI). Here, the shear rate of 10,000 (1/s) was assumed to be the blink rate. The viscosity at a shear rate of 10,000 (1/s) indicates a viscosity during blinking, and a decrease in the value indicates that discomfort was not particularly felt during blinking and it was easier to blink. viscosity reduction percentage (%)=(1-the viscosity of each test solution (mPa/s)/the viscosity of the test solution 4-2 (mPa/s))×100

The results are also shown in Tables 35 and 36.

**[Table 35]**

| | Test solution 4-1 | Test solution 4-2 |
|---|---|---|
| Sodium chondroitin sulfate (weight-average-molecular weight of about 56,000) | 0.5 | 0.5 |
| 1-menthol | - | 0.008 |
| Boric acid | 1.2 | 1.2 |
| Borax | 0.1 | 0.1 |
| Polyoxyethylene hydrogenated castor oil 60 | 0.35 | 0.35 |
| Polyoxyethylene castor oil 10 | 0.2 | 0.2 |
| Hydrochloric acid/sodium hydroxide | Appropriate amount | Appropriate amount |
| Purified water | Residual amount | Residual amount |
| Total amount | 100 mL | 100 mL |
| pH | 5.8 | 5.8 |
| Ratio of viscosity when viscosity of test solution 4-1 is set to 1 | 1 | 1.5 |

**[Table 36]**

| | Test solutio n 4-2 | Test solutio n 4-3 | Test solutio n 4-4 | Test solutio n 4-5 | Test solutio n 4-6 | Test solutio n 4-7 | Test solutio n 4-8 | Test solutio n 4-9 | Test solutio n 4-10 | Test solutio n 4-11 |
|---|---|---|---|---|---|---|---|---|---|---|
| Sodium chondroitin sulfate (weight-average-molecular weight of about 56,000) | 0.5 | 0.5 | 0.5 | 0.5 | 0.5 | 0.5 | 0.5 | 0.5 | 0.5 | 0.5 |
| Tetrahydrozolin e hydrochloride | - | 0.05 | - | - | - | - | - | - | - | - |
| Neostigmine methyl sulfate | - | - | 0.005 | - | - | - | - | - | - | - |
| Allantoin | - | - | - | 0.3 | - | - | - | - | - | - |
| Dipotassium glycyrrhizinate | - | - | - | - | 0.25 | - | - | - | - | - |
| Zinc sulfate | - | - | - | - | - | 0.25 | - | - | - | - |
| Chlorpheniramin e maleate | - | - | - | - | - | - | 0.03 | - | - | - |
| Panthenol | - | - | - | - | - | - | - | 0.1 | - | - |
| Potassium aspartate | - | - | - | - | - | - | - | - | 1 | - |
| Aminoethylsulfo nic acid | - | - | - | - | - | - | - | - | - | 1 |
| l-menthol | 0.008 | 0.008 | 0.008 | 0.008 | 0.008 | 0.008 | 0.008 | 0.008 | 0.008 | 0.008 |
| Boric acid | 1.2 | 1.2 | 1.2 | 1.2 | 1.2 | 1.2 | 1.2 | 1.2 | 1.2 | 1.2 |
| Borax | 0.1 | 0.1 | 0.1 | 0.1 | 0.1 | 0.1 | 0.1 | 0.1 | 0.1 | 0.1 |
| Polyoxyethylene hydrogenated castor oil 60 | 0.35 | 0.35 | 0.35 | 0.35 | 0.35 | 0.35 | 0.35 | 0.35 | 0.35 | 0.35 |
| Polyoxyethylene | 0.2 | 0.2 | 0.2 | 0.2 | 0.2 | 0.2 | 0.2 | 0.2 | 0.2 | 0.2 |
| castor oil 10 | | | | | | | | | | |
| Hydrochloric acid/sodium hydroxide | Approp riate amount | Appro priate amount | Appro priate amount | Appro priate amount | Appro priate amount | Appro priate amount | Appro priate amount | Appro priate amount | Appro priate amount | Appro priate amount |
| Purified water | Residu al amount | Residu al amount | Residu al amount | Residu al amount | Residu al amount | Residu al amount | Residu al amount | Residu al amount | Residu al amount | Residu al amount |
| Total amount | 100 mL | 100 mL | 100 mL | 100 mL | 100 mL | 100 mL | 100 mL | 100 mL | 100 mL | 100 mL |
| pH | 5.8 | 5.8 | 5.8 | 5.8 | 5.8 | 5.8 | 5.8 | 5.8 | 5.8 | 5.8 |
| Viscosity reduction percentage (%) | - | 13.2 | 10.4 | 8.2 | 13.6 | 24.6 | 12.1 | 8.9 | 42.1 | 17.5 |

It was confirmed that, when the test solution 4-1 containing only sodium chondroitin sulfate having a weight-average-molecular weight of about 56,000 additionally contained menthol, the viscosity at a shear rate of 10,000 (1/s) was improved (test solution 4-2). On the other hand, it was confirmed that, when tetrahydrozoline hydrochloride, neostigmine methyl sulfate, allantoin, dipotassium glycyrrhizinate, zinc sulfate, chlorpheniramine maleate, panthenol, potassium aspartate or aminoethylsulfonic acid was additionally added to the test solution 4-2 containing sodium chondroitin sulfate having a weight-average-molecular weight of about 56,000 and menthol together, the viscosity at a shear rate of 10,000 (1/s) significantly decreased.

### [Test Example 5: feeling of use test (2)]

Ophthalmic compositions of Comparative Examples 5-1 and Example 5-1 were prepared according to a general method using the formulations shown in Table 37. In Table 37, the units of the components are w/v%. Each prepared ophthalmic composition was filtered through a 0.2 µm membrane filter, and filled into a sterilized PET eye drop container (volume of 15 mL) to prepare sterile eye drops. These eye drops were used to evaluate "unpleasant irritation" and "burning sensation" according to a visual analog scale (VAS) method. Specifically, one of the eye drops was instilled in the left and right eyes of three subjects with naked eyes, and regarding "unpleasant irritation" and "burning sensation" felt when instilled, the subjects were asked to check the degree of "unpleasant irritation" and "burning sensation" they felt using subjective symptom survey sheets with 10 cm lines drawn such as 0 cm when "unpleasant irritation" and "burning sensation" were not felt at all and 10 cm when "unpleasant irritation" and "burning sensation" were very strongly felt, and the length (mm) was measured as the degree of the subjective symptom intensity, and this was used to score each item. Each item was evaluated by calculating the score average value of the three subjects. Based on the obtained values, the improvement percentage of the VAS value was calculated using Formula (I) in Test Example 1. The comparative example corresponding to Example 5-1 was Comparative Example 5-1.

The results are also shown in Table 37.

**[Table 37]**

| | | Comparative Example 5-1 | Example 5-1 |
|---|---|---|---|
| Sodium chondroitin sulfate (weight-average-molecular weight of about 28,000) | | 1 | - |
| Sodium chondroitin sulfate (weight-average-molecular weight of about 56,000) | | - | 1 |
| 1-menthol | | 0.005 | 0.005 |
| Boric acid | | 1.5 | 1.5 |
| Borax | | 0.1 | 0.1 |
| Polysorbate 80 | | 0.1 | 0.1 |
| Polyoxyethylene hydrogenated castor oil 60 | | 0.1 | 0.1 |
| Sodium chloride | | 0.5 | 0.5 |
| Hydrochloric acid/sodium hydroxide | | Appropriate amount | Appropriate amount |
| Purified water | | Residual amount | Residual amount |
| Total amount | | 100 mL | 100 mL |
| pH | | 7 | 7 |
| VAS improvement percentage (%) | Unpleasant irritation | - | 89.2 |
| | Burning sensation | - | 86.9 |

For the ophthalmic compositions shown in Table 37, the same tendency as in Test Example 1 was confirmed.

### [Formulation Examples]

Eye drops and artificial tears were prepared according to a general method using the formulations shown in the following Tables 38 to 41. Here, the units of the component amounts in the following Tables 38 to 41 are w/v% unless specified in the tables. Formulation Examples 1 to 10, 21 to 27, and 29 to 32 were eye drops, and Formulation Examples 11 to 16, and 28 were artificial tears. In addition, those obtained by filling Formulation Examples 1 to 32 into a polyethylene terephthalate container (volume of 15 mL) and fitted with a polyethylene nozzle were used as Formulation Examples 1' to 32', those with the innermost layer fitted with a polybutylene terephthalate nozzle were used as Formulation Examples 1" to 32", and those with the innermost layer fitted with a polyethylene naphthalate nozzle were used as Formulation Examples 1‴ to 32"'.

**[Table 38]**

| Component name | Formul ation Examp le 1 | Formul ation Examp le 2 | Formul ation Examp le 3 | Formul ation Examp le 4 | Formul ation Examp le 5 | Formul ation Examp le 6 | Formul ation Examp le 7 | Formul ation Examp le 8 | Formul ation Examp le 9 | Formul ation Examp le 10 |
|---|---|---|---|---|---|---|---|---|---|---|
| Sodium chondroitin sulfate (weight-average -molecular weight of about 56,000) | 0.5 | 1 | 0.1 | 0.25 | 0.1 | 0.5 | 0.25 | 0.4 | 0.25 | 0.15 |
| 1-menthol | 0.02 | 0.01 | 0.0005 | 0.005 | 0.008 | 0.001 | 0.005 | 0.002 | 0.0045 | 0.050 |
| Chlorobutanol | 0.1 | 0.08 | - | - | - | - | 0.05 | - | - | 0.2 |
| Tetrahydrozolin e hydrochloride | 0.01 | 0.03 | 0.1 | 0.01 | 0.01 | 0.02 | - | - | - | 0.02 |
| Neostigmine methyl sulfate | - | 0.005 | | 0.005 | 0.005 | - | - | - | - | 0.0025 |
| Epsilon-aminoc aproic acid | - | 1 | 1 | 1 | 1 | - | - | - | 1 | - |
| Allantoin | 0.1 | - | - | - | - | - | 0.05 | - | - | - |
| Dipotassium glycyrrhizinate | - | 0.1 | 0.25 | - | - | 0.25 | 0.1 | - | - | 0.25 |
| Sodium azulene sulfonate | - | - | - | - | - | - | - | 0.02 | - | - |
| Berberine chloride | - | - | - | - | - | 0.01 | | 0.01 | - | - |
| Zinc sulfate | - | 0.05 | - | - | - | - | 0.1 | - | - | 0.1 |
| Chlorphenirami ne maleate | - | 0.03 | 0.03 | 0.03 | 0.03 | 0.02 | 0.01 | 0.03 | - | - |
| Flavin adenine dinucleotide sodium | - | - | 0.05 | - | - | - | - | 0.01 | 0.05 | - |
| Cyanocobalami n | - | 0.01 | - | - | - | - | - | - | - | - |
| Retinol palmitate | - | 50,000 units | - | 50,000 units | 50,000 units | 10,000 units | - | 50,000 units | - | - |
| Pyridoxine hydrochloride | - | 0.05 | 0.1 | - | 0.01 | 0.08 | - | - | 0.01 | - |
| d-α-tocopherol acetate | 0.03 | - | - | 0.05 | 0.03 | - | 0.025 | 0.01 | - | - |
| Potassium L-aspartate | 0.5 | 0.5 | 0.5 | - | 0.8 | - | - | 0.1 | 1 | - |
| Magnesium/pota ssium L-aspartate | - | - | - | 0.8 | - | - | 1 | - | 2 | - |
| Taurine | 0.5 | 0.5 | 1 | 0.5 | 1 | - | 0.5 | 0.1 | 1 | - |
| Sodium chloride | - | 0.008 | - | - | - | - | - | - | - | - |
| Potassium chloride | - | - | - | - | - | - | 0.1 | - | - | - |
| d-camphor | - | 0.001 | - | - | - | - | - | - | 0.001 | - |
| d-borneol | - | 0.001 | | 0.001 | - | - | - | - | - | - |
| Geraniol | 0.001 | - | 0.002 | - | - | - | 0.0001 | 0.003 | - | - |
| Eucalyptus oil | 0.001 | - | - | 0.001 | - | - | | 0.003 | - | - |
| Bereamot oil | - | - | - | 0.001 | - | 0.0005 | - | | - | - |
| Mint oil | - | - | - | - | 0.001 | 0.0005 | - | - | - | 0.001 |
| Polyoxyethylene hydrogenated castor oil | - | - | 0.05 | 0.2 | 0.3 | - | - | - | 0.1 | - |
| Polyoxyethylene castor oil | - | - | - | - | - | - | 0.1 | - | - | - |
| Polysorbate 80 | 0.2 | - | - | 0.2 | 0.2 | - | 0.3 | 0.5 | 0.1 | 0.25 |
| Polyoxyl stearate | - | - | - | - | - | - | 0.1 | - | - | - |
| Polyoxyethylene polyoxypropyle ne glycol | 0.05 | - | - | - | - | - | - | - | - | - |
| | | | | | | | | | | |
| Benzalkonium chloride | - | 0.01 | - | - | - | - | - | - | - | - |
| Chlorhexidine gluconate | - | - | - | - | - | - | - | - | 0.005 | - |
| Polyhexanide hydrochloride | 1 ppm | - | 1 ppm | - | - | - | - | - | - | - |
| Zinc chloride | - | - | - | - | - | - | - | - | - | 0.001 |
| Edetate sodium | 0.01 | 0.05 | 0.01 | 0.05 | 0.05 | - | 0.01 | 0.01 | 0.05 | 0.05 |
| Boric acid | 1.2 | 0.2 | 0.3 | 0.1 | 0.5 | 0.3 | 0.1 | 1 | 0.4 | 0.1 |
| Borax | 0.3 | 0.1 | 0.05 | 0.025 | - | - | 0.01 | 0.1 | 0.1 | 0.2 |
| Dibasic sodium phosphate | - | - | - | - | - | - | - | - | - | 0.15 |
| Sodium dihydrogen phosphate | - | - | - | - | - | - | - | - | - | 0.2 |
| Citric acid | - | - | - | - | - | - | - | - | 0.1 | - |
| Trometamol | - | - | - | - | 0.3 | - | - | - | - | - |
| Sesame oil | - | - | - | - | - | - | 0.001 | | | |
| Propylene glycol | - | - | - | - | 0.2 | - | - | - | - | - |
| Concentrated glycerin | 0.1 | - | - | - | - | - | - | - | - | - |
| Sodium hyaluronate | 0.005 | 0.002 | - | - | - | - | - | - | - | 0.01 |
| Dextran | - | - | - | 0.05 | - | - | - | - | - | - |
| L-arginine | - | - | - | - | - | - | - | - | - | 0.1 |
| Polyvinylpyrroli done | - | - | - | - | - | - | 0.1 | - | - | - |
| Hydroxyethyl cellulose | - | - | 0.1 | - | - | - | - | - | - | - |
| Hydroxypropyl methylcellulose | 0.1 | - | - | - | - | - | 0.2 | - | 0.01 | - |
| Dibutyl hydroxy toluene | - | - | - | 0.005 | 0.01 | - | - | - | - | - |
| Hydrochloric acid | Approp riate amount | Approp riate amount | Approp riate amount | Approp riate amount | Approp riate amount | Approp riate amount | Approp riate amount | Approp riate amount | Approp riate amount | Approp riate amount |
| Sodium hydroxide | Approp riate amount | Approp riate amount | Approp riate amount | Approp riate amount | Approp riate amount | Approp riate amount | Approp riate amount | Approp riate amount | Approp riate amount | Approp riate amount |
| Purified water | Residu al amount | Residu al amount | Residu al amount | Residu al amount | Residu al amount | Residu al amount | Residu al amount | Residu al amount | Residu al amount | Residu al amount |
| Total amount | 100 mL | 100 mL | 100 mL | 100 mL | 100 mL | 100 mL | 100 mL | 100 mL | 100 mL | 100 mL |
| pH | 5.8 | 6.5 | 5.7 | 6.2 | 5.8 | 7.0 | 5.3 | 7.5 | 7.0 | 5.5 |

**[Table 39]**

| Component name | Formulation Example 11 | Formulation Example 12 | Formulation Example 13 | Formulation Example 14 | Formulation Example 15 | Formulation Example 16 |
|---|---|---|---|---|---|---|
| Sodium chondroitin sulfate (weight-average-molecular weight of about 56,000) | 0.2 | 1 | 0.5 | 0.3 | 0.5 | 0.5 |
| l-menthol | 0.04 | 0.002 | 0.005 | 0.015 | 0.0008 | 0.005 |
| Chlorobutanol | 0.15 | - | - | 0.08 | - | - |
| Magnesium/potassium L-aspartate | - | 1 | - | - | 0.5 | - |
| Taurine | - | 0.5 | - | 1 | - | 0.5 |
| Sodium chloride | - | - | 0.08 | - | - | 0.05 |
| Potassium chloride | 0.6 | 0.4 | 0.1 | 0.3 | 0.45 | 0.4 |
| d-camphor | 0.01 | - | - | 0.003 | - | 0.003 |
| Geraniol | 0.003 | - | - | - | - | - |
| Eucalyptus oil | - | 0.002 | - | - | - | - |
| Bergamot oil | - | - | - | 0.001 | - | - |
| Mint oil | - | 0.002 | - | - | - | - |
| Polyoxyethylene hydrogenated castor oil | - | - | 0.1 | 0.1 | - | - |
| Polyoxyethylene castor oil | - | - | - | - | 0.3 | - |
| Polysorbate 80 | 0.3 | 0.2 | - | - | - | 0.2 |
| Polyoxyl stearate | - | - | - | - | 0.1 | - |
| Polyoxyethylene polyoxypropylene glycol | - | - | - | - | 0.05 | - |
| Benzalkonium chloride | - | 0.01 | 0.01 | - | - | - |
| Chlorhexidine gluconate | - | - | - | - | 0.02 | - |
| Polyhexanide hydrochloride | 1 ppm | - | - | 1 ppm | - | - |
| Zinc chloride | - | - | - | - | - | 0.0001 |
| Edetate sodium | - | 0.005 | 0.05 | 0.005 | 0.01 | 0.05 |
| Boric acid | 1.5 | 1 | 0.4 | 1 | - | 1 |
| Borax | | 0.1 | 0.1 | 0.2 | | 0.2 |
| Dibasic sodium phosphate | - | - | - | - | 0.15 | - |
| Sodium dihydrogen phosphate | - | - | - | - | 0.1 | - |
| Citric acid | - | - | - | 0.1 | - | 0.1 |
| Sesame oil | - | - | - | - | 0.05 | |
| Concentrated glycerin | - | - | - | - | - | 0.1 |
| Sodium hyaluronate | - | 0.02 | - | - | - | - |
| L-arginine | - | 0.5 | - | - | - | - |
| Polyvinylpyrrolidone | - | - | 0.5 | 0.1 | - | - |
| Hydroxyethyl cellulose | 0.07 | - | - | - | 0.2 | |
| Hydroxypropyl methylcellulose | - | - | 0.1 | - | 0.2 | 0.3 |
| Hydrochloric acid | Appropriate amount | Appropriate amount | Appropriate amount | Appropriate amount | Appropriate amount | Appropriate amount |
| Sodium hydroxide | Appropriate amount | Appropriate amount | Appropriate amount | Appropriate amount | Appropriate amount | Appropriate amount |
| Purified water | Residual amount | Residual amount | Residual amount | Residual amount | Residual amount | Residual amount |
| Total amount | 100 mL | 100 mL | 100 mL | 100 mL | 100 mL | 100 mL |
| pH | 6.5 | 7.2 | 6.8 | 7.0 | 7.5 | 7.0 |

**[Table 40]**

| Component name | Formulati on Example 17 | Formulati on Example 18 | Formulati on Example 19 | Formulati on Example 20 | Formulati on Example 21 | Formulati on Example 22 | Formulati on Example 23 | Formulati on Example 24 |
|---|---|---|---|---|---|---|---|---|
| Sodium chondroitin sulfate (MW of about 56,000) | - | 0.25 | 0.05 | 0.4 | - | 0.25 | 0.05 | 0.4 |
| Sodium chondroitin sulfate (MW of about 45,000) | 0.5 | - | - | - | 0.5 | - | - | - |
| Sodium chondroitin sulfate (MW of about 25,000) | - | 0.25 | 0.45 | 0.1 | - | 0.25 | 0.45 | 0.1 |
| Tetrahydrozoline hydrochloride | 0.01 | 0.01 | 0.01 | 0.01 | 0.05 | 0.05 | 0.05 | 0.05 |
| Neostigmine methyl sulfate | - | - | - | - | 0.005 | 0.005 | 0.005 | 0.005 |
| Epsilon-aminocap roic acid | - | - | - | - | 1 | 1 | 1 | 1 |
| Dipotassium glycyrrhizinate | 0.25 | 0.25 | 0.25 | 0.25 | 0.1 | 0.1 | 0.1 | 0.1 |
| Chlorpheniramine maleate | 0.03 | 0.03 | 0.03 | 0.03 | - | - | - | - |
| Retinol palmitate | - | - | - | - | 50,000 units | 50,000 units | 50,000 units | 50,000 units |
| Pyridoxine hydrochloride | 0.1 | 0.1 | 0.1 | 0.1 | 0.05 | 0.05 | 0.05 | 0.05 |
| Potassium L-aspartate | - | - | - | - | 1 | 1 | 1 | 1 |
| Taurine | - | - | - | - | 1 | 1 | 1 | 1 |
| 1-menthol | 0.01 | 0.01 | 0.01 | 0.01 | 0.002 | 0.002 | 0.002 | 0.002 |
| d-camphor | 0.005 | 0.005 | 0.005 | 0.005 | 0.003 | 0.003 | 0.003 | 0.003 |
| d-borneol | 0.003 | 0.003 | 0.003 | 0.003 | - | - | - | - |
| Polyoxyethylene hydrogenated castor oil | - | - | - | - | 0.1 | 0.1 | 0.1 | 0.1 |
| Polyoxyethylene castor oil | 0.1 | 0.1 | 0.1 | 0.1 | - | - | - | - |
| Polysorbate 80 | 0.5 | 0.5 | 0.5 | 0.5 | | | | |
| Benzalkonium chloride | - | - | - | - | 0.01 | 0.01 | 0.01 | 0.01 |
| Edetate sodium | 0.01 | 0.01 | 0.01 | 0.01 | 0.05 | 0.05 | 0.05 | 0.05 |
| Boric acid | 1.5 | 1.5 | 1.5 | 1.5 | 0.4 | 0.4 | 0.4 | 0.4 |
| Borax | 0.5 | 0.5 | 0.5 | 0.5 | 0.1 | 0.1 | 0.1 | 0.1 |
| Citric acid | - | - | - | - | 0.1 | 0.1 | 0.1 | 0.1 |
| Polyvinylpyrrolid one | - | - | - | - | 0.5 | 0.5 | 0.5 | 0.5 |
| Dibutyl hydroxy toluene | - | - | - | - | 0.005 | 0.005 | 0.005 | 0.005 |
| Hydrochloric acid | Appropri ate amount | Appropri ate amount | Appropri ate amount | Appropri ate amount | Appropri ate amount | Appropri ate amount | Appropri ate amount | Appropri ate amount |
| Sodium hydroxide | Appropri ate amount | Appropri ate amount | Appropri ate amount | Appropri ate amount | Appropri ate amount | Appropri ate amount | Appropri ate amount | Appropri ate amount |
| Purified water | Residual amount | Residual amount | Residual amount | Residual amount | Residual amount | Residual amount | Residual amount | Residual amount |
| Total amount | 100 mL | 100 mL | 100 mL | 100 mL | 100 mL | 100 mL | 100 mL | 100 mL |
| pH | 7.0 | 7.0 | 7.0 | 7.0 | 7.0 | 7.0 | 7.0 | 7.0 |

**[Table 41]**

| Component name | Formulati on Example 25 | Formulati on Example 26 | Formulati on Example 27 | Formulati on Example 28 | Formulati on Example 29 | Formulati on Example 30 | Formulati on Example 31 | Formulati on Example 32 |
|---|---|---|---|---|---|---|---|---|
| Sodium chondroitin sulfate (MW of about 56,000) | 0.5 | 0.5 | 0.5 | 0.5 | 0.5 | 0.5 | 0.5 | 0.5 |
| Neostigmine methyl sulfate | - | 0.005 | - | - | - | - | - | - |
| Epsilon-aminocap roic acid | - | 1 | - | - | - | - | - | - |
| Chlorpheniramine maleate | - | 0.03 | - | - | 0.03 | 0.03 | 0.03 | 0.03 |
| Retinol palmitate | 50,000 units | 50,000 units | 50,000 units | - | - | - | - | - |
| d-α-tocopherol acetate | 0.05 | 0.045 | 0.05 | - | 0.005 | - | 0.005 | - |
| potassium L-aspartate | - | 0.8 | - | - | 0.2 | - | 0.2 | - |
| Taurine | - | 1 | - | - | 0.1 | - | 0.1 | - |
| Sodium chloride | 0.1 | - | - | 0.1 | - | - | - | - |
| Potassium chloride | 0.2 | - | - | 0.4 | - | - | - | - |
| 1-menthol | - | 0.002 | - | - | 0.03 | 0.015 | 0.03 | 0.015 |
| d-borneol | - | 0.01 | - | - | - | - | - | - |
| Polyoxyethylene hydrogenated castor oil | - | 0.3 | - | 0.5 | - | - | 0.2 | - |
| Polysorbate 80 | - | 0.3 | - | 0.5 | 0.3 | 0.3 | - | 0.3 |
| Polyoxyethylene polyoxypropylene glycol | 0.55 | - | 0.55 | - | - | - | - | - |
| Benzalkonium chloride | - | - | - | - | 0.01 | 0.01 | - | 0.01 |
| Sodium bisulfite | 0.4 | - | 0.4 | 0.2 | - | - | - | - |
| Polyhexanide hydrochloride | - | - | - | - | - | - | 1 ppm | - |
| Sodium cromoglicate | - | - | - | - | 1 | 1 | - | - |
| Tranilast | - | - | - | - | - | - | 0.5 | 0.5 |
| Pranoprofen | - | - | - | - | 0.05 | 0.05 | 0.05 | 0.05 |
| Edetate sodium | 0.05 | 0.1 | 0.05 | 0.1 | 0.05 | 0.05 | 0.05 | 0.05 |
| Boric acid | 1 | 1 | 1 | 0.5 | 1 | 0.8 | 1 | 0.8 |
| Borax | 0.02 | 0.02 | 0.02 | 0.05 | 0.1 | 0.05 | 0.1 | 0.05 |
| Trometamol | - | 0.1 | 0.1 | - | - | - | - | - |
| Liquid paraffin | - | - | - | 0.1 | - | - | - | - |
| Propylene glycol | - | 0.2 | 0.3 | - | - | - | - | - |
| Polyvinylpyrrolid one | - | - | 0.2 | 0.2 | - | - | 1.5 | 3 |
| Hydroxypropyl methylcellulose | - | - | 0.1 | 0.1 | - | - | | |
| Monoethanolami ne | - | - | - | - | - | - | 0.2 | 0.1 |
| Dibutyl hydroxy toluene | 0.01 | 0.01 | 0.01 | 0.01 | 0.01 | 0.01 | 0.01 | 0.01 |
| Hydrochloric acid | Appropri ate amount | Appropri ate amount | Appropri ate amount | Appropri ate amount | Appropri ate amount | Appropri ate amount | Appropri ate amount | Appropri ate amount |
| Sodium hydroxide | Appropri ate amount | Appropri ate amount | Appropri ate amount | Appropri ate amount | Appropri ate amount | Appropri ate amount | Appropri ate amount | Appropri ate amount |
| Purified water | Residual amount | Residual amount | Residual amount | Residual amount | Residual amount | Residual amount | Residual amount | Residual amount |
| Total amount | 100 mL | 100 mL | 100 mL | 100 mL | 100 mL | 100 mL | 100 mL | 100 mL |
| pH | 7.0 | 6.0 | 7.0 | 7.0 | 7.0 | 6.0 | 8.0 | 7.5 |

## Claims

1. An ophthalmic composition for reducing eye dryness containing at least one selected from the group consisting of chondroitin sulfate and salts thereof which have a weight-average-molecular weight of 40,000 to 70,000.

2. An ophthalmic composition for reducing dryness of contact lenses containing at least one selected from the group consisting of chondroitin sulfate and salts thereof which have a weight-average-molecular weight of 40,000 to 70,000.

3. An ophthalmic composition containing at least one selected from the group consisting of chondroitin sulfate and salts thereof which have a weight-average-molecular weight of 40,000 to 70,000, and at least one selected from the group consisting of an anti-inflammatory agent, A vitamins, B vitamins, E vitamins, aminoethylsulfonic acid and salts thereof, aspartic acid and salts thereof, neostigmine and salts thereof, and a cellulose-based polymer compound.

4. The ophthalmic composition according to claim 3,
wherein the anti-inflammatory agent is at least one selected from the group consisting of allantoin and salts thereof, and glycyrrhizic acid and salts thereof, the B vitamins are at least one selected from the group consisting of panthenol, and pyridoxine and salts thereof, and the cellulose-based polymer compound is at least one selected from the group consisting of hydroxypropyl methylcellulose and salts thereof.

5. An ophthalmic composition containing at least one selected from the group consisting of chondroitin sulfate and salts thereof which have a weight-average-molecular weight of 40,000 to 70,000, and at least one selected from the group consisting of an antihistamine, a zinc salt, a decongestant, hydroxyethyl cellulose and salts thereof, and a polyvinyl-based polymer compound.

6. The ophthalmic composition according to claim 5,
wherein the antihistamine is at least one selected from the group consisting of chlorpheniramine and salts thereof, the decongestant is at least one selected from the group consisting of tetrahydrozoline and salts thereof, and the polyvinyl-based polymer compound is polyvinylpyrrolidone.

7. An ophthalmic composition containing at least one selected from the group consisting of chondroitin sulfate and salts thereof which have a weight-average-molecular weight of 40,000 to 70,000, and menthol.

8. The ophthalmic composition according to claim 7, further containing at least one selected from the group consisting of boric acid and salts thereof.

9. The ophthalmic composition according to claim 7 or 8, further containing chlorobutanol.

10. The ophthalmic composition according to claim 7 or 8, further containing at least one selected from the group consisting of a decongestant, an eye muscle regulating agent, an anti-inflammatory agent, an antihistamine, A vitamins, B vitamins, E vitamins, amino acids and salts thereof, and a cellulose-based polymer compound.
